(19) Europäisches Patentamt
European Patent Office
Office européen des brevets

(11) EP 1 611 890 B1

(12) **EUROPEAN PATENT SPECIFICATION**

(45) Date of publication and mention of the grant of the patent:
**26.08.2009 Bulletin 2009/35**

(51) Int Cl.:
*A61K 31/47* *(2006.01)* *C12Q 1/68* *(2006.01)*

(21) Application number: **05254157.0**

(22) Date of filing: **01.07.2005**

(54) **Methods for assessing and treating cancer**

Verfahren zum Evaluieren und Behandeln von Krebs

Méthodes pour évaluer et traiter le cancer

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.07.2004 US 883436**

(43) Date of publication of application:
**04.01.2006 Bulletin 2006/01**

(73) Proprietor: **Veridex, LLC**
**Warren, NJ 07059 (US)**

(72) Inventor: **Raponi, Mitch**
**San Diego, CA 92101 (US)**

(74) Representative: **Mercer, Christopher Paul et al**
**Carpmaels & Ransford**
**43-45 Bloomsbury Square**
**London WC1A 2RA (GB)**

(56) References cited:
**EP-A- 1 367 138** **EP-A- 1 502 962**
**WO-A-03/038129**

- **MAZIERES JULIEN ET AL: "Perspectives on farnesyl transferase inhibitors in cancer therapy." CANCER LETTERS. 8 APR 2004, vol. 206, no. 2, 8 April 2004 (2004-04-08), pages 159-167, XP002349862 ISSN: 0304-3835**
- **SEBTI SAÏD M: "Blocked pathways: FTIs shut down oncogene signals." 2003, THE ONCOLOGIST. 2003, VOL. 8 SUPPL 3, PAGE(S) 30 - 38 , XP002349948 ISSN: 1083-7159 * page 30 - page 38 ***

EP 1 611 890 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

BACKGROUND OF THE INVENTION

This invention relates to diagnostics, prognostics, and treatments for cancer based on the detection of molecular markers and/or gene expression analysis.

Some molecules, such as Ras, that are implicatext in cancers must be farnesylated by the farnesyl transferase enzyme to interact with the inner leaflet of the plasma membrane of the cell and become involved in various signaling pathways. However, Ras is not the only such protein implicated in cancer. Farnesyl transferase inhibitors (FTIs) are therapeutic agents that inhibit the covalent attachment of the carbon farnesyl moieties to the C-terminal CAAX motif of proteins. They have utility in cancer and proliferative disorder treatment such as hermatological malignancies. Hematological malignancies such as leukemias, lymphomas, and myclomas (e.g., acute myeloid leukemia; AML) are among the diseases that can most beneficially be addressed with FTIs. Solid tumors such as breast cancer and glioblastomas may also be treated with FTIs. WO 03/038129 describes methods for treating Acute Meylord Leukemia using and FTI. Also described therein are methods for monitoring patient therapy and selecting a course of therapy which involve analysing The presence of the LCB oncogene.

As is true in the case of many treatment regimens, some patients respond to treatment with FTIs and others do not. Prescribing the treatment to a patient who is unlikely to respond to it is not desirable. Thus, it would be useful to know how a patient could be expected to respond to such treatment before a drug is administered so that non-responders would not be unnecessarily treated and so that those with the best chance of benefiting from the drug are properly treated and monitored. Further, of those who respond to treatment there may be varying degrees of response. Treatment with therapeutics other than FTIs or treatment with therapeutics in addition to FTIs may be beneficial for those patients who would not respond to FTIs or in whom response to FTIs alone is less than desired

BRIEF SUMMARY OF THE INVENTION

The present invention relates to a method of determining whether a patient will respond to treatment with a farnesyl transferase inhibitor and a method of monitoring a patient being treated with a FTI by analyzing the presence of the LCB oncogene (SEQ ID NO 2), further including the analysis of the expression of a gene that is differentially modulated in the presence of FTI, wherein said gene is SEQ ID NO 29, wherein said patient has Acute Meyloid Leukemia (AML),

further described herein is a method of treating a cancer patient with an FTI. In one such method, the presence or absence of a molecular marker is determinative of whether the patient is likely to respond to the FTI. The patient is treated with an FTI if they are likely to respond. If the patient is not likely to respond to treatment with an FTI, then it may be withheld. As further described herein gene expression profiles are obtained for a set of genes that are predictive of FTI response. The presence of a molecular marker and gene expression profiles are used in combination to determine likelihood of response to FTI treatment. In yet another aspect herein described expression profiles are used in combination with leukemic blast counts and/or leukemic cell antigen expression to determine likelihood of response to FTI treatment. In yet another aspect the assay samples arc obtained from bone marrow.

As described herein a cancer patient is monitored for treatment with an FTI in which the patient's gene expression profile and/or the presence of a molecular marker is analyzed to determine whether the patient is responding to the FTI and treating a patient with the FTI if they are likely to respond in a desirable fashion.

In yet another aspect described herein a patient is treated if the gene expression profile shows modulation of one or more particular genes indicative of FTI responders.

In another aspect of the invention, a patient is treated if the gene expression profile shows modulation of one or more particular genes indicative of FTI responders and either a bone marrow blast cell count of 0 - 60%, or the presence of less than 60% of cells having positive expression of the CD33 cell surface antigen and/or the presence of less than 10% of cells having positive expression of the CD34 cell surface antigen.

In yet another aspect described herein the molecular marker is one or more of the following: AHR, MINA53, IDS, GPR105, TEM6, TNFSF13, SVIL, C6orf56, FRAG1, GOSR1, KIAA1036, BTG3, MAPK8IP3, LILRB3, ARHH and NPTX2. The marker can also be OPN3 and/or IL3RA in combination with one or more of the above.

In another aspect of the invention, the FTI is a quinilone or quinoline derivative. In yet another aspect of the invention, the FTI is (R)-6-[amino(4-chlorophenyl) (1-methyl-1H-imidazol-5-yl)methyl]-4-(3-chlorophenyl)-1-methyl-2(1H)-quinolinone).

Articles used in practicing the methods are also an aspect of the invention. Such articles include gene expression profiles or representations of them. The representations can be fixed in computer readable media. Other articles according to the invention include nucleic acid arrays used to determine the gene expression profiles of the invention and devices and components for practicing other nucleic acid detection technologies.

Kits are also described herein. Such kits include reagents for detecting the expression of genes and/or the presence of a molecular marker that distinguish responders from non-responders to FTI treatment. The kits can include instructions.

Further described herein is a method of treating a cancer patient comprises administering an FTI and a therapeutic composition that modulates the MAPK/ERK signaling pathways, TGFβ, WNT, Rho, or apoptotic pathways.

The patient may be treated with an FTI and a therapeutic composition selected from the group consisting of tyrosine kinase inhibitors, MEK kinase inhibitors, PI3K kinase inhibitors, MAP kinase inhibitors, apoptosis modulators and combinations thereof.

The gene expression profile and/or the presence or absence of a molecular marker of a patient may be analyzed to determine whether the patient would likely benefit from the combination of an FTI and another drug. The patient is then treated with such combination or, if the patient is unlikely to respond to an FTI, the patient is treated with another drug such as one selected from the group consisting of tyrosine kinase inhibitors, MEK kinase inhibitors, PI3K kinase inhibitors, MAP kinase inhibitors, apoptosis modulators and combinations thereof.

The gene expression profile and/or the presence of a molecular marker of a patient may be analyzed to determine whether the patient would likely benefit from the combination of an FTI and another form of therapy. The patient is then treated with such combination or, if the patient is unlikely to respond to an FTI, the patient is treated with another therapy without the inclusion of an FTI.

BRIEF DESCRIPTON OF DRAWINGS

Fig. 1 is a graphic presentation of a Kaplan-Meier analysis of patients with high and low CD33 antigen expression.

Fig. 2 is a graphic presentation of a Kaplan Meier analysis of patients with high and low blast counts.

Fig. 3A is a graphic presentation of a Kaplan-Meicr survival analysis of patients using clinieal response classifiers, 3B is a graphic presentation of a Kaplan-Meier survival analysis of patients using oncol.HC gene expression to classify patients. 3C is a graphic presentation of a Kaplan-Meier survival analysis of patients using oncoLBC and AHR gene expressions to classify patients.

Fig. 4 depicts the identification of a minimal set of predictive markers. In Panel A an LOOCV was performed using a sensitivity of 100%. Independent classifiers were tested that contained from one to 19 genes. The resulting error rate is plotted. Panel B shows a 2x2 table generated from a LOOCV performed using the 3-gene signature as a classifier on the responders (R) and non-responders (NR). Panel C shows the scores generated from the 3-gene classifier. The p-value indicates a significant difference in gene expression between the response groups. Panel D depicts the Kaplan-Meier curves performed on patients classified by the 3-gene signature as being responders and non-responders. Median survival times are also indicated.

Fig. 5 depicts a Kaplan-Meier analysis performed on patients classified by the 3-gene signature as being responders and non-responders. The survival curve of patients who were clinically defined as non-responders but classified as responders using the 3-gene signature is shown. Median survival times are also indicated.

DETAILED DESCRIPTION OF THE INVENTION

The therapeutic agents referred to in this specification are FTIs. They take on a multitude of forms but share the essential inhibitory function of interfering with or lessening the farnesylation of proteins implicated in cancer and proliferative diseases. Preferably, the FTIs are those indicated for the treatment of solid tumors such as glioblastoma and breast cancer. More preferably, the FTIs are indicated for the treatment of hematological malignancies such as leukemias, lymphomas, and myelomas. Most preferably, the FTIs are contemplated for use in AML, myelodysplastic syndrome (MDS), chronic myeloid leukemia (CML), chronic myelomonocytic leukemia (CMML), and multiple myeloma (MM). In the case of hematological malignancies, a patient who responds to an FTI is one in whom a reduction of more than 50% of blast cells is seen in bone marrow following treatment with the FTI. In solid tumors, a patient who responds to an FTI is one in whom their tumor ceases to grow. Response in patients with solid tumors can alternatively be measured according to RECIST (Response Evaluation Criteria in Solid Tumors) criteria as such term is commonly used in oncology.

Numerous FTIs are within the scope of the invention and include those described in US Patents: 5,976,851 to Brown et al.; 5,972,984 to Anthony et al.; 5,972,966 to deSolms; 5,968,965 to Dinsmore et al.; 5,968,952 to Venet et al. ; 6,187,786 to Venet et al.; 6,169,096 to Venet et al.; 6,037,350 to Venet et al.; 6,177,432 to Angibaud ct al.; 5,965,578 to Graham et al.; 5,965,539 to Sebti et al.; 5,958,939 to Afonso et al.; 5,939,557 to Anthony et al.; 5,936,097 to Commercon et al.; 5,891,889 to Anthony et al.; 5,889,053 to Baudin et al.; 5,880,140 to Anthony; 5,872,135 to deSolms; 5,869,682 to deSolms; 5,861,529 to Baudoin; 5,859,015 to Graharn et al.; 5,856,439 to Clere; 5,856,326 to Anthony et al.; 5,852,010 to Graham et al.; 5,843,941 to Marsters et al.; 5,807,852 to Doll; 5,780,492 to Dinsmore et al.; 5,773,455 to Dong et al.

; 5,767,274 to Kim et al.; 5,756,528 to Anthony et al.; 5,750,567 to Baudoin et al.; 5,721,236 to Dishop ct al.; 5,700,806 to Doll et al.; 5,661,161 to Anthony et al.; 5,602,098 to Sehti et al.; 5,585,359 to Breslin et al.; 5,578,629 to Ciccarone et al.; 5,534,537 to Ciccarone et al.; 5,532,359 to Marsters et al.; 5,523,430 to Patel et al.; 5,501,212 to de Solms et al. ; 5,491,164 to deSolms et al.; 5,420,245 to Brown et al.; and 5,238,922 to Graham et al.

Non-peptidal, so-called "small molecule" therapeutics are preferred. More preferred FTIs are quinolines or quinoline: derivatives such as: 7-(3-chlorophenyl)-9-[(4-chlorophenyl)-1H-imidazol-1-yl)methyl]-2,3-dihydro-1H,5H-benzo[ij]quinolizin-5-one, 7-(3-chlorophenyl)-9-[(4-chlorophenyl)-1H-imidazol-1-yl)methyl]-1,2-dihydro-4H pyrrolo[3,2,1-ij] quinoline-4-one, 8-[amino(4-chlorophenyl)(1-methyl-1H-imidazol-5-yl),methyl]-6-(3-chlorophenyl)-1,2-dihydro-4H-pyrrolo[3,2,1-ij]quinolin-4-one, and 8-[amino(4-chlorophenyl)(1-methyl-1H-imidazol-5-yl)methyl]-6-(3-chlorophenyl)-2,3-dihydro-1H,5H-benzo[ij]quinolizin-5-one.

The most preferred FTI is (R)-6-[amino(4-chlorophenyl)(1-methyl-1H-imidazol-5-yl)methyl]-4-(3-chlorophenyl)-1-methyl-2(1H)-quinolinone), described in US Patent 6,420,387 to Venet et al. as the (+) enantiomer.

Another preferred FTI is (-)-5-(3-chlorophenyl)-α-(1-chlorophenyl)-α-(1-methyl-1*H*-imidazol-5-yl)tetrazolo[1,5-a]quinazoline-7-methanamine and its pharmaceutically acceptable acid addition salts, described in WO 01/98302.

Other useful FTIs include Arglabin i.e. 1(R)-10-epoxy-5(S),7(S)-guaia-3(4),11(13)-dien-6,12-olide (WO 98/28303); perrilyl alcohol (WO 99/45912); SCH-66336, i.e. (+)-(R)-4-[2-[4-(3,10-dibrunio-8-chloro-5,6-dihydro-11H-benzo[5,6]cyclohepta[1,2-b]pyridin-11 yl)piperidin-1-yl]-2-oxoethyl]piperidine-1-carboxamide, (US Patent 5874442); L778123, i.e. 1-(3-chlorophenyl)-4-[1-(4-cyanobenzyl)-5-imidazolylmethyl]-2-piperazinone, (WO 00/01691); compound 2(S)-[2(S)-[2(R)-amino-3-mercapto]propylamino-3(S)-methyl]-pentyloxy-3-phenylpropionyl-methionine sulfone (WO 94/10138); BMS 214662, i.e. (R)-2,3,4,5-tetrahydro-1-(1H-imidazol-4-ylmethyl)-3-(phenylmethyl)-4-(2-thienylsulphonyl)-1H-1,4-benzodiazapine-7-carbonitrile, (WO 97/30992); CP 609754, i.e. N-(3-ethynylphenyl)-6,7-bis(2-methoxyethoxy)-4-quinazolinamine (US Patent 5,747,498); and 6-[amino-(4-chloro-phenyl)-(3-methyl-3H-imidazol-4-yl)-methyl]-4-(3-ethynyl-phenyl)-1-methyl-1H-quinolin-2-one (WO 00/12499):

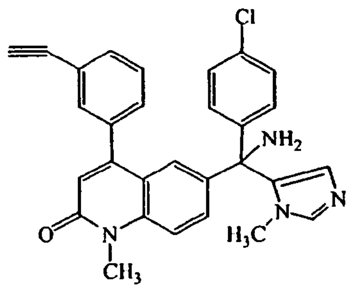

The mere presence of nucleic acid sequences having the potential to express proteins or peptides ("genes") within the genome is not determinative of whether a protein or peptide is expressed in a given cell. Whether or not a given gene capable of expressing proteins or peptides or transcribing RNA does so and to what extent such expression or transcription occurs, if at all, is determined by a variety of complex factors. Nevertheless, assaying gene expression can provide useful information about the cellular response to a given stimulus such as the introduction of a drug or other therapeutic agent. Relative indications of the degree to which genes are active or inactive can be found in such gene expression profiles. In some instances, the presence of a molecular marker can, by itself or with the use of gene expression information, provide useful information about treatment efficacy too. The gene expression profiles and molecular markers of this invention are used to identify and treat patients who will likely benefit from FTI therapy or exclude patients from FTI therapy where the patient likely would experience little or no beneficial response to the drug or therapy.

Cancers, including hematological malignancies, typically arise from mutations in a variety of genes. The same type of cancer may arise as a result of, or coincident with, one or more mutations that differ from those of another patient having the same type of cancer. The fact that there are often multiple molecular bases underlying the same cancers is consistent with the observation that some therapies that affect one patient do not necessarily equally affect another patient with the same type of cancer. Further, from a diagnostic point of view, the presence of particular mutations such as translations, deletions, or SNPs can have powerful implications. In some instances, such molecular markers are themselves useful indicators for diagnosis, prognosis, or treatment response determinations. This is particularly true where the molecular mutations can be associated with response to particular treatments. As described herein cancers coincident with the absence of the LBC (lymphoid blast crisis) oncogene (also known as AKAPI3, SUQ ID NO: 2) respond to FTI treatment. Therefore, the expression of this gene, the lack of expression of the gene and its presence or absence are useful in predicting resistance to FTI treatment prior to actually prescribing such treatment.

The LBC oncogene is a chimera derived from the fusion of an LBC proto-oncogene SEQ ID NOs: 23-27) on chromosome 15q with an unrelated sequence originating in chromosome 7q. The truncation of the proto-oncogcne at the C-terminus of the sequence results in the gene gaining transforming ability. This truncation could also arise from mechanisms other than a translocation. For example, aberrant splicing could result in RNA transcripts with C-terminus traneations. The gene has a number of expression products including mRNA and a protein (SEQ ID NO: 28, Human LBC protein, Genbank Accession numher GI: 29791897). While the precise manner in which the LBC oncogene functions is not completely understood, it is clear that it can be present in a range of tissues including skeletal musele, heart, lung, prostate, ovary, small intestine, and hematopoietic cells. Treatment of cancers originating in tissues where the oncogene could be manifested (but is not) is within the scope of this invention.

There is great flexibility available in formatting the assays of this invention because the gene is the product of a truncation and because it produces recognizable expression products. Not only can the absence of the gene or its products be used, but so too can detection of the modulated expression of this gene. Thus, a gene expression profile can include this gene. Preferably, the absence or modulation of the gene is used as an indicator of FTI treatment response in hematological malignancies, more preferably in leukemias, and most preferably in AMI.

Any suitable method of detection may be used to identify the LBC oncogene as a molecular marker. The presence of the molecular marker is indicative of a poor prognosis for treatment with an FTI and its absence is indicative of a greater likelihood of response to such treatment. Methods useful for detecting the presence of the LBC oncogene include any method for detecting known mutant genes or sequences including, without limitation, the single strand conformation polymorphism technique, chemical and thermal denaturing gradient analysis, chemical and enzymatic cleavage methods, mass spectrometry, RFLP and allele specific amplification, ratiometric PCR, bead-based and micro-array-based systems as well as in situ hybridization, heteroduplex analysis, and microarray analysis, ELISA, Western, fluorescence activated cell sorting (FACS), antibody-based techniques, methylation-based PCR, and SNP detection.

The most preferred method for detecting the presence or absence of the LBC oncogene is via PCR. In this method, cells are first obtained from the patient according to routine sample preparation methods. Where the malignancy is hematological, a simple peripheral blood sample or a bone marrow sample is preferable. RNA is then extracted according to well-accepted procedures and amplified as follows. Target sequences are amplified using, e.g., 250 nM of primers and 250 nM of TaqMan® probe in ABI TaqMan® buffer. Thermal cycling is conducted at 50°C for 2 minutes, 95°C for 10 minutes, followed by 50 cycles of 95°C for 15 minutes and 62°C for 1 minute. Examples of the primers and probe are shown in Table 1.

This technique measures the amount of LBC transcript present in the sample. Measured quantities can be normalized by running similar RT-PCR experiments in which the same samples are used to amplify endogenous control genes such as HPRT.

Table 1

| Name | Sequence (5'-3') | SEQ ID NO: |
|---|---|---|
| LBC forward | GGTCAGATGTTTGCCAAGGAA | 19 |
| LBC reverse | TCTTCAGAAACACACTCCCATCAC | 20 |
| LBC TaqMan® probe | TGAAACGGAAGAAGCTTGTA | 21 |

Another method for determining the presence or absence of the LBC oncogene is to assay the length of the RNA transcript. Since the LBC oncogene has a 3' translocation, the transcript length will be shorter than the LBC proto-oncogene transcript. This end point is favored as a diagnostic. To diagnose the transcript size a forward primer homologous to both the proto- and onco LBC transcripts is used (e.g. LBC forward primer from above Table 1) in conjunction with a reverse primer homologous to the universal polyA tail of RNA transcripts. Preferably initial cDNA synthesis will incorporate an additional unique sequence tag 3' of the polyA sequence to confer additional specificity for the PCR reaction.

If the genomic translocation is being measured then genomic DNA will be isolated using standard techniques and PCR primers used that are specific for the LBC oncogene. For example, the forward primer homologous for both the onco- and proto-LBC genes could be used in conjunction with the polyA sequence as described above. Alternatively, the reverse primer could be homologous to the 3' translocated sequence. The readout would be the size of the amplicon where presence of the oncogene is consistent with a shorter product than the proto-oncogene, or presence or absence of an oncoLBC-specific amplicon.

Assays for the LBC oncogene status of the cell also can determine normal/abnormal tissue distribution for diagnostic purposes using techniques such as immunohistochemical analysis (IHC). For example, the antibodies to LBC protein may be used in conjunction with both fresh-frozen and formalin-fixed, paraffin-embedded tissue blocks prepared

for study by IHC. Each tissue block may consist of 50 mg of residual "pulverized" tumor.

Briefly, frozen-sections may be prepared by rehydrating 50 ng of frozen pulverized tumor at room temperature in phosphate buffered saline (PBS) in small plastic capsules; pelleting the particles by centrifugation; resuspending them in a viscous embedding medium (OCT); inverting the capsule and pelleting again by centrifugation; snap-freezing in -70°C isopentane; cutting the plastic capsule and removing the frozen cylinder of tissue; securing the tissue cylinder on a cryostat microtome chuck; and cutting 25-50 serial sections containing intact tumor cells.

Permanent-sections may be prepared by a similar method involving rehydration of the 50 mg sample in a plastic microfuge tube; pelleting; resuspending in 10% formalin for 4 hr fixation; washing/pelleting; resuspending in warm 2.5% agar; pelleting; cooling in ice water to harden the agar; removing the tissue/agar block from the tube; infiltrating and embedding the block in paraffin; and cutting up to 50 serial permanent sections.

For the IHC assay, the sections are overlaid with a blocking solution containing: 3% bovine serum albumin (BSA) in PBS or other blocking reagents. The blocking reagents include non-specific serum or dry milk. Blocking is allowed to proceed for 1 hr at room temperature. Anti-LBC protein antibody is diluted with PBS buffer containing 3% BSA, 0.1 % TritonX™-100 and t-octylphenoxypolyethoxyethanol, at a ratio of 1:100. The sample sections are generally overlaid with the antibody solution for 16 hr at 4°C. The duration and temperature conditions may be varied according to the antibody selected and the material tested. The optimal conditions are determined empirically. The antibody treated sections are then washed three times in PBS for 15 min. each to remove unbound antibody and then overlaid with PBS containing 3% BSA and a secondary antibody at a dilution of 1:2000. The secondary antibodies may be coupled to a chromogenic enzyme such as: horseradish peroxidase, alkaline phosphatase, fluorescein iso-thiocyanate, or other suitable enzymes. Alternatively, the secondary antibody may be conjugated to biotin and used in conjunction with chromophore-labeled avidin.

Another exemplary method for detecting the presence of the LBC oncogene is via in situ hybridization. Generally, in situ hybridization comprises the following major steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization and (5) detection of the hybridized nucleic acid fragments. The reagent used in each of these steps and the conditions for use vary depending on the particular application.

In this case, a hybridization solution comprising at least one detectable nucleic acid probe capable of hybridizing to the LBC oncogene (at its chromosomal locus) is contacted with the cell under hybridization conditions. Any hybridization is then detected and compared to a predetermined hybridization pattern from normal or control cells. Preferably, the probes are alpha-centromeric probes. Such probes can be made commercially available from a number of sources (e.g., from Visys Inc., Downers Grove, IL). In a preferred embodiment, the hybridization solution contains a multiplicity of probes, specific for an area on the chromosome that corresponds to the translocation of the sequences that make up the chimera (e.g., 15q24-25).

Hybridization protocols suitable for use with the methods of the invention are described, e.g., in Albertson (1984) EMBO J. 3:1227-1234; Pinkel (1988) Proc. Natl. Acad. Sci. USA 85:9138-9142; EPO Pub. No. 430,402; and Methods in Molecular Biology, Vol. 33: In Situ Hybridization Protocols, Choo, ed., Humana Press, Totowa, NJ (1994), etc. In one particularly preferred embodiment, the hybridization protocol of Pinkel et al. (1998) Nature Genetics 20: 207-211 or of Kallioniemi (1992) Proc. Natl. Acad. Sci. USA 89:5321-5325 is used. Methods of optimizing hybridization conditions are well known (see, e.g., Tijssen (1993) Laboratory Techniques in Biochemistry and Molecular Biology, Vol. 24: Hybridization With Nucleic Acid Probes, Elsevier, NY).

As described herein, background signal is reduced by the use of a detergent (e.g., C-TAB) or a blocking reagent (e.g., sperm DNA, cot-1 DNA, etc.) during the hybridization to reduce non-specific binding. Preferably, the hybridization is performed in the presence of about 0.1 to about 0.5 mg/ml DNA (e.g., cot-1 DNA).

The probes may be prepared by any method known in the art, including synthetically or grown in a biological host. Synthetic methods include oligonucleotide synthesis, riboprobes, and PCR.

The probe may be labeled with a detectable marker by any method known in the art. Methods for labeling probes include random priming, end labeling, PCR and nick translation. Enzymatic labeling is conducted in the presence of nucleic acid polymerase, three unlabeled nucleotides, and a fourth nucleotiode which is either directly labeled, contains a linker arm for attaching a label, or is attached to a bapten or other molecule to which a labeled binding molecule may bind. Suitable direct labels include radioactive labels such as $^{32}P$, $^{3}H$, and $^{35}S$ and non-radioactive labels such as fluorescent markers, such as fluoreseein. Texas Red, AMCA blue, lucifer yellow, rhodamine, and the like; cyanin dyes which are detectable with visible light; enzymes and the like. Labels may also be incorporated chemically into DNA probes by bisulfite-mediated transamination or directly during oligonucleotide synthesis.

Fluorescent markers can readily be attached to nucleotides with activated linker arms incorporated into the probe. Probes may be indirectly labeled by the methods disclosed above, by incorporating a nucleotide covalently linked to a hapten or other molecule such as biotin or digoxygenin, and performing a sandwich hybridization with a labeled

antibody directed to that hapten or other molecule, or in the case of biotin, with avidin conjugated to a detectable label. Antibodies and avidin may be conjugated with a fluorescent marker, or with an enzymatic marker such as alkaline phosphatase or horseradish peroxidase to render them detectable. Conjugated avidin and antibodies are commercially available from companies such as Vector Laboratories (Burlingame, CA) and Bochringer Mannheim (Indianapolis, IN).

The enzyme can be detected through a colorimetric reaction by providing a substrate for the enzyme. In the presence of various substrates, different colors are produced by the reaction, and these colors can be visualized to separately detect multiple probes. Any substrate known in the art may be used. Preferred substrates for alkaline phosphatase include 5-bromo-4-chloro-3-indolylphosphate (BCIP) and nitro blue tetrazolium (NBT). The preferred substrate for horseradish peroxidase is diaminobenzoate (DAB).

Fluorescently labeled probes suitable for use in the in situ hybridization methods of the invention are preferably in the range of 150-500 nucleotides long. Probes may be DNA or RNA, preferably DNA.

Hybridization of the detectable probes to the cells is conducted with a probe concentration of 0.1-500 ng/μl, preferably 5-250 ng/μl. The hybridization mixture will preferably contain a denaturing agent such as formamide. In general, hybridization is carried out at 25°C-45°C, more preferably at 32°C-40°C, and most preferably at 37°C-38°C. The time required for hybridization is about 0.25-96 hours, more preferably 1-72 hours, and most preferably for 4-24 hours. Hybridization time will vary based on probe concentration and hybridization solution content which may contain accelerators such as hnRNP binding protein, trialkyl ammonium salts, lactams, and the like. Slides are then washed with solutions containing a denaturing agent, such as formamide, and decreasing concentrations of sodium chloride or in any solution that removes unbound and mismatched probe.

The temperature and concentration of salt will vary depending on the stringency of hybridization desired. For example, high stringency washes may be carried out at 42°C-68°C, while intermediate stringency may be in the range of 37°C-55°C, and low stringency may be in the range of 30°C-37°C. Salt concentration for a high stringency wash may be 0.5-1 times SSC (0.15M NaCl, 0.015M Na citrate), while medium stringency may be 1-4 times, and low stringency may be 2-6 times SSC.

The detection incubation steps, if required, should preferably be carried out in a moist chamber at 23°C-42°C, more preferably at 25°C-38°C and most preferably at 37-38°C. Labeled reagents should preferably be diluted in a solution containing a blocking reagent, such as BSA, non-fat dry milk, or the like. Dilutions may range from 1:10-1:10,000, more preferably 1:50-1:5,000, and most preferably at 1:100-1:1,000. The slides or other solid support should be washed between each incubation step to remove excess reagent.

Slides may then be mounted and analyzed by microscopy in the case of a visible detectable marker, or by exposure to autoradiographic film in the case of a radioactive marker. In the case of a fluorescent marker, slides are preferably mounted in a solution that contains an antifade reagent, and analyzed using a fluorescence microscope. Multiple nuclei may be examined for increased accuracy of detection.

Additionally, assays for the expression product of the LBC oncogene can also be used to determine whether the LBC oncogene mutation has occurred. Most preferably, such assays are immunoassays. Immunoassays, in their most simple and direct sense, are binding assays. Certain preferred immunoassays are the various types of enzyme linked immunosorbent assays (ELISAs) and radioimmunoassays (RIA) known in the art. IHC detection using tissue sections is also particularly useful.

In one exemplary ELISA, anti-oncoLBC protein-specific antibodies are immobilized onto a selected surface exhibiting protein affinity, such as a well in a polystyrene microtiter plate. Then, a test composition containing the desired antigen, such as a clinical sample, is added to the wells. After binding and washing to remove non-specifically bound immune complexes, the bound antigen may be detected. Detection is generally achieved by the addition of another antibody, specific for the desired antigen, that is linked to a detectable label. This type of ELISA is a simple "sandwich ELISA". Detection may also be achieved by the addition of a second antibody specific for the desired antigen, followed by the addition of a third antibody that has binding affinity for the second antibody, with the third antibody being linked to a detectable label.

Variations of ELISA techniques are well known. In one such variation, the samples containing the desired antigen are immobilized onto the well surface and then contacted with the antibodies of the invention. After binding and appropriate washing, the bound immune complexes are detected. Where the initial antigen specific antibodies are linked to a detectable label, the immune complexes may be detected directly. Again, the immune complexes may be detected using a second antibody that has binding affinity for the first antigen specific antibody, with the second antibody being linked to a detectable label.

Competitive ELISAs are also possible in which test samples compete for binding with known amounts of labeled antigens or antibodies. The amount of reactive species in the unknown sample is determined by mixing the sample with the known labeled species before or during incubation with coated wells. The presence of reactive species in the sample acts to reduce the amount of labeled species available for binding to the well and thus reduces the ultimate signal

Antigen or antibodies may also be linked to a solid support, such as plates, beads, dipsticks, membranes or column matrices, and the sample to be analyzed applied to the immobilized antigen or antibody. In coating a plate with

either antigen or antibody, one will generally incubate the wells of the plate with a solution of the antigen or antibody, either overnight or for a specified period. The wells of the plate will then be washed to remove incompletely adsorbed material. Any remaining available surfaces of the wells are then "coated" with a nonspecific protein that is antigenically neutral with regard to the test antisera. These include BSA, casein and solutions of milk powder. The coating allows for blocking of nonspecific adsorption sites on the immobilizing surface and thus reduces the background caused by non-specific binding of antisera onto the surface.

In ELISAs, it is customary to use a secondary or tertiary detection means rather than a direct procedure. Thus, after binding of the antigen or antibody to the well, coating with a non-reactive material to reduce background, and washing to remove unbound material, the immobilizing surface is contacted with the clinical or biological sample to be tested under conditions effective to allow immune complex (antigen/antibody). This can include diluting the antigens and antibodies with solutions such as BSA, bovine gamma globulin (BGG) and PBS/Tween. These agents also tend to assist in the reduction of nonspecific background. Detection of the immune complex then requires a labeled secondary binding ligand or antibody, or a secondary binding ligand or antibody in conjunction with a labeled tertiary antibody or third binding ligand.

Following all incubation steps in an ELISA, the contacted surface is washed so as to remove non-complexed material. Washing often includes washing with a solution of PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, contacting and incubating the first or second immune complex with a urease, glucose oxidase, alkaline phosphatase or hydrogen peroxidase-conjugated antibody for a period of time and under conditions that favor the development of further immune complex formation, e.g., incubation for 2 hours at room temperature in a PBS-containing solution such as PBS-Tween.

After incubation with the labeled antibody, and subsequent to washing to remove unbound material, the amount of label is quantified, e.g., by incubation with a chromogenic substrate such as urea and bromocresol purple and $H_2O_2$, in the case of peroxidase as the enzyme label. Quantification is then achieved by measuring the degree of color generation, e.g., using a visible spectra spectrophotometer. Alternatively, the label may be a chemiluminescent one. The use of such labels is described in US Patents 5,310,687, 5,238,808 and 5,221,605.

In embodiments of the invention in which gene expression is detected for determining response to FTIs, the use of gene expression portfolios is most preferred. A portfolio of genes is a set of genes grouped so that expression information obtained about them provides the basis for making a clinically relevant judgment such as a diagnosis, prognosis, or treatment choice. In this case, gene expression portfolios can be fashioned to help make therapeutic decisions regarding the use of FTIs in cancer patients.

It is most preferred to detect the expression of the LBC oncogene as part of a gene expression profile with one or more other genes whose differential expression is indicative of likelihood of response to FTI treatment. One or more of the following genes can also be used, most preferably, in combination with the LBC oncogene: AHR, MINA53, IDS, OPN3, GPR105, TEM6, TNFSF13, SVIL, IL3RA, C6orf56, FRAG1, GOSR1, KIAA1036, RTG3, MAPK81P3, LILRB3, ARHH, NPTX2 (SEQ ID NOs: 1, 3-18 and 29). OPN3 and IL3RA are used in combination with one or more other genes and preferably, the profile includes two or more of any of the genes. The most preferred gene expression profile detects the differential expression of LBC oncogene and AHR. A preferred set of three genes is LBC, AHR and MINA53. Variants, such as splice variants, of the aforementioned genes are also useful in this application.

Preferred methods for establishing gene expression profiles (including those used to arrive at the explication of the relevant biological pathways) include determining the amount of RNA that is produced by a gene that can code for a protein or peptide or transcribe RNA. This is best accomplished by reverse transcription PCR (RT-PCR), competitive RT-PCR, real time RT-PCR, differential display RT-PCR, Northern Blot analysis and other related tests. While it is possible to conduct these techniques using individual PCR reactions, it is often desirable to amplify copy DNA (cDNA) or copy RNA (cRNA) produced from mRNA and analyze it via microarray. A number of different array configurations and production methods are known to those of skill in the art and are described in US Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637

Microarray technology allows for the measurement of the steady-state mRNA level of thousands of genes simultaneously thereby presenting a powerful tool for identifying the effect of FTIs on cell biology and the likely effect of treatment based on analysis of such effects. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a tabeled probe used to detect a cDNA sequence from the sample that hybridizes

to a nucleic acid sequence at a known location on the microarray. Typically, the signal intensity is proportional to the cDNA quantity, and thus mRNA, expressed in the sample cells. A large number of such techniques are available and useful. Preferred methods can be found in US Patents 6,271,002 to Linsley, et al.; 6,718,122 to Friend, et al.; 6,218,114 to Peck, et al.; and 6,004,755 to Wang, et al.

Analysis of the expression levels is conducted by comparing such intensities. This is best done by generating a ratio matrix of the expression intensities of genes in a test sample versus those in a control sample. For instance, the gene expression intensities from a tissue that has been treated with a drug can be compared with the expression intensities generated from the same tissue that has not been treated with the drug. A ratio of these expression intensities indicates the fold-change in gene expression between the test and control samples.

Gene expression profiles can also be displayed in a number of ways. A common method is to arrange a ratio matrix into a graphical denclogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRINT" from Silicon Genetics, Inc. and "DISCOVERY" and "INFER" software from Partek, Inc.

The differentially expressed genes are either up regulated or down regulated in diseased cells following treatment with an FTI, or in responders versus non-responders prior to treatment, as deduced by an assessment of gene expression as described above. Up regulation and down regulation are relative terms meaning that a detectable difference (beyond the contribution of noise in the system used to measure it) is found in the amount of expression of the genes relative to some baseline. In this case, the baseline is the measured gene expression of the untreated diseased cell. The genes of interest in the treated discased cells are then either up regulated or down regulated relative to the bascline level using the same measurement method. Preferably, levels of up and down regulation are distinguished based on fold changes of the intensity measurements of hybridized microarray probes. A 1.5 fold difference is preferred for making such distinctions. That is, before a gene is said to be differentially expressed in treated versus untreated diseased cells, the treated cell is found to yield at least 1.5 limes more, or 1.5 times less intensity than the untreated cells. A 1.7 fold difference is more preferred and a 2 or more fold difference in gene expression measurement is most preferred.

One method described herein involves comparing gene expression profiles for various genes to determine whether a person is likely to respond to the use of a therapeutic agent. Having established the gene expression profiles that distinguish responder from non-responder, the gene expression profiles of each are fixed in a medium such as a computer readable medium as described below. A patient sample is obtained that contains diseased cells (such as hematopoietic blast cells in the case of AMI) is then obtained. Most preferably, the samples are of bone marrow and are extracted from the patient's sternum or diac crest according to routine methods before the patient has been treated with drug. Preferably the bone marrow aspirate is processed to enrich for leukemic blast cells using routine methods. Sample RNA is then obtained and amplified from the diseased patient cells and a gene expression profile is obtained, preferably (in the case of a large gene portfolio) via micro-array, for genes in the appropriate portfolios. The expression profiles of the samples are then compared to those previously determined as responder and non-responder. If the sample expression patterns are consistent with an FTI responder expression pattern then treatment with an FTI could be indicated (in the absence of countervailing medical considerations). If the sample expression patterns are consistent with an FTI non-responder expression pattern then treatment with an FTI would not he indicated. When a small number of genes are used in the portfolio such as when the single gene, LBC oncogene, is used, a simple nucleic acid amplification and detection scheme is the most preferred method of measuring gene modulation. In such a case, PCR, NASBA, rolling circle, LCR, and other amplification schemes known to skilled artisans can be used with PCR being most preferred. Where the portfolios include a large number of genes or it is desirable to measure the expression of numerous other genes then it is preferred to assess the expression patterns based on intensity measurements of microarrays as described above.

In similar fashion, gene expression profile analysis can be conducted to monitor treatment response. In one aspect of this method, gene expression analysis as described above is conducted on a patient treated with an FTI at various periods throughout the course of treatment. If the gene expression patterns are consistent with a responder then the patient's therapy is continued. If it is not, then the patient's therapy is altered as with additional therapeutics such as tyrosine kinase inhibitor, changes to the dosage, or elimination of FTI treatment. Such analysis permits intervention and therapy adjustment prior to detectable clinical indicia or in the face of otherwise ambiguous clinical indicia.

With respect to the molecular markers of the invention, a number of other formats and approaches are available for diagnostic use. Methylation of genomic regions can affect gene expression levels. For example, hypermethylation of gene promoter regions can constitutively down-regulate gene expression whereas hypomethylation can lead to an increase in steady-state mRNA levels. As such, detection of methylated regions associated to genes predictive of drug response can be used as an alternative method for diagnosing gene expression levels. Such methods are known to those skilled in the art. Alternatively, single nucleotide polymorphisms (SNPs) that are present in promoter regions can

also affect transcriptional activity of a gene. Therefore, detection of these SNPs by methods known to those skilled in the art can also be used as a diagnostic for detecting genes that are differentially expressed between responders and non-responders.

The distinction between responder and non-responder can also be advantageously made with the additional assay of the proportion of bone marrow leukemic blasts present prior to treatment and/or the presence of cell surface antigens such as CD33 and/or CD34. Low expression of CD33 and CD34 surface antigens indicates an increased likelihood of response to FTI treatment. This is most conveniently measured as the percent of cells in a sample that express such antigens with responders having about 60% or less of such cells expressing CD33 and about 15% or less expressing CD34. A percentage of such cells exceeding 60% expressing CD33 or 15% CD34 antigens indicates that the patient is likely a non-responder to FTI treatment. Further, a sample taken as described above in which the percentage of cells that are blast cells is less than about 60% is likely to respond to FTI treatment while those with blast cell counts that exceed this percentage is not. Determinations of percent of CD33+, CD34+, and blast cell count are conducted according to any of the well known methods and are most efficiently conducted using standard pathological tests and preparations conducted in most clinical laboratories.

Articles described herein are representations of the gene expression profiles useful for treating, diagnosing, prognosticating, staging, and otherwise assessing diseases. Preferably they are reduced to a medium that can be automatically read such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. Clustering algorithms such as those incorporated in "DISCOVERY" and "INFER" software from Partek, Inc. mentioned above can best assist in the visualization of such data.

Additional articles described herein are kits for conducting the assays described above. Each such kit would preferably include instructions in human or machine readable form as well as the reagents typical for the type of assay described. These can include, for example, nucleic acid arrays (e.g. cDNA or oligonucleotide arrays), as described above, configured to discern the gene expression profiles of the invention. They can also contain reagents used to conduct nucleic acid amplification and detection including, for example, reverse transcriptase, reverse transcriptase primer, a corresponding PCR primer set, a thermostable DNA polymerase, such as Taq polymerase, and a suitable detection reagent(s), such as, without limitation, a scorpion probe, a probe for a fluorescent probe assay, a molecular beacon probe, a single dye primer or a fluorescent dye specific to doublestranded DNA, such as ethidium bromide. Kits for detecting surface antigens contain staining materials or are antibody based including components such as buffer, anti-antigenic antibody, detection enzyme and substrate such as Horse Radish Peroxidase or biotin-avidin based reagents. Kit components for detecting blast cells generally include reagents for conducting flow cytometry, blast cell adhesion assays, and other commonly practiced blast cell assays.

As described in the pending application of the inventor entitled, METHODS FOR ASSESSING AND TREATING LEUKEMIA, and filed October 30, 2002 (Serial Number 10/283,975), in addition to the FTIs that are preferred, the preferred drugs of this invention are those that modulate the MAPK/ERK signaling pathways, TGFβ, WNT or apoptotic pathways. These include, without limitation, tyrosine kinase inhibitors, MEK kinase inhibitors, P13K kinase inhibitors, MAP kinase inhibitors, apoptosis modulators and combinations thereof. Exemplary drugs that are most preferred among these are the "GLEEVEC" tyrosine kinase inhibitor of Novartis, U-0126 MAP kinase inhibitor, PD-098059 MAP kinase inhibitor, SB-203580 MAP kinase inhibitor, and antisense, ribozyme, and DNAzyme, Bcl-XL, and anti-apoptotics. Examples of other useful drugs include, without limitation, the calanolides of US Patent 6,306,897; the substituted bicyclics of US Patent 6,284,764; the indolines of US Patent 6,133,305; and the antisense oligonucleotides of US Patent 6,271,210.

The FTI may also be used in combination with other conventional anti-cancer agents for example selected from platinum coordination compounds for example cisplatin or carboplatin, taxane compounds for example paclitaxel or docetaxel, camptothecin compounds for example irinotecan or topotecan, anti-tumor vinca alkaloids for example vinblastine, vincristine or vinorelbine, anti-tumor nucleoside derivatives for example 5-fluorouracil, gemcitabine or capecitabine, nitrogen mustard or nitrosourea alkylating agents for example cyclophosphamide, chlorambucil, carmustine or lomustine, anti-tumor anthracycline derivatives for example daunorubicin, doxorubicin or idarubicin; HER2 antibodies for example trastzumab; and anti-tumor podophyllotoxin derivatives for example etoposide or teniposide; and antiestrogen agents including estrogen receptor antagonists or selective estrogen receptor modulators preferably tamoxifen, or alternatively toremifene, droloxifene, faslodex and raloxifene, or aromatase inhibitors such as exemestane, anastrozole, letrazole and vorozole.

The FTI can be administered to a patient as described above in conjunction with irradiation; such treatment may be especially beneficial as FTIs can act as radiosensitisers for example as described in WO 00/01411, enhancing the therapeutic effect of such irradiation. Irradiation means ionizing radiation and in particular gamma radiation, especially that emitted by linear accelerators or by radionuclides that are in common use today. The irradiation of the tumor by

radionuclides can be external or internal.

Preferably, FTI administration commences up to one month, in particular up to 10 days or a week, before the irradiation of the tumor. Additionally, it is advantageous to fractionate the irradiation of the tumor and maintain FTI administration in the interval between the first and the last irradiation session. The amount of FTI, the dose of irradiation and the intermittence of the irradiation doses will depend on a series of parameters such as the type of tumor, its location, the patients' reaction to chemo- or radiotherapy and ultimately is for the physician and radiologists to determine in each individual case. Thus, cancer therapy according to the inventive method also includes, for a host harboring a tumor, administering a radiation-sensitizing effective amount of an FTI according to the invention before, during or after administering radiation to said host in the proximity to the tumor.

As noted, the drugs described herein can be therapeutics directed to gene therapy or antisense therapy or RNA interference. Oligonucleotides with sequences complementary to an mRNA sequence can be introduced into cells to block the translation of the mRNA, thus blocking the function of the gene encoding the mRNA. The use of oligonucleotides to block gene expression is described, for example, in, Strachan and Read, Human Molecular Genetics, 1996, incorporated herein by reference.

These antisense molecules may be DNA, stable derivatives of DNA such as phosphorothioates or methylphosphonates, RNA, stable derivatives of RNA such as 2'-*O*-alkylRNA, or other antisense oligonucleotide mimetics. Antisense molecules may be introduced into cells by microinjection, liposome encapsulation or by expression from vectors harboring the antisense sequence.

In the case of gene therapy, the gene of interest can be ligated into viral vectors that mediate transfer of the therapeutic DNA by infection of recipient host cells. Suitable viral vectors include retrovirus, adenovirus, adeno-associated virus, herpes virus, vaccinia virus, polio virus and the like. Alternatively, therapeutic DNA can be transferred into cells for gene therapy by non-viral techniques including receptor-mediated targeted DNA transfer using ligand DNA conjugates or adenovirus-ligand-DNA conjugates, lipofection membrane fusion or direct microinjection. These procedures and variations thereof are suitable for *ex vivo* as well as *in vivo* gene therapy. Protocols for molecular methodology of gene therapy suitable for use with the gene is described in Gene Therapy Protocols, edited by Paul D. Robbins, Human press, Totowa NJ, 1996.

As described herein, the FTI may be used to treat various types of cancer including lung cancer (e.g. adenocarcinoma and including non-small cell lung cancer), pancreatic cancers (e.g. pancreatic carcinoma such as, exocrine pancreatic carcinoma), colon cancers (e.g. colorectal carcinomas, such as, for example, colon adenocarcinoma and colon adenoma), prostate cancer including the advanced disease, hematopoietic tumors of lymphoid lineage (e.g. acute lymphocytic leukemia, B-cell lymphoma, Burkitt's lymphoma), myeloid leukemias (for example, AML), thyroid follicular cancer, myelodysplastic syndrome (MDS), tumors of mesenchymal origin (e.g. fibrosarcomas and rhabdomyosarcomas), melanomas, teratocarcinomas, neuroblastomas, gliomas, benign tumor of the skin (e.g. keratoacanthomas), breast carcinoma (e.g. advanced breast cancer), kidney carcinoma, ovary carcinoma, bladder carcinoma and epidermal carcinoma.

Pharmaceutically useful compositions comprising the drugs of this invention may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the drug. The effective amount of the drug may vary according to a variety of factors such as the individual's condition, weight, sex and age. Other factors include the mode of administration. The pharmaceutical compositions may be provided to the individual by a variety of routes such as subcutaneous, topical, oral and intramuscular.

The drugs of this invention include chemical derivatives of the base molecules of the drug. That is, they may contain additional chemical moieties that are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.

Compounds identified according to the methods disclosed herein may be used alone at appropriate dosages defined by routine testing in order to obtain optimal inhibition or activity while minimizing any potential toxicity. In addition, co-administration or sequential administration of other agents may be desirable.

The drugs of this invention can be administered in a wide variety of therapeutic dosage forms in conventional vehicles for administration. For example, the drugs can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as a modulating agent.

The daily dosage of the products may be varied over a wide range from 0.01 to 1,000 mg per patient, per day.

For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. The dosages are adjusted when combined to achieve desired effects. On the other hand, dosages of these various agents may be independently optimized and combined to achieve a synergistic result wherein the pathology is reduced more than it would be if either agent were used alone.

Advantageously, compounds or modulators as described herein may be administered in a single daily dose, or the total daily dosage may be administered in divided doses of two, three or four times daily. Furthermore, compounds or modulators can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents can be administered concurrently, or they each can be administered at separately staggered times.

The dosage regimen utilizing the compounds or modulators in the present invention is selected in accordance with a variety of factors including type, species, age, weight, sex and medical condition of the patient; the severity of the condition to be treated; the route of administration; the renal and hepatic function of the patient; and the particular drug employed. A physician or veterinarian of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

The drugs can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form uf a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or β-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium sicarate, magnesium stearate, sodium benzoate, sodium acetate, sodium chloride and the like. Disintegrators include, without limitation, starch, methylcellulose, agar, bentonite, xanthan gum and the like.

For liquid forms the active drug component can be combined in suitably flavored suspending or dispersing agents such as the synthetic and natural gums, for example, tragacanth, acacia, methyl-cellulose and the like. Other dispersing agents that may be employed include glycerin and the like. For parenteral administration, sterile suspensions and solutions are desired. Isotonic preparations, which generally contain suitable preservatives, arc employed when intravenous administration is desired.

The drugs can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Drugs may also be delivered by the use of monoclonal antibodies as individual carriers to which the compounds are coupled. The drugs may also be coupled with soluble polymers as targetable drug carriers. Such polymers can include polyvinyl-pyrrolidone, pyran copolymer, polyhydroxypropylmethacryl-amidephenol, polyhydroxy-ethylaspartamidephenol, or polyethyl-eneoxidepolylysine substituted with palmitoyl residues. Furthermore, the drugs in the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

For oral administration, the drugs may be administered in capsule, tablet, or bolus form or alternatively they can be mixed with feed. The capsules, tablets, and boluses are comprised of the active ingredient in combination with an appropriate carrier vehicle such as starch, talc, magnesium stearate, or di-calcium phosphate. These unit dosage forms are prepared by intimately mixing the active ingredient with suitable finely-powdered inert ingredients including diluents, fillers, disintegrating agents, and/or binders such that a uniform mixture is obtained. An inert ingredient is one that will not react with the drugs and which is non-toxic to the animal being treated. Suitable inert ingredients include starch, lactose, talc, magnesium stearate, vegetable gums and oils, and the like. These formulations may contain a widely variable amount of the active and inactive ingredients depending on numerous factors such as the size and type

of the animal species to be treated and the type and severity of the infection. The active ingredient may also be administered by simply mixing the compound with the feedstuff or by applying the compound to the surface of the foodstuff.

The compounds or modulators may alternatively be administered parenterally via injection of a formulation consisting of the active ingredient dissolved in an inert liquid carrier. Injection may be either intramuscular, intraluminal, intratracheal, or subcutaneous. The injectable formulation consists of the active ingredient mixed with an appropriate inert liquid carrier. Acceptable liquid carriers include the vegetable oils such as peanut oil, cotton seed oil, sesame oil and the like as well as organic solvents such as solketal, glycerol formal and the like. As an alternative, aqueous parenteral formulations may also be used. The vegetable oils are the preferred liquid carriers. The formulations are prepared by dissolving or suspending the active ingredient in the liquid carrier such that the final formulation contains from 0.005 to 10% by weight of the active ingredient.

The invention is further illustrated by the following nonlimiting examples.

EXAMPLES

In the nonprophetic examples, AML patients were administered 600 mg of(R)-6-[amino(4-chlorophenyl)(1-methyl-1H-imidazol-5-yl)methyl]-4-(3-chlorophenyl)-1-methyl-2(1H)-quinolinone) (referred to as Zarnestra™) at a starting oral dose of 600 mg b.i.d. for the first 21 days of each 28 day cycle in AML. Subjects were enrolled into two cohorts, those with relapsed AML and those with refractory AML. A total of 257 patients (135 relapsed and 117 refractory) were treated.

Response to Zamestra treatment was defined as patients who had an objective response (CR, CRp, or PR) as shown in Table 2, or patients who demonstrated stable disease and anti-leukemic activity (decrease of >50% of leukemic blast cells) as determined by either central review or the clinical site. Anti-leukemic activity is defined as any bone marrow blast count less than 5% or a reduction in bone marrow blasts by at least half.

Table 2

| Response | Criteria |
|---|---|
| Complete Response (CR) | Neutrophil count >1,000/μL **AND** <5% blasts in bone marrow aspirate **AND** platelet count >100,000/μL and nc extramedullary disease. |
| Complete Response incomplete platelet recovery (CRp) | Meets all the criteria for CR except recovery up to 100,000/μL platelets, but have sufficient bone marrow recovery to be platelet transfusion independent. |
| Partial Response (PR) | At least a 50% decrease in bone marrow blasts **AND** partial recovery of neutrophil count (> 500/μL) **AND** platelet count > 50,000/μL. |
| Stable Disease (SD) | Any response not meeting CR, CRp, PR or PD Criteria **AND** absence of clinical deterioration. |
| Progressive Disease (PD) | > 50% increase in bone marrow blasts OR circulating blasts OR interval development of circulating blasts that persist on at least two consecutive occasions OR interva development of extramedullary disease. |

**Example 1: Microarray Analysis**

Bone marrow samples were collected from consenting patients both before and after treatment with Zamestra™ FTI (active ingredient, (R)-6-[amino(4-chlorophenyl)(1-methyl-1H-imidazol-5-yl)methyl]-4-(3-chlorophenyl)-1-methyl-2(1H)-quinolinone), diluted with PBS and centrifuged with Ficoll-diatrizoate (1.077g/ml). White blood cells were washed twice with PBS, resuspended in FBS with 10% DMSO and immediately frozen at -80°C. Samples were thawed at 37°C and 10X volume of RPMI with 20% FBS was added dropwise over a period of 5 min. Cells were centrifuged at 500g for 10 min and resuspended in 10 ml PBS with 2 mM EDTA and 0.5% BSA. Samples were then passed through a 70 μM filter (Becton Dickinson Labware, Franklin lakes, NJ) to remove any cell clumps. Cell viability was determined by trypan blue dye exclusion assay. The mean viability of the bone marrow samples upon thawing was 35% (range 0-96%). Due to the relatively low number of viable cells present the samples were not further enriched for myeloid cells. Approximately $2x10^5$ cells were double labeled with CD33-FITC and CD34-PE antibodies (Becton Dickinson Biosciences Pharmingen,

San Diego, CA) and FACS analysis was performed.

Total RNA was extracted from cell samples using the RNeasy Kit (Qiagen, Santa Clarita, CA). Synthesis of cDNA and cRNA were performed according to Affymetrix (Santa Clara, CA) protocols. Two rounds of linear amplification were performed since the RNA yield for several samples was less than 1 μg. For hybridization, 11 μg of cRNA were fragmented randomly by incubation at 94°C for 35 min in 40 mM Tris-acetate, pH 8.1, 100 mM potassium acetate, and 30 mM magnesium acetate. Fragmented cRNA was hybridized to U133A arrays at 45°C for 16 hr in a rotisserie oven set at 60 rpm. Following hybridization, arrays were washed (with 6x SSPE and 0.5x SSPE containing Triton X-100 (0.005%)), and stained with streptavidin-phycoerythrin (SAPE; Molecular Probes, Eugene, OR). Quantification of bound labeled probe was conducted using the Agilent G2500A GeneArray scanner (Agilent Technologies, Palo Alto, CA).

The total fluorescence intensity for each array was scaled to the uniform value of 600. Chip performance was quantitated by calculating a signal to noise ratio (raw average signal/noise). Chips were removed from further analysis if their signal-to-noise ratio was less than 5. Genes were only included in further analysis if they were called "present" in at least 10% of the chips. Approximately 12,000 Affymetrix probe sets remained following this cut-off. The quality of the gene expression data was further controlled by identifying outliers based on principal components analysis and by analyzing the normal distributions of the gene intensities.

Chi-squared tests and Student's t-test were used to identify correlations between patient response and patient co-variates, mutational status, CD33 and CD34 antigen expression, leukemic blast counts, and gene expression. To identify genes that could predict response with high sensitivity, a percentile analysis was employed. For example, genes that were up- or down-regulated in all responders compared to at least 40% of non-responders were identified. Genes that did not reveal significant p-values ($P < 0.05$) based on a two-tailed Student's t-test (unequal variance) were removed. The predictive value of the top gene(s) was analyzed by a leave-one-out cross validation method. Here, one sample was removed from the data set and the marker was reselected from the 12,000 genes. The predictive value of this gene was then tested on the left-out sample using a linear discriminant analysis. Sensitivity was calculated as the number of true positives divided by the sum of true positives plus false negatives. Specificity was calculated as the number of true negatives divided by the sum of true negatives and false positives. Positive predictive value was calculated as the number of true positives divided by the sum of true positives and false positives. Negative predictive value was calculated as the number of true negatives divided by the sum of false negatives and true negatives.

Univariate cox proportional hazard models were used to assess the association of each parameter (genes or blast counts) with patient survival outcome. The coefficient estimate of each parameter from the cox model measures the strength of such association. When more than one gene was used a multivariate hazard model was employed. The classifier that distinguishes responders from non-responders was defined as:

$$b1*x1 + b2*x2 + b3*x3 +$$

where b1, b2, b3 are coefficient estimates from the cox model, and x2, x2, x3 are standardized parameter values (blast counts or $\log_{10}$ of gene expression values).

Receiver operator curves (ROC) were used to choose appropriate thresholds for each classifier, requiring a sensitivity of at least 90%. The ROC diagnostic calculates the sensitivity and specificity for each parameter. In addition, gene markers were first ranked for their ability to stratify good outcome from poor outcome using a training set of 29 randomly chosen samples. The predictive value of each gene was then tested on the remaining 29 samples. This allowed for the identification of genes with the most robust predictive values.

**Example 2: Leukemic Cell Antigen Expression**

Leukemic blast cells are known to express the surface antigens CD33 and CD34. 95% and 55% of patient bone marrow leukemic cells were positive for CD33 and CD34, respectively. The mean percentage of cells expressing the antigens in each sample was 13% for CD34 and 43% for CD33. Chi-squared analysis was performed to investigate the correlation between the level of antigen expression and patient outcome. Cutoffs of 15% and 60% were chosen for CD34 and CD33 levels, respectively. Low expression (positive in less than about 15% of the cell population for CD34 and positive in less than about 60% of the cell population for CD33) of CD33 and CD34 correlated with patient response to Zarnestra™ (p=0.137, p=0.052, respectively). High expression of both antigens (positive in more than about 15% of the cell population for CD34 and positive in less than about 60% of the cell population for CD33) was also positively correlated with high blast counts. A Kaplan Meier analysis also indicated that high CD33 expression correlated with poor overall survival (Fig. 1).

**Example 3: Leukemic Blast Count Analysis**

The analysis of CD33 and CD34 antigen expression suggested that the level of leukemic blast cells correlated with patient response. The average value of the site and DCL blast count measurements were calculated and a Student's t-test was performed to investigate if blast counts correlated with patient response in the AML patients. From a total of 199 evaluable patients, 24 of which had a CR, CRp, PR, or SD, the percentage of blasts correlated significantly with patient outcome (p = 0.0006). Responders had a lower number of blasts (mean 34%) than those with progressive disease (mean 51%). Only one of the 24 total responders (defined as having SD) had a blast count higher than 60%. Chi-squared analysis for all evaluable patients also found a significant correlation between high blast counts and resistance to treatment ($\chi^2 = 9.53$).

A Kaplan-Meier analysis found that those patients with a low level of blast counts (<60%) had a significantly better overall survival than those with high blast counts (Fig. 2). These analyses indicate that patients with blast counts higher than approximately 60% will be unlikely to respond to Zarnestra™.

**Example 4: Identification of genes that are differentially expressed between responders and non-responders**

Bone marrow samples were obtained for gene expression analysis from 80 patients prior to drug treatment. Of the 80 base-line samples, 14 were removed from the analysis since they came from non-evaluable patients. Samples were enriched for myeloid cells, processed for messenger RNA (the molecules that encode for gene-specific proteins), and hybridized to the Affymetrix U133A gene chip. 58 of the 66 samples passed additional quality control measures following hybridization to the U133A chip. The gene expression data was integrated with the clinical information and retrospective analyses were performed to identify genes that could stratify responders from non-responders with a high level of sensitivity. Several gene markers were identified that are useful in predicting response to Zarnestra™ (Table 3). In the case of the LBC oncogene (oncoLBC) the predictive value of this gene was calculated for the dataset using a leave-one-out cross validation (Table 3). The oncoLBC gene expression levels were able to capture all of the clinically identified responders while removing over half of the non-responders.

Table 3: Genes Differentially Expressed in Responders versus Non-Responders

| Title | Gene Symbol | ttest | Ratio* | RefSeq | Seq Id N |
|---|---|---|---|---|---|
| aryl hydrocarbon receptor | AHR | 2.55E-06 | 0.39 | NM_001621 | 1 |
| A kinase (PRKA) anchor protein 1 (oncoLBC) | AKAP13 | 6.13E-05 | 0.51 | NM_006738 | 2 |
| myc-induced nuclear antigen, 53 kD | MINA53 | 6.93E-05 | 0.71 | NM_032778 | 3 |
| iduronate 2-sulfatase (Hunte syndrome) | IDS | 0.00024 | 0.36 | NM_000202 | 4 |
| opsin 3 (encephalopsin, panopsin) | OPN3 | 0.000643 | 0.54 | NM_014322 | 5 |
| G protein-coupled receptor 105 | GPR105 | 0.000876 | 0.34 | NM_014879 | 6 |
| tumor endothelial marker 6 | TEM6 | 0.001031 | 0.62 | NM_022748 | 7 |
| tumor necrosis factor (ligand superfamily, member 13 | TNFSF 13 | 0.001042 | 0.59 | NM_003808 | 8 |
| Supervillin | SVIL | 0.001457 | 0.62 | NM_003174 | 9 |
| interleukin 3 receptor, alpha (low affinity) | IL3RA | 0.001984 | 0.40 | NM_002183 | 10 |
| chromosome 6 open reading frame 56 | C6orf56 | 0.002616 | 0.57 | NM_014721 | 11 |
| FGF receptor activating protein 1 | FRAG1 | 0.00299 | 1.24 | NM_014489 | 12 |
| golgi SNAP receptor comple member 1 | GOSR1 | 0.012011 | 1.50 | NM_004871 | 13 |
| KIAA1036 protein | KIAA1036 | 0.012621 | 0.72 | NM_014909 | 14 |
| BTG family, member 3 | BTG3 | 0.016594 | 1.48 | NM_006806 | 15 |
| mitogen-activated protein kinase interacting protein 3 | MAPK8IP3 | 0.018174 | 1.39 | NM_015133 | 16 |
| ras homolog gene family member H | ARHH | 0.027219 | 1.64 | NM_004310 | 17 |

(continued)

| Title | Gene Symbol | ttest | Ratio* | RefSeq | Seq Id N |
|---|---|---|---|---|---|
| neuronal pentraxin II | NPTX2 | 0.033468 | 0.14 | NM_002523 | 18 |
| *Ratios indicate fold-change in responders compared to non-responders | | | | | |

Table 4: Leave-one-out cross validation using oncoLBC as a marker of response.

| | | Gold Std | |
|---|---|---|---|
| | | Resp | Non-resp |
| Test | Predict response | 14 | 20 |
| | Predict non-resp | 0 | 24 |
| | Total | 14 | 44 |

Sensitivity =100%
Specificity = 55%
Positive predictive value = 41 %
Negative predictive value =100%

A survival analysis showed that patients who were classified as responders based on oncoLBC expression (Fig. 3A, p = 0.00841) significantly outperformed patient classification using the clinical data (Fig. 3B, p = 0.0827). This was due to the oncoLBC marker identifying a subset of non-responders with an increased overall survival. Based on the Cox hazard model, combining the oncoLBC and a second gene marker, the aryl hydrocarbon receptor (AHR), increased the specificity and positive predictive value to 75% and 56%, respectively (Fig. 3C). These results indicated that using either the oncoLBC alone, or in combination with the AHR gene presents an effective array for predicting response to Zarnestra™ treatment.

A leave-one-out cross validation using the oncoLBC and AHR as markers was also performed. When using the cut-off to identify the highest sensitivity and best specificity the PPV and sensitivity remained the same. Results of leave-one out cross validation of other gene combinations are shown in Table 5. This illustrates that marker combinations can improve the predictive value of this method.

Table 5

| **Markers** | **Sensitivity** | **specificity** | **PPV** | **NPV** | **prior probability*** |
|---|---|---|---|---|---|
| LBC, AHR, katanin, IDS, MINA53 | 100 | 73 | 54 | 100 | 0.32 |
| LBC, katanin, MINA53 | 100 | 70 | 52 | 100 | 0.47 |
| LBC, AHR, MINA53 | 100 | 66 | 48 | 100 | 0.23 |
| LBC, AHR, IDS, MINA53 | 100 | 66 | 48 | 100 | 0.28 |
| LBC, AHR, IDS | 100 | 61 | 45 | 100 | 0.2 |
| LBC, katanin | 100 | 61 | 45 | 100 | 0.41 |
| LBC, MINA53 | 100 | 57 | 42 | 100 | 0.2 |
| Katanin | 100 | 56 | 42 | 100 | 0.49 |
| LBC | 100 | 55 | 41 | 100 | 0.4 |
| LBC, AHR | 100 | 55 | 41 | 100 | 0.19 |
| LBC, IDS, katanin | 100 | 55 | 41 | 100 | 0.38 |
| LBC, AHR, katanin | 100 | 52 | 40 | 100 | 0.18 |
| LBC, IDS | 100 | 50 | 39 | 100 | 0.38 |
| IDS | 100 | 50 | 39 | 100 | 0.42 |
| MINA53 | 100 | 45 | 37 | 100 | 0.39 |
| AHR | 100 | 45 | 37 | 100 | 0.17 |
| AHR, katanin | 100 | 43 | 36 | 100 | 0.17 |

Furthermore, stratification of patients using the LBC and AHR classifier showed a similar difference in median

survival time between the two patient populations compared with using the oncoLBC gene or the clinical response definitions (Fig. 4C). These results indicated that using either the oncoLBC alone, or in combination with the AHR gene could be used as effective biomarkers for predicting response to ZARNESTRA in the current dataset.

A Cox hazard model was used to analyze other combinations of markers in stratifying poor survivors from good survivors (Table 6). Here, data from 51 patients was used since only this number of patient samples had CD33 and CD34 antigen levels measured. A sensitivity of greater than 90% was used in determining the appropriate cutoffs for the markers. The use of multiple markers can improve the difference in median survival times of the 2 survival groups.

Table 6

| Markers* | P-value | Median survival poor (days) | Median survival good (days) | Delta (days) | Sens (%) | Spec (%) |
|---|---|---|---|---|---|---|
| LBC | 0.0098 | 64 | 177 | 113 | 91 | 65 |
| CD33 | 0.0029 | 40 | 105 | 65 | 91 | 23 |
| CD34 | 0.643 | 64 | 103 | 39 | 91 | 23 |
| AHR | 0.12 | 59 | 106 | 47 | 91 | 53 |
| % blasts[p] | 0.1241 | 60 | 103 | 43 | 91 | 38 |
| LBC + Blasts | 0.00223 | 59 | 154 | 95 | 93 | 61 |
| LBC + AHR | 0.0111 | 71 | 171 | 100 | 91 | 73 |
| LBC + CD33 | 0.00266 | 71 | 182 | 111 | 91 | 73 |
| LBC + CD34 | 0.00038 | 64 | 192 | 128 | 91 | 80 |
| LBC +AHR+CD34 | 0.003 | 71 | 213 | 142 | 91 | 83 |
| LBC +AHR+CD33 | 0.00062 | 64 | 192 | 128 | 91 | 80 |

**Example 5: Identification of genes that are differentially expressed between responders and non-responders (repeat analysis)**

We performed supervised analysis using the gene expression data to identify additional genes that were differentially expressed between all responders and at least 40% of non-responders. These criteria were chosen to identify genes that could predict response to Tipifamib with the highest level of sensitivity possible. A total of 19 genes were identified that could stratify responders and non-responders (Tables 7 and 8) and that gave significant p-values in the t-test ($p < 0.05$). Interestingly, the genes include those involved in signal transduction, apoptosis, cell proliferation, oncogenesis, and potentially, FTI biology (ARHH, LBC and, IL3RA).

Table 7 List of top 19 genes that can predict response to Tipifarnib

| SEQ ID NO: | Gene Title | Symbol | Gene ID |
|---|---|---|---|
| 1 | Aryl hydrocarbon receptor | AHR | NM_001621 |
| 2 | A kinase (PRKA) anchor protein 13 | AKAP 13 | NM_006738 |
| 3 | Myc-induced nuclear antigen, 53 kDa | MINA53 | NM_032778 |
| 4 | iduronate 2-sulfatase (Hunter syndrome) | IDS | NM_000202 |
| 5 | opsin 3 (encephalopsin, panopsin) | OPN3 | NM_014322 |
| 6 | G protein-coupled receptor 105 | GPR105 | NM_014879 |
| 7 | tensin-like SH2 domain-containing 1 | TENS1 | NM_022748 |
| 8 | tumor necrosis factor (ligand) superfamily, member 13 | TNFSF13 | NM_003808 |
| 9 | supervillin | SVIL | NM_003174 |
| 10 | interleukin 3 receptor, $\alpha$ (low affinity) | IL3RA | NM_002183 |
| 11 | chromosome 6 open reading frame 56 | C6orf56 | NM_014721 |

(continued)

| SEQ ID NO: | Gene Title | Symbol | Gene ID |
|---|---|---|---|
| 12 | FGF receptor activating protein 1 | FRAG1 | NM_014489 |
| 13 | golgi SNAP receptor complex member 1 | GOSR1 | NM_004871 |
| 14 | KIAA1036 | KIAA1036 | NM_014909 |
| 15 | BTG family, member 3 | BTG3 | NM_006806 |
| 16 | mitogen-activated protein kinase 8 interacting protein 3 | MAPK8IP3 | NM_015133 |
| 29 | leukocyte immunoglobulin-like receptor, subfamily B, member 3 | LILRB3 | NM_006864 |
| 17 | Ras homolog gene family, member H | ARHH | NM_004310 |
| 18 | neuronal pentraxin II | NPTX2 | NM_002523 |

Table 8 Characteristics of genes in Table 7

| Symbol | Specificity | ratio | p-value |
|---|---|---|---|
| AHR | 0.52 | 0.386 | 0.00000255 |
| AKAP13 | 0.63 | 0.514 | 0.00006133 |
| Mina53 | 0.5 | 0.707 | 0.00006934 |
| IDS | 0.5 | 0.364 | 0.00023964 |
| OPN3 | 0.4 | 0.541 | 0.00064297 |
| GPR105 | 0.43 | 0.345 | 0.00087608 |
| TENS1 | 0.43 | 0.622 | 0.0010309 |
| TNFSF13 | 0.4 | 0.588 | 0.00104219 |
| SVIL | 0.45 | 0.618 | 0.00145723 |
| IL3RA | 0.4 | 0.398 | 0.00198392 |
| C6orf56 | 0.4 | 0.575 | 0.00261553 |
| FRAG1 | 0.45 | 1.240 | 0.00298989 |
| GOSR1 | 0.45 | 1.498 | 0.01201057 |
| K1AA1036 | 0.43 | 0.716 | 0.01262079 |
| BTG3 | 0.47 | 1.483 | 0.01659402 |
| MAPK8IP3 | 0.4 | 1.391 | 0.01817428 |
| LILRB3 | 0.41 | 0.657 | 0.02374898 |
| ARHH | 0.4 | 1.639 | 0.02721922 |
| NPTX2 | 0.45 | 0.137 | 0.03346833 |

**Example 6: Identification of a minimal set of 3 gene markers**

To identify a candidate set of gene markers that could predict response to tipifarnib with an improved accuracy compared to LBC alone we used LOOCV to determine the optimal number of genes. We built classifiers with increasing number of genes based on t-test p-values, and calculated the error rate of these classifiers using LOOCV while keeping the sensitivity of predicting response at 100% (Fig. 4A). It was found that a 3-gene classifier (including LBC, AHR, and MINA53) could predict response with the lowest error rate (Fig. 4A). This was also seen when a leave-five-out cross validation was performed. When more genes were added the error rate increased indicating that additional genes introduced noise to the classifier. For the 3-gene classifier the LOOCV demonstrated a sensitivity of 86% and specificity of 70% with an overall diagnostic accuracy of 74% (Fig. 4B). The combined expression values for the 3 genes in each

patient are shown in Figure 4C.

Kaplan-Meier analysis again showed a significant difference in survival between the predicted responder group and the non-responder group (Fig. 4D). Moreover, if we compared the incorrectly classified non-responders to the correctly classified non-responders, the misclassified non-responders showed a better overall survival (Fig. 5). This implies that the gene signature could predict a level of response to therapy that cannot be observed by the clinical criteria. Alternatively, this may allow the gene signature to predict response to FTI treatment thus providing prognostic value. Further analysis carried out in untreated patients can be used to determine the relationship between the gene expression signatures and prognosis.

**Example 7: Antibodies (Prophetic)**

An LBC oncogene-derived peptide is synthesized, coupled to keyhole limpet hemocyanin, and used to immunize rabbits for production of polyclonal antibodies. The sera are tested for reactivity against the corresponding peptide with ELISA, and the positive batches are affinity-purified. The purified antibody specifically detects the peptide that has the epitope in tissue sections. This is verified by complete abolishment of the signal if the corresponding peptide is added simultaneously with the antibody. In addition to this polyclonal antibody, which works well in IHC, monoclonal antibodies able to detect the protein in its natural fold are produced. To produce monoclonal antibodies, a purified antigen, produced in mammalian cells to ensure natural fold and posttranslational modifications, is generated. The antigen, LBC onco protein-IgG constant part fusion protein, is expressed in mouse myeloma cells, and the protein is purified using the Fc part as bait. This purified antigen is recognized in Western blot by the C-terminal polyclonal antibody. The antigen is used to generate mouse monoclonal antibodies against LBC peptides by selecting out of the positive clones those that produce antibodies that react against LBC peptide instead of the IgG constant part.

Kits for the clinical identification of LBC oncogene can be readily fashioned employing these and similar antibodies. Such kits would include antibodies directed to LBC peptide identification (and hence, LBC oncogene), appropriate indicator reagents (e.g., enzymes, labels, and the like), and (optionally) other reagents useful in the clinical application of such a kit such as dilution buffers, stabilizers, and other materials typically used in such assays.

**Example 8: In situ hybridization (Prophetic)**

Formalin fixed paraffin embedded tissue samples are cut into 5-7 μm thick sections, mounted on silane coated glass slides, and incubated at 37°C over night and at 65°C for 30 min before deparaffinating twice for 10 min in xylene. Thereafter the samples are rehydrated through a graded series of ethanol solutions (100 to 70%), and rinsed twice for 5 min in PBS pH 7.0, treated twice for 5 min with 0.1 mol/L glycine in PBS, permeabilized for 15 min with 0.3% Triton X-100 in PBS. The sections are treated with proteinase K (Finnzymes, Helsinki, Finland) treatment (μg/ml, in TE buffer; 100 mmol/L Tris-HCl, 50 mmol/L EDTA, pH 8.0) at 37°C for 30 min, postfixed in 3% paraformaldehyde in PBS at 4°C for 5 min and rinsed twice in PBS. Positive charges are blocked by soaking the slides in 0.25% (v/v) acetic anhydride, 100 mmol/L triethanolamine, pH 8.0, twice for 5 min. The slides are equilibrated in 4xSSC, 50% (v/v) deionized formamide at 37°C for 10 min.

Probes are prepared by ligating a PCR-amplified 0.4 kb LBC oncogene cDNA insert into the pCR-II vector using a TA cloning kit (Invitrogen, San Diego CA). The templates for LBC oncogene antisense or sense RNA probes are generated by linearizing the appropriate vector construct (in 3' to 5' direction or 5' to 3' direction, respectively). An RNA Labeling Kit (Boehringer-Mannheim) is used to generate digoxygenin labeled RNA probes by in vitro transcription. The hybridization is performed overnight at 45°C using a hybridization mixture containing I xDenhardt's solution (0.2g/L Ficoll Type 400, Pharmacia), 0.2g/L polyvinylpyrrollidone, 0.2g/L BSA (fraction V; Sigma), 40% formamide, 10% dextran sulfate, 4xSSC, 10 mmol/L dithiothreitol, 1mg/mL yeast tRNA, 1 mg/mL herring sperm DNA and 300 ng/mL digoxygenin-labeled RNA probe. After hybridization, the tissue sections are washed at 37°C twice for 5 min in 2xSSC and once for 15 min in 60% formamide, 0.2xSSC, followed by two 5 minute rinses in 2xSSC at room temperature and two 10 minute washes in 100 mmol/L Tris-HCl, pH 8.0, 150 mmol/L NaCl. The signal detection is carried out using 1:250 alkaline phosphatase-conjugated sheep antidigoxygenin Fab fragments (Boehringer Mannheim). The signal is visualized by incubating the sections with NBT/BCIP Stock Solution (Boehringer Mannheim) for 1.5 hours.

LBC oncogene-positive cells seen in the tumorigenic cells of cancer patients indicates that response to an FTI is unlikely.

**Example 9: Immunohistochemistry (Prophetic)**

An affinity-purified polyclonal antibody against the C-terminal peptide of LBC oncogene is used for the IHC detection and localization of LBC oncogene. Four μm sections from formalin-fixed and paraffin embedded normal and tumor tissue is mounted on 3-aminopropyl-triethoxy-silane (APES, Sigma, St. Louis, MO) coated slides. The sections

are deparaffinized and rehydrated in graded concentrations of ethanol and treated with methanolic peroxide (0.5% hydrogen peroxide in absolute methanol) for 30 minutes at room temperature to block the endogenous peroxidase activity. Antigen retrieval is done in a microwave oven twice for 5 minutes (650W).

An Elite ABC Kit (Vectastain, Vector Laboratories, Burlingame, CA) is used for immunoperoxidase staining. The LBC peptide antibody is used at an optimal dilution of 1:2000. Both the biotinylated second antibody and the peroxidase-labeled avidin-biotin complex are incubated on the sections for 30 minutes. The dilutions are made in PBS (pH 7.2), and all incubations are carried out in a moist chamber at room temperature. Between the different staining steps the slides are rinsed three times with PBS. The peroxidase staining is visualized with a 3-amino-9-ethylcarbazole (Sigma) solution (0.2 mg/ml in 0.05 M acetate buffer containing 0.03% hydrogen peroxide, pH 5.0) at room temperature for 15 minutes. Finally, the sections are lightly counterstained with Mayer's haematoxylin and mounted with aqueous mounting media (Aquamount, BDH). In control experiments the primary antibodies are replaced with the IgG fraction of normal rabbit serum or the primary antibody was preabsorbed with the LBC peptide. These stainings indicate the presence of the LBC oncogene in a subset of cells.

**Example 10: Enzyme Immunoassay (Prophetic)**

Immunoassays are prepared for the LBC protein or characteristic peptides related to the LBC oncogene. Antigen standards comprising a digest of colon tumor specimens (shown to contain the antigen by immunoperoxidase staining) are used. Human white blood cells from AML patients. The specimens are pooled and homogenized in 10 volumes of 10 mM Tris buffer, pH 7.4, containing 0.2% (w/v) sodium deoxycholate at 4°C. The homogenate is quickly brought to 37°C and the following reagents (final concentration) are added while stirring: I mM cysteine (Sigma), 1 mM EDTA (Sigma), and papain (0.8 unit/ml) (Boehringer-Mannheim, Indianapolis, IN). After 5 minutes, digestion is stopped by the addition of 5 mM iodoacetamide (Sigma). The homogenate is centrifuged at 100,000Xg for 1 hour at 4°C, then extensively dialyzed against 10 mM Tris/0.9% NaCl solution buffer, pH 7.4, containing phenylmethylsulfonyl fluoride and aminocaproic acid, each at 10 mM. The homogenate is frozen in small aliquots at a concentration of 0.5 mg of protein/ml.

The dose response curve that will be generated for the immunoassay procedure measuring LBC protein or peptides demonstrates linearity between antigen input of 100ng to 100μg/ml. For serum analysis, the range is 1ng to 1000ng/ml, since these samples are diluted 10-fold prior to assay.

Solid-phase preparations of the antibodies described in the examples above are prepared using CNBr-activated Sepharose (Pharmacia). Microtiter plates (Nunc I Immunoplates; Grand Island Biological Co., Grand Island, NY) are coated with the antibodies (200 μl/well) in 50 mM carbonate-bicarbonate buffer, pH 9.6, for 18 hours at 4°C. After removal of the antibody solution, residual protein binding sites on the plastic are blocked by the addition of 200 μl of assay buffer [PBS containing 1% (v/v) rabbit serum and 1% (w/v) bovine albumin]. After 1 hour of incubation at room temperature, the coated plates are used immediately for the assay procedure.

To perform the assay, 200 μl samples, diluted in assay buffer, are applied for 1-5 hours at 37°C. After 3 washes using assay buffer, 200 μl of the antibody covalently conjugated to horseradish peroxidase (Sigma, Type VI) is applied to each well for 1.5 hours at 37°C. The conjugate is diluted to a concentration of 0.5 μg of immunoglobulin per ml of PBS containing 10% (v/v) murine serum. Following a wash procedure as above, 200 μl of substrate per well are applied for 0.5 hours at room temperature. Substrate solution contains 0.4 mg of o-phenylenediamine per ml of pH 5.0 citrate buffer and 0.003% hydrogen peroxide. The reaction is stopped by addition of 50 μl of 2N sulfuric acid, and absorbance is monitored at 488 nM using an enzyme assay plate reader (Fisher Scientific Co., Pittsburgh, PA).

The percentage of bound enzyme conjugate is calculated by the formula:

$$(B-B_0)(B_t-B_0)(100)$$

where B=absorbance of the sample, $B_t$ =maximal absorbance, and Bo =absorbance of the blank. Each assay is performed in triplicate using a standard digest and 26-fold diluted serum samples diluted in assay buffer. Immunoassay specificity is examined by substituting various antibody reagents at the solid phase, including an antibody to an unrelated protein and non-immune rabbit serum. Of the solid phase antibodies only antibody prepared according to the examples above bind antigen at high dilutions.

Levels of serum LBC protein are detected for normal control subjects, patients with benign and malignant hematological disorders.

Sera obtained from apparently healthy individuals exhibits no LBC protein. Only 5% of the samples express serum antigen at the limit of detection or above, and this value is chosen as the cutoff for elevated serum levels. Cancer patients below the cutoff are likely to respond to treatment with an FTI.

## Claims

1. A method of determining whether a patient will respond to treatment with a farnesyl transferase inhibitor (FTI) by analyzing the presence or expression of the LBC oncogene (SEQ ID NO: 2), further including the analysis of the expression of a gene that is differentially modulated in the presence of an FTI wherein the gene is SEQ ID NO: 29, wherein said patient has acute myeloid leukaemia (AML).

2. The method of claim 1 wherein a patient in whom the LBC oncogene is determined to be absent or is not expressed is treated with an FTI.

3. The method of claim 1 wherein a patient in whom the LBC oncogene is determined to be present or express is treated with an agent other than an FTI.

4. The method of claim 1 further comprising the step of determining whether the patient samples used to determine response to the FTI includes cell surface antigens selected from the group consisting of CD33 and CD34.

5. The method of claim 4 wherein the presence of cells having said surface antigens is prognostic of response to FTI treatment.

6. The method of claim 1 further comprising the step of determining the percentage of cells in the patient sample used to determine response to the FTI that includes blast cells.

7. The method of claim 6 wherein the presence of less than 60% blast cells in said sample is prognostic of response to the FTI.

8. A method of monitoring the therapy of a patient being treated with a farnesyl transferase inhibitor (FTI) by analyzing the presence or expression of the LBC oncogene (SEQ ID NO: 2), further including the analysis of the expression of a gene that is differentially modulated in the presence of an FTI, wherein the gene is SEQ ID NOs: 29, wherein said patient has AML.

9. The method of claim 8 wherein a patient in whom the LBC oncogene is determined to be absent or is not expressed is treated as patient responding to the FTI.

10. The method of claim 8 further comprising the step of determining whether the patient samples used to determine response to the FTI includes cell surface antigens selected from the group consisting of CD33 and CD34.

11. The method of claim 10 wherein the presence of cells having said surface antigens comprises less than 15% in the case of CD33 or less than 60% in the case of CD34 is indicative of response to FTI treatment.

12. The method of claim 10 wherein the absence of cells having said surface antigens is indicative of response to FTI treatment.

13. The method of claim 8 further comprising the step of determining the percentage of cells in the patient sample used to determine response to the FTI that includes blast cells.

14. The method of claim 13 wherein the presence of less than or equal to 60% blast cells in said sample is indicative of response to the FTI.

15. The method of claim 14 wherein the presence of more than 60% blast cells in said sample is indicative of non-response to the FTI.

16. The method of claim 8 wherein a patient in whom the LBC oncogene is determined to be present or expressed without a reduction in amount is treated as not responding to the FTI.

17. The method of claim 8 wherein the FTI is (R)-6-[amino(4-chlorcphenyl)(1-methyl-1H-imidazol-5-yl)methyl]-4-(3-chlorophenyl)-1-methyl-2(1H)-quinolinone).

18. The method of claim 8 wherein the FTI is (R)-6-[amino(4-chlorophenyl)(1-methyl-1H-imidazol-5-yl)methyl]-4-(3-chlorophenyl)-1-methyl-2(1H)-quinolinone).

**Patentansprüche**

1. Verfahren zur Feststellung, ob ein Patient auf eine Behandlung mit einem Farnesyltransferaseinhibitor (FTI) ansprechen wird, durch Analysieren des Vorhandenseins oder der Expression des LBC-Onkogens (SEQ ID NO: 2), das weiter die Analyse der Expression eines Gens einschließt, das bei Vorhandensein eines FTIs differentiell moduliert wird, wobei das Gen SEQ ID NO: 29 ist, wobei der Patient akute myeloische Leukämie (AML) hat.

2. Verfahren nach Anspruch 1, wobei ein Patient, bei dem festgestellt wird, dass das LBC-Onkogen nicht vorhanden ist oder nicht exprimiert wird, mit einem FTI behandelt wird.

3. Verfahren nach Anspruch 1, wobei ein Patient, bei dem festgestellt wird, dass das LBC-Onkogen vorhanden ist oder exprimiert wird, mit einem anderen Mittel als einem FTI behandelt wird.

4. Verfahren nach Anspruch 1, das weiter den Schritt der Feststellung umfasst, ob die Patientenprobe, die verwendet wird, um das Ansprechen auf den FTI festzustellen, Zelloberflächenantigene einschließt, die ausgewählt sind aus der Gruppe, bestehend aus CD33 und CD34.

5. Verfahren nach Anspruch 4, wobei das Vorhandensein von Zellen mit den Oberflächenantigenen prognostisch für ein Ansprechen auf FTI-Behandlung ist.

6. Verfahren nach Anspruch 1, das weiter den Schritt der Bestimmung des Prozentanteils an Zellen in der Patientenprobe, die verwendet wird, um das Ansprechen auf den FTI festzustellen, der Stammzellen einschließt, umfasst.

7. Verfahren nach Anspruch 6, wobei das Vorhandensein von weniger als 60% Stammzellen in der Probe prognostisch für ein Ansprechen auf den FTI ist.

8. Verfahren zur Überwachung der Therapie eines Patienten, der mit einem Farnesyltransferaseinhibitor (FTI) behandelt wird, durch Analysieren des Vorhandenseins oder der Expression des LBC-Onkogens (SEQ ID NO: 2), das weiter die Analyse der Expression eines Gens einschließt, das bei Vorhandensein eines FTIs differentiell moduliert wird, wobei das Gen SEQ ID NO: 29 ist, wobei der Patient AML hat.

9. Verfahren nach Anspruch 8, wobei ein Patient, bei dem festgestellt wird, dass das LBC-Onkogen nicht vorhanden ist oder nicht exprimiert wird, als Patient behandelt wird, der auf den FTI anspricht.

10. Verfahren nach Anspruch 8, das weiter den Schritt der Feststellung umfasst, ob die Patientenprobe, die verwendet wird, um das Ansprechen auf den FTI festzustellen, Zelloberflächenantigene einschließt, die ausgewählt sind aus der Gruppe, bestehend aus CD33 und CD34.

11. Verfahren nach Anspruch 10, wobei das Vorhandensein von Zellen mit den Oberflächenantigenen von weniger als 15% im Falle von CD33 und weniger als 60% im Falle von CD34 indikativ für ein Ansprechen auf FTI-Behandlung ist.

12. Verfahren nach Anspruch 10, wobei das Nichtvorhandensein von Zellen mit den Oberflächenantigenen indikativ für ein Ansprechen auf FTI-Behandlung ist.

13. Verfahren nach Anspruch 8, das weiter den Schritt der Bestimmung des Prozentanteils an Zellen in der Patientenprobe, die verwendet wird, um Ansprechen auf den FTI festzustellen, der Stammzellen einschließt, umfasst.

14. Verfahren nach Anspruch 13, wobei das Vorhandensein von weniger als oder gleich 60% Stammzellen in der Probe indikativ für ein Ansprechen auf den FTI ist.

15. Verfahren nach Anspruch 14, wobei das Vorhandensein von mehr als 60% Stammzellen in der Probe indikativ für ein Nicht-Ansprechen auf den FTI ist.

16. Verfahren nach Anspruch 8, wobei ein Patient, bei dem festgestellt wird, dass das LBC-Onkogen vorhanden ist oder exprimiert wird, ohne eine Verringerung der Menge, als nicht auf den FTI ansprechend behandelt wird.

17. Verfahren nach Anspruch 8, wobei der FTI (R)-6-[Amino(4-chlorphenyl)(1-methyl-1H-imidazol-5-yl)methyl]-4-(3-chlorphenyl)-1-methyl-2(1H)-chinolinon) ist.

18. Verfahren nach Anspruch 8, wobei der FTI (R)-6-[Amino(4-chlorphenyl)(1-methyl-1H-imidazol-5-yl)methyl]-4-(3-chlorphenyl)-1-methyl-2(1H)-chinolinon) ist.

## Revendications

1. Méthode permettant de déterminer si un patient répondra au traitement avec un inhibiteur de la farnésyl-transférase (FTI) en analysant la présence ou l'expression de l'oncogène LBC (SÉQ ID NO : 2), comprenant en outre l'analyse de l'expression d'un gène qui est modulé de manière différentielle en présence d'un FTI où le gène est SÉQ ID NO : 29, où ledit patient souffre d'une leucémie aiguë myéloide (LAM).

2. Méthode selon la revendication 1, où un patient chez lequel l'oncogène LBC est déterminé comme étant absent ou n'est pas exprimé est traité avec un FTI.

3. Méthode selon la revendication 1, où un patient chez lequel l'oncogène LBC est déterminé comme étant présent ou exprimé est traité avec un agent autre qu'un FTI.

4. Méthode selon la revendication 1, comprenant en outre l'étape consistant à déterminer si l'échantillon de patient utilisé pour déterminer la réponse au FTI comprend des antigènes de la surface cellulaire choisis dans le groupe

constitué de CD33 et CD34.

5. Méthode selon la revendication 4, où la présence de cellules ayant lesdits antigènes de surface est le pronostic d'une réponse au traitement avec un FTI.

6. Méthode selon la revendication 1, comprenant en outre l'étape consistant à déterminer le pourcentage de cellules dans l'échantillon de patient utilisé pour déterminer la réponse au FTI qui comprend des blastocytes.

7. Méthode selon la revendication 6, où la présence de moins de 60 % de blastocytes dans ledit échantillon est le pronostic d'une réponse au FTI.

8. Méthode de contrôle de la thérapie d'un patient traité avec un inhibiteur de la farnésyl-transférase (FTI) en analysant la présence ou l'expression de l'oncogène LBC (SÉQ ID NO : 2), comprenant en outre l'analyse de l'expression d'un gène qui est modulé de manière différentielle en présence d'un FTI, où le gène est SÉQ ID NO : 29, où ledit patient souffre d'une LAM.

9. Méthode selon la revendication 8, où un patient chez lequel l'oncogène LBC est déterminé comme étant absent ou n'est pas exprimé est traité comme un patient répondant au FTI.

10. Méthode selon la revendication 8, comprenant en outre l'étape consistant à déterminer si l'échantillon de patient utilisé pour déterminer la réponse au FTI comprend des antigènes de la surface cellulaire choisis dans le groupe constitué de CD33 et CD34.

11. Méthode selon la revendication 10, où la présence de cellules ayant lesdits antigènes de surface comprend moins de 15 % dans le cas de CD33 ou moins de 60 % dans le cas de CD34 est indicative d'une réponse au traitement avec un FTI.

12. Méthode selon la revendication 10, où l'absence de cellules ayant lesdits antigènes de surface est indicative d'une réponse au traitement avec un FTI.

13. Méthode selon la revendication 8, comprenant en outre l'étape consistant à déterminer le pourcentage de cellules dans l'échantillon de patient utilisé pour déterminer la réponse au FTI qui comprend des blastocytes.

14. Méthode selon la revendication 13, où la présence de moins de ou de 60 % de blastocytes dans ledit échantillon est indicative d'une réponse au FTI.

15. Méthode selon la revendication 14, où la présence de plus de 60 % de blastocytes dans ledit échantillon est indicative de l'absence de réponse au FTI.

16. Méthode selon la revendication 8, où un patient chez lequel l'oncogène LBC est déterminé comme étant présent ou exprimé sans réduction de quantité est traité comme ne répondant pas au FTI.

17. Méthode selon la revendication 8, où le FTI est la (R)-6-[amino(4-chlorophényl)(1-méthyl-1H-imidazol-5-yl)méthyl]-4-(3-chlorophényl)-1-méthyl-2(1H)-quinoléinone).

18. Méthode selon la revendication 8, où le FTI est la (R)-6-[amino(4-chlorophényl)(1-méthyl-1H-imidazol-5-yl)méthyl]-4-(3-chlorophényl)-1-méthyl-2(1H)-quinoléinone).

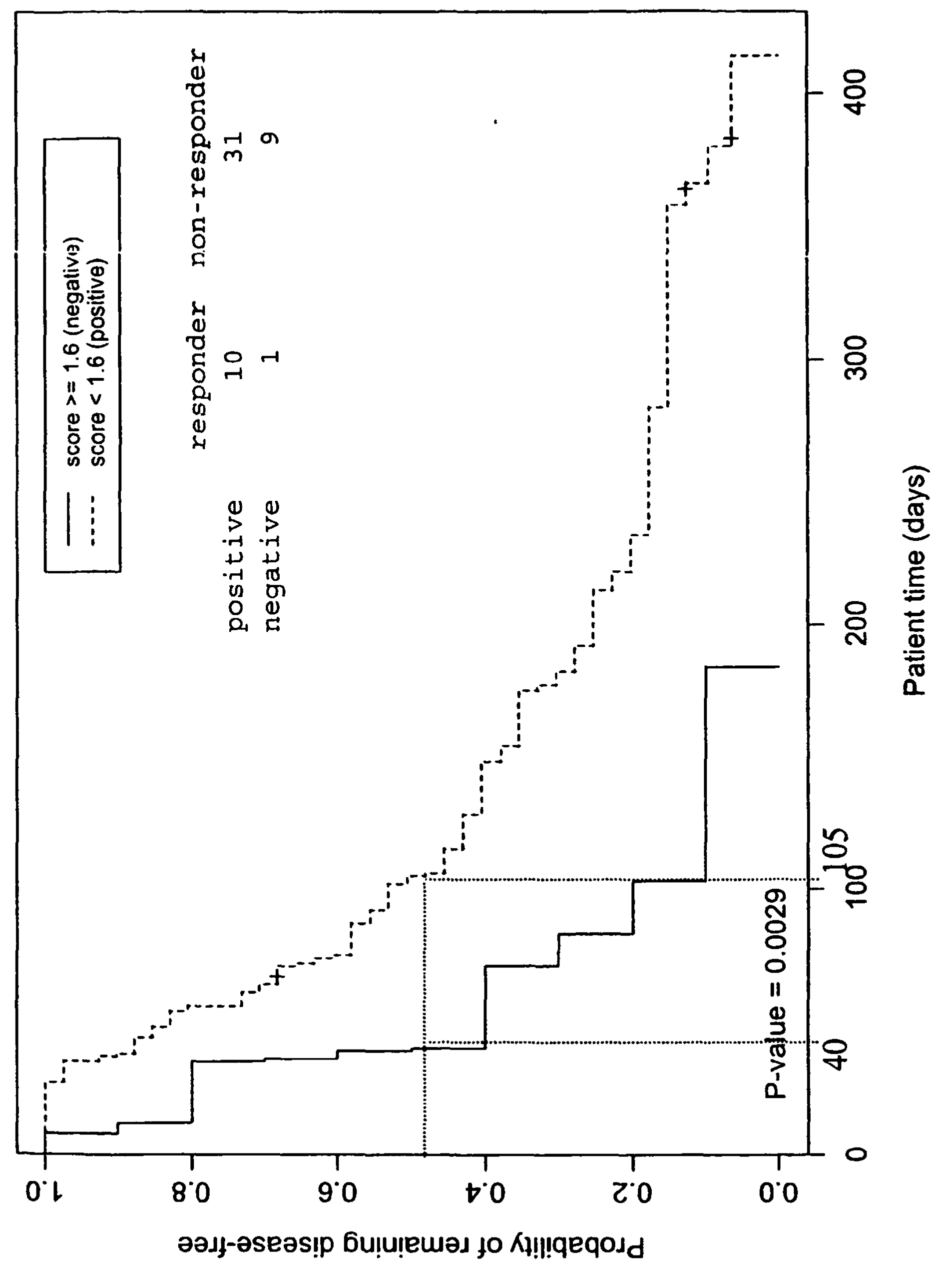
score >= 1.6 (negative)
score < 1.6 (positive)
responder non-responder
positive 10 31
negative 1 9
P-value = 0.0029
Probability of remaining disease-free
Patient time (days)
0
40
100
105
200
300
400
0.0
0.2
0.4
0.6
0.8
1.0

Fig 1.

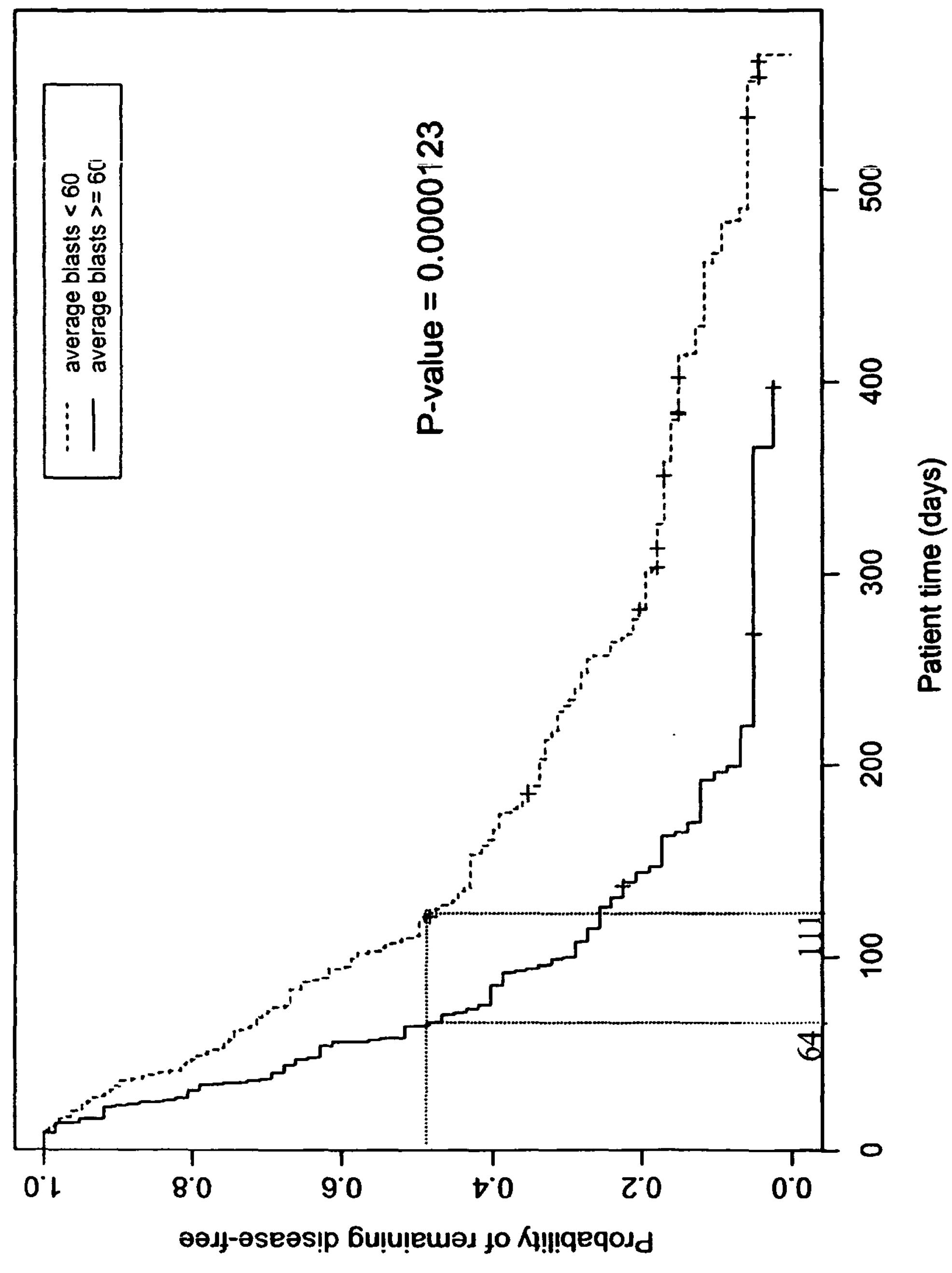
average blasts < 60
average blasts >= 60
P-value = 0.0000123
Probability of remaining disease-free
1.0
0.8
0.6
0.4
0.2
0.0
Patient time (days)
0
100
200
300
400
500
64
111

Fig 2.

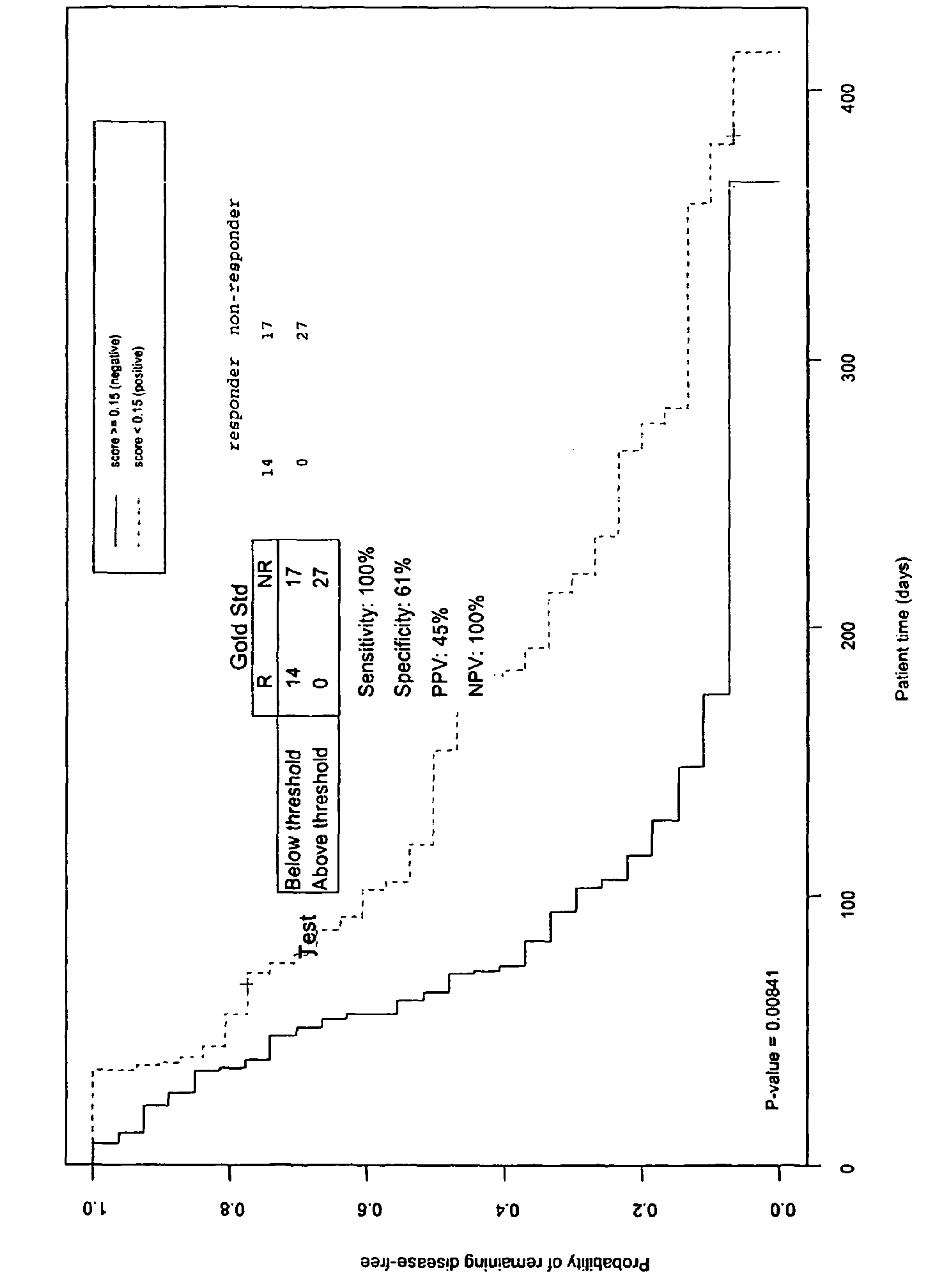
score >= 0.15 (negative)
score < 0.15 (positive)
responder non-responder
14 17
0 27
Gold Std
R NR
Below threshold 14 17
Above threshold 0 27
Test
Sensitivity: 100%
Specificity: 61%
PPV: 45%
NPV: 100%
P-value = 0.00841
Probability of remaining disease-free
Patient time (days)
0
100
200
300
400
0.0
0.2
0.4
0.6
0.8
1.0

Fig. 3A

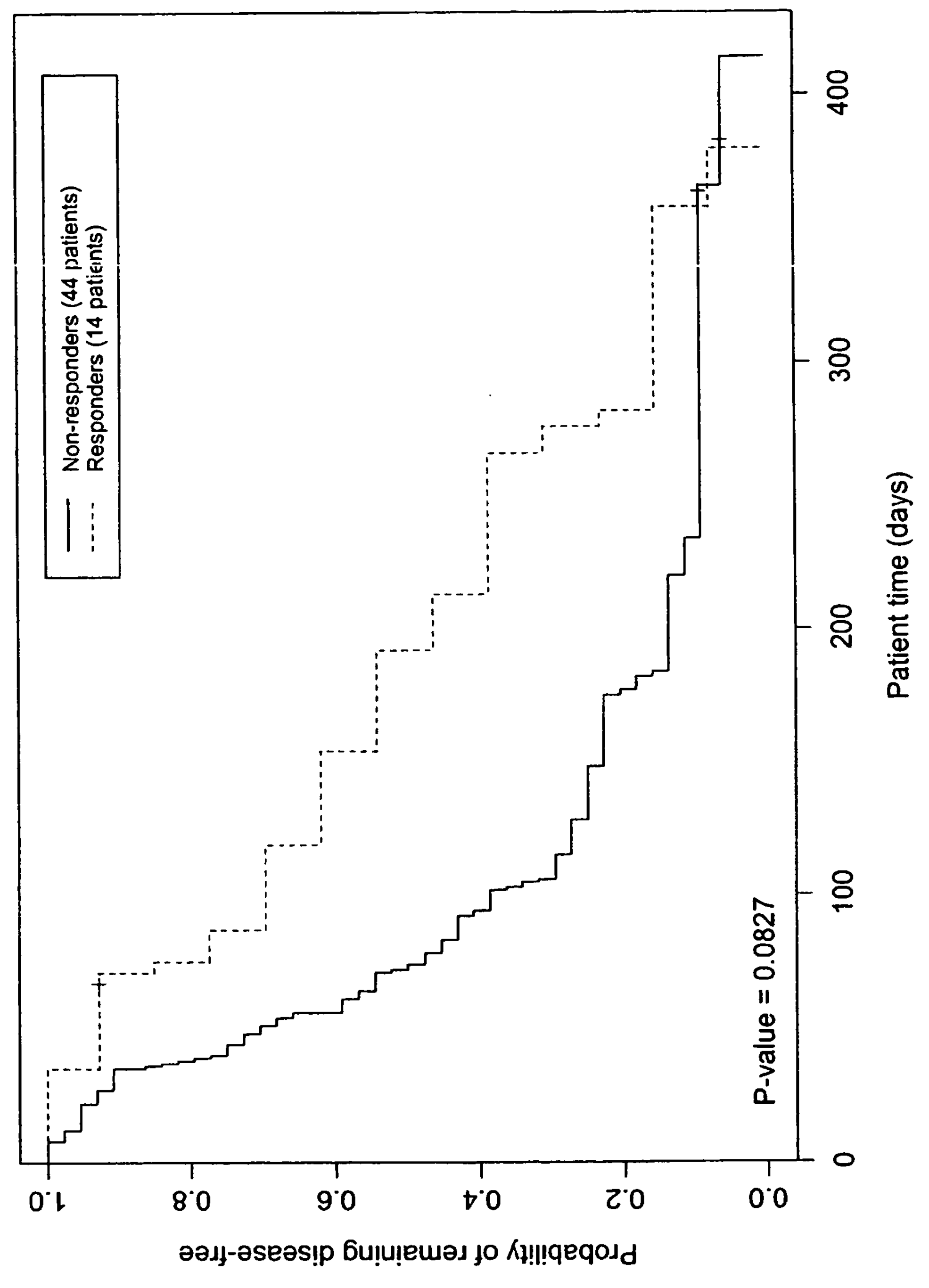
Non-responders (44 patients)
Responders (14 patients)
P-value = 0.0827
Probability of remaining disease-free
1.0
0.8
0.6
0.4
0.2
0.0
Patient time (days)
0
100
200
300
400

Fig. 3B

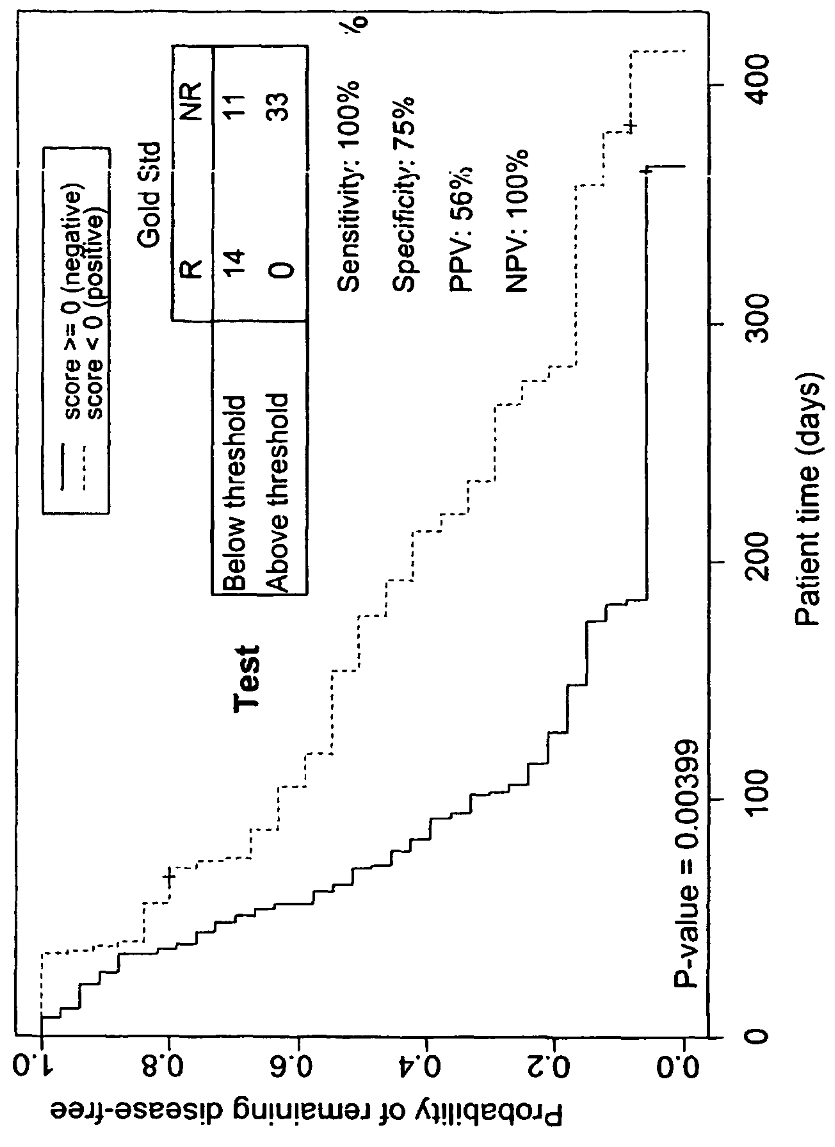
score >= 0 (negative)
score < 0 (positive)
Test
Gold Std
R
NR
Below threshold
14
11
Above threshold
0
33
Sensitivity: 100%
Specificity: 75%
PPV: 56%
NPV: 100%
P-value = 0.00399
Probability of remaining disease-free
Patient time (days)
0.0
0.2
0.4
0.6
0.8
1.0
0
100
200
300
400

Figure 3C

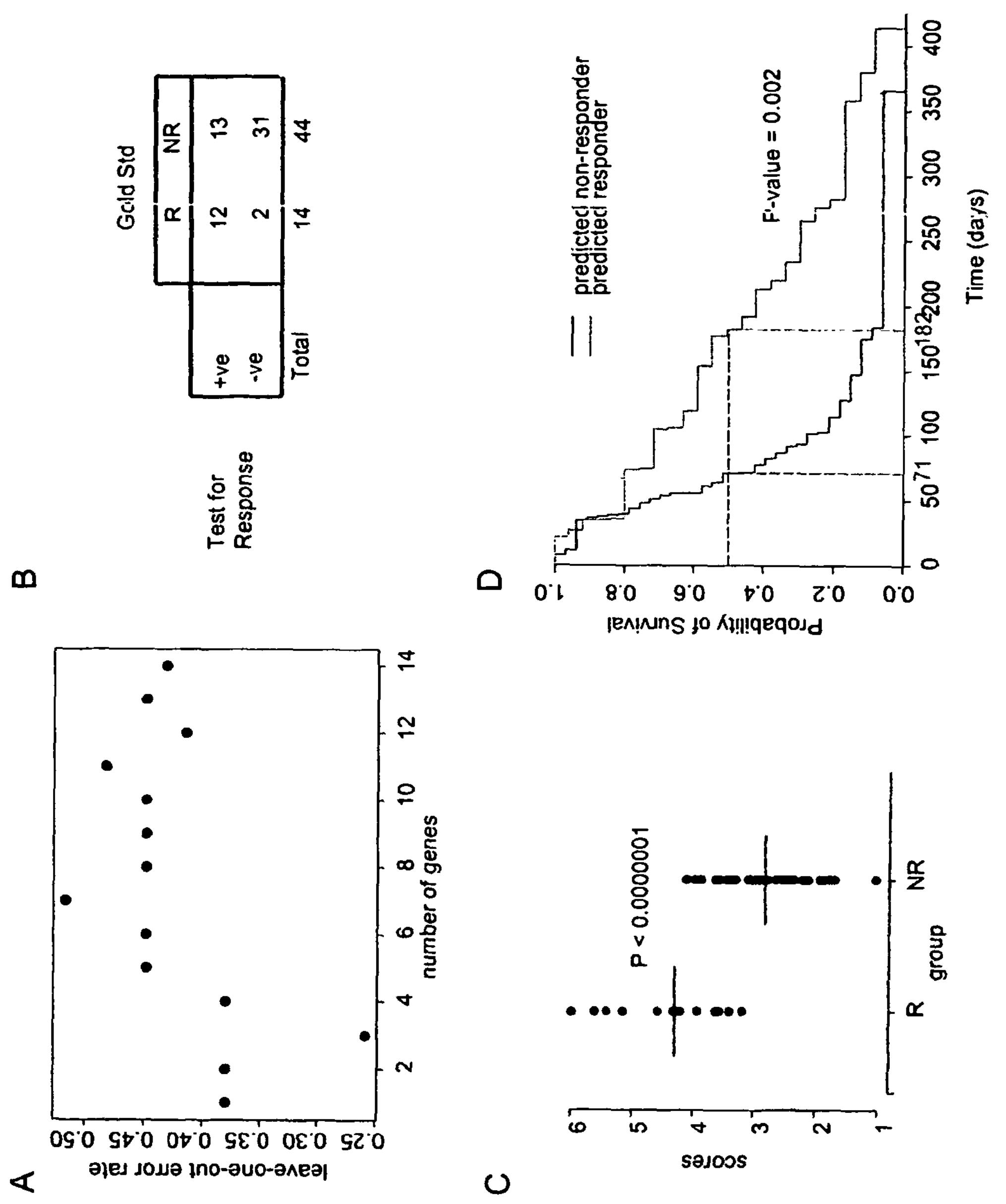
A
leave-one-out error rate
0.25 0.30 0.35 0.40 0.45 0.50
2 4 6 8 10 12 14
number of genes
B
Test for Response
Gold Std
R NR
+ve 12 13
-ve 2 31
Total 14 44
C
scores
1 2 3 4 5 6
P < 0.0000001
R NR
group
D
Probability of Survival
0.0 0.2 0.4 0.6 0.8 1.0
predicted non-responder
predicted responder
P-value = 0.002
0 50 71 100 150 182 200 250 300 350 400
Time (days)

Fig 4

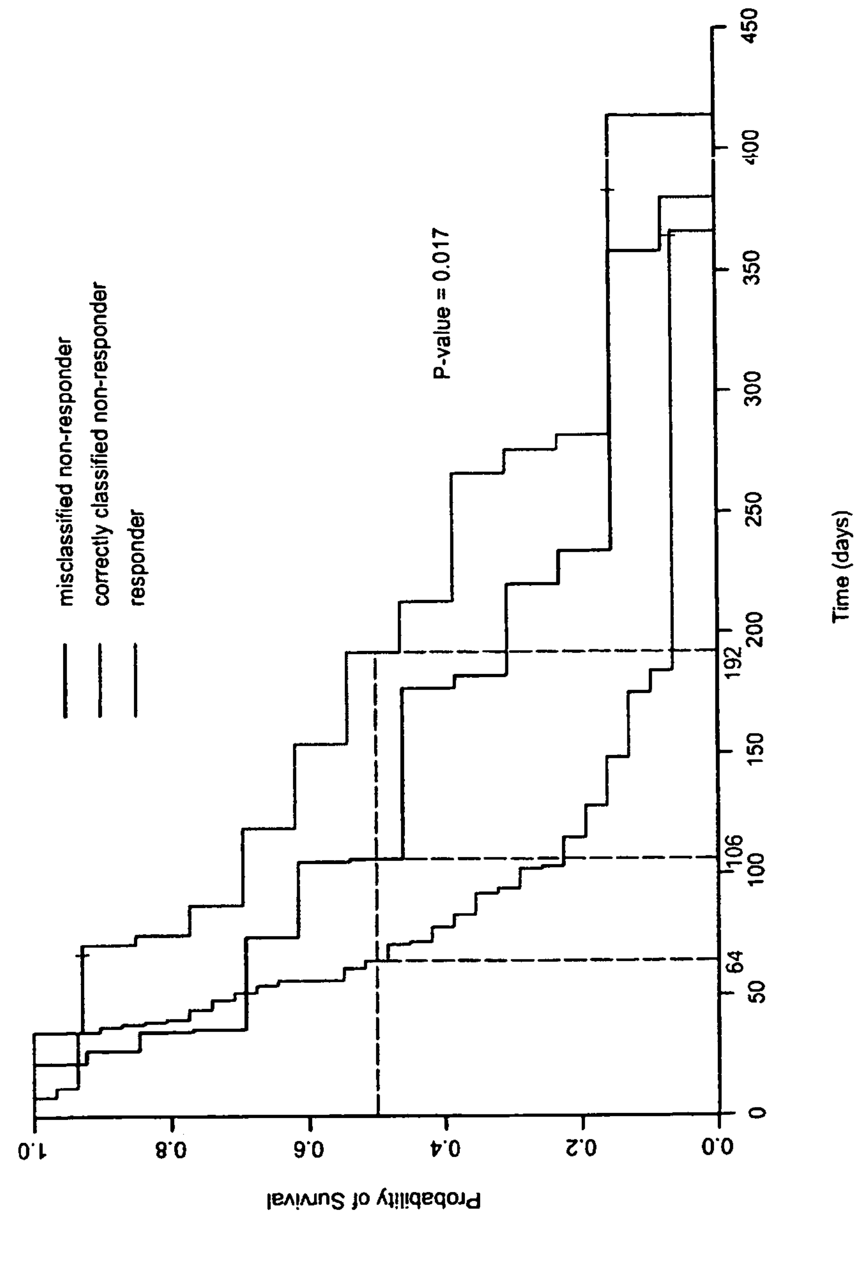
misclassified non-responder
correctly classified non-responder
responder
P-value = 0.017
Probability of Survival
1.0
0.8
0.6
0.4
0.2
0.0
0
50
64
100
106
150
192
200
250
300
350
400
450
Time (days)

Fig 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03038129 A
- US 5976851 A, Brown
- US 5972984 A, Anthony
- US 5972966 A, deSolms
- US 5968965 A, Dinsmore
- US 5968952 A, Venet
- US 6187786 A, Venet
- US 6169096 A, Venet
- US 6037350 A, Venet
- US 6177432 A, Angibaud
- US 5965578 A, Graham
- US 5965539 A, Sebti
- US 5958939 A, Afonso
- US 5939557 A, Anthony
- US 5936097 A, Commercon
- US 5891889 A, Anthony
- US 5889053 A, Baudin
- US 5880140 A, Anthony
- US 5872135 A, deSolms
- US 5869682 A, deSolms
- US 5861529 A, Baudoin
- US 5859015 A, Graharn
- US 5856439 A, Clere
- US 5856326 A, Anthony
- US 5852010 A, Graham
- US 5843941 A, Marsters
- US 5807852 A, Doll
- US 5780492 A, Dinsmore
- US 5773455 A, Dong
- US 5767274 A, Kim
- US 5756528 A, Anthony
- US 5750567 A, Baudoin
- US 5721236 A, Dishop
- US 5700806 A, Doll
- US 5661161 A, Anthony
- US 5602098 A, Sehti
- US 5585359 A, Breslin
- US 5578629 A, Ciccarone
- US 5534537 A, Ciccarone
- US 5532359 A, Marsters
- US 5523430 A, Patel
- US 5501212 A, de Solms
- US 5491164 A, deSolms
- US 5420245 A, Brown
- US 5238922 A, Graham
- US 6420387 B, Venet
- WO 0198302 A
- WO 9828303 A
- WO 9945912 A
- US 5874442 A
- WO 0001691 A
- WO 9410138 A
- WO 9730992 A
- US 5747498 A
- WO 0012499 A
- EP 430402 A
- US 5310687 A
- US 5238808 A
- US 5221605 A
- US 6271002 B, Linsley
- US 6718122 B, Friend
- US 6218114 B, Peck
- US 6004755 B, Wang
- US 10283975 B
- US 6306897 B
- US 6284764 B
- US 6133305 A
- US 6271210 B
- WO 0001411 A
- US 10883436 B


### Non-patent literature cited in the description


- **Albertson.** *EMBO J.,* 1984, vol. 3, 1227-1234
- **Pinkel.** *Proc. Natl. Acad. Sci. USA,* 1988, vol. 85, 9138-9142
- Methods in Molecular Biology. In Situ Hybridization Protocols. Humana Press, 1994, vol. 33
- **Pinkel et al.** *Nature Genetics,* 1998, vol. 20, 207-211
- **Kallioniemi.** *Proc. Natl. Acad. Sci. USA,* 1992, vol. 89, 5321-5325
- Laboratory Techniques in Biochemistry and Molecular Biology. **Tijssen.** Hybridization With Nucleic Acid Probes. Elsevier, 1993, vol. 24
- **Strachan ; Read.** *Human Molecular Genetics,* 1996
- Gene Therapy Protocols. Human press, 1996

SEQUENCE LISTING

<110> VERIDEX, LLC
RAPONI, MITCH

<120> METHODS FOR ASSESSING AND TREATING CANCER

<130> P041089EP

<150> 10/883,436
<151> 2004-07-01

<160> 29

<170> PatentIn version 3.2

<210> 1
<211> 5496
<212> DNA
<213> Human

<400> 1

```
attcagccgg tgcgcgcggc ggcgggaggc agtggctggg gagtcccgtc gacgctctgt     60
tccgagagcg tgccccggac cgccagctca gaacaggggc agccgtgtag ccgaacggaa    120
gctgggagca gccgggactg gtggcccgcg cccgagctcc gcaggcggga agcaccctgg    180
atttgggaag tcccgggagc agcgcggcgg cacctccctc acccaagggg ccgcggcgac    240
ggtcacgggg cgcggcgcca ccgtgagcga cccaggccag gattctaaat agacggccca    300
ggctcctcct ccgcccgggc cgcctcacct gcgggcattg ccgcgccgcc tccgccggtg    360
tagacggcac ctgcgccgcc ttgctcgcgg gtctccgccc ctcgcccacc ctcactgcgc    420
caggcccagg cagctcacct gtactggcgc gggctgcgga agcctgcgtg agccgaggcg    480
ttgaggcgcg gcgcccacgc cactgtcccg agaggacgca ggtggagcgg gcgcggcttc    540
gcggaacccg gcgccggccg ccgcagtggt cccagcctac accgggttcc ggggacccgg    600
ccgccagtgc ccggggagta gccgccgccg tcggctgggc accatgaaca gcagcagcgc    660
caacatcacc tacgccagtc gcaagcggcg gaagccggtg cagaaaacag taaagccaat    720
cccagctgaa ggaatcaagt caaatccttc caagcggcat agagaccgac ttaatacaga    780
gttggaccgt ttggctagcc tgctgccttt cccacaagat gttattaata agttggacaa    840
actttcagtt cttaggctca gcgtcagtta cctgagagcc aagagcttct ttgatgttgc    900
attaaaatcc tcccctactg aaagaaacgg aggccaggat aactgtagag cagcaaattt    960
cagagaaggc ctgaacttac aagaaggaga attcttatta caggctctga atggctttgt   1020
attagttgtc actacagatg ctttggtctt ttatgcttct tctactatac aagattatct   1080
agggtttcag cagtctgatg tcatacatca gagtgtatat gaacttatcc ataccgaaga   1140
ccgagctgaa tttcagcgtc agctacactg ggcattaaat ccttctcagt gtacagagtc   1200
```

tggacaagga attgaagaag ccactggtct cccccagaca gtagtctgtt ataacccaga 1260
ccagattcct ccagaaaact ctcctttaat ggagaggtgc ttcatatgtc gtctaaggtg 1320
tctgctggat aattcatctg gttttctggc aatgaatttc caagggaagt taaagtatct 1380
tcatggacag aaaaagaaag ggaaagatgg atcaatactt ccacctcagt tggctttgtt 1440
tgcgatagct actccacttc agccaccatc catacttgaa atccggacca aaaattttat 1500
ctttagaacc aaacacaaac tagacttcac acctattggt tgtgatgcca aaggaagaat 1560
tgttttagga tatactgaag cagagctgtg cacgagaggc tcaggttatc agtttattca 1620
tgcagctgat atgctttatt gtgccgagtc ccatatccga atgattaaga ctggagaaag 1680
tggcatgata gttttccggc ttcttacaaa aaacaaccga tggacttggg tccagtctaa 1740
tgcacgcctg ctttataaaa atggaagacc agattatatc attgtaactc agagaccact 1800
aacagatgag gaaggaacag agcatttacg aaaacgaaat acgaagttgc cttttatgtt 1860
taccactgga gaagctgtgt tgtatgaggc aaccaacccct tttcctgcca taatggatcc 1920
cttaccacta aggactaaaa atggcactag tggaaaagac tctgctacca catccactct 1980
aagcaaggac tctctcaatc ctagttccct cctggctgcc atgatgcaac aagatgagtc 2040
tatttatctc tatcctgctt caagtacttc aagtactgca ccttttgaaa acaacttttt 2100
caacgaatct atgaatgaat gcagaaattg gcaagataat actgcaccga tgggaaatga 2160
tactatcctg aaacatgagc aaattgacca gcctcaggat gtgaactcat ttgctggagg 2220
tcacccaggg ctctttcaag atagtaaaaa cagtgacttg tacagcataa tgaaaaacct 2280
aggcattgat tttgaagaca tcagacacat gcagaatgaa aaatttttca gaaatgattt 2340
ttctggtgag gttgacttca gagacattga cttaacggat gaaatcctga cgtatgtcca 2400
agattcttta agtaagtctc ccttcatacc ttcagattat caacagcaac agtccttggc 2460
tctgaactca agctgtatgg tacaggaaca cctacatcta gaacagcaac agcaacatca 2520
ccaaaagcaa gtagtagtgg agccacagca acagctgtgt cagaagatga agcacatgca 2580
agttaatggc atgtttgaaa attggaactc taaccaattc gtgcctttca attgtccaca 2640
gcaagaccca caacaatata atgtctttac agacttacat gggatcagtc aagagttccc 2700
ctacaaatct gaaatggatt ctatgcctta tacacagaac tttatttcct gtaatcagcc 2760
tgtattacca caacattcca aatgtacaga gctggactac cctatgggga gttttgaacc 2820
atccccatac cccactactt ctagtttaga agattttgtc acttgtttac aacttcctga 2880
aaaccaaaag catggattaa atccacagtc agccataata actcctcaga catgttatgc 2940
tggggccgtg tcgatgtatc agtgccagcc agaacctcag cacacccacg tgggtcagat 3000
gcagtacaat ccagtactgc caggccaaca ggcattttta aacaagtttc agaatggagt 3060

tttaaatgaa acatatccag ctgaattaaa taacataaat aacactcaga ctaccacaca 3120
tcttcagcca cttcatcatc cgtcagaagc cagacctttt cctgatttga catccagtgg 3180
attcctgtaa ttccaagccc aattttgacc ctggtttttg gattaaatta gtttgtgaag 3240
gattatggaa aaataaaact gtcactgttg gacgtcagca agttcacatg gaggcattga 3300
tgcatgctat tcacaattat tccaaaccaa attttaattt ttgcttttag aaaagggagt 3360
ttaaaaatgg tatcaaaatt acatatacta cagtcaagat agaaagggtg ctgccacgga 3420
gtggtgaggt accgtctaca tttcacatta ttctgggcac cacaaaatat acaaaacttt 3480
atcagggaaa ctaagattct tttaaattag aaaatattct ctatttgaat tatttctgtc 3540
acagtaaaaa taaaatactt tgagttttga gctactggat tcttattagt tccccaaata 3600
caaagttaga gaactaaact agtttttcct atcatgttaa cctctgcttt tatctcagat 3660
gttaaaataa atggtttggt gctttttata aaaagataat ctcagtgctt tcctccttca 3720
ctgtttcatc taagtgcctc acattttttt ctacctataa cactctagga tgtatatttt 3780
atataaagta ttcttttct tttttaaatt aatatctttc tgcacacaaa tattatttgt 3840
gtttcctaaa tccaaccaat tttcattaat tcaggcatat tttaactcca ctgcttacct 3900
actttcttca ggtaaaaggg caaataatga tcgaaaaaat aattatttat tacataattt 3960
agttgtttct agactataaa tgttgctatg tgccttatgt tgaaaaaatt taaaagtaaa 4020
atgtctttcc aaattatttc ttaattatta taaaaatatt aagacaatag cacttaaatt 4080
cctcaacagt gttttcagaa gaaataaata taccactctt tacctttatt gatatctcca 4140
tgatgatagt tgaatgttgc aatgtgaaaa atctgctgtt aactgcaacc ttgtttatta 4200
aattgcaaga agctttattt ctagcttttt aattaagcaa agcacccatt tcaatgtgta 4260
taaattgtct ttaaaaactg ttttagacct ataatccttg ataatatatt gtgttgactt 4320
tataaatttc gcttcttaga acagtggaaa ctatgtgttt ttctcatatt tgaggagtgt 4380
taagattgca gatagcaagg tttggtgcaa agtattgtaa tgagtgaatt gaatggtgca 4440
ttgtatagat ataatgaaca aaattatttg taagatattt gcagtttttc attttaaaaa 4500
gtccatacct tatatatgca cttaatttgt tggggcttta catactttat caatgtgtct 4560
ttctaagaaa tcaagtaatg aatccaactg cttaaagttg gtattaataa aaagacaacc 4620
acatagttcg tttaccttca aactttaggt ttttttaatg atatactgat cttcattacc 4680
aataggcaaa ttaatcaccc taccaacttt actgtcctaa catggacttt caaaaagaaa 4740
aaatgacacc atcttttatt cttttttttt ttttttttt gagagagagt cttactctgc 4800
cgcccaaact ggagtgcagt ggcacaatct tggctcactg caacctctac ctcctgggtt 4860

caagtgattc tcttgcctca gcctcccgag ttgctgggat tgcgggcatg gtggcgtgag 4920

cctgtagtcc tagctactcg ggaggctgag gcaggagaat agcctgaacc tgggaatcgg 4980

aggttgcagg gccaagatcg ccccactgca ctccagcctg gcaatagacc gagctccgtc 5040

tccaaaaaaa aaaatacaat ttttatttct tttacttttt ttagtaagtt aatgtatata 5100

aaaatggctt cggacaaaat atctctgagt tctgtgtatt ttcagtcaaa actttaaacc 5160

tgtagaatca atttaagtgt tgaaaaaaat ttgtctgaaa catttcataa tttgtttcca 5220

gcatgaggta tctaaggatt tagaccagag gtctagatta atactctatt tttacattta 5280

aacctttat tataagtctt acataaacca tttttgttac tctcttccac atgttactgg 5340

ataaattgtt tagtggaaaa taggcttttt aatcatgaat atgatgacaa tcagttatac 5400

agttataaaa ttaaaagttt gaaaagcaat attgtatatt tttatctata taaaataact 5460

aaaatgtatc taagaataat aaaatcacgt taaacc 5496

<210> 2
<211> 1737
<212> DNA
<213> Human

<400> 2

```
gtggtcacct tccccagcta gaaagccttc tttttatatc aacttccttt taattatgaa      60
gtattacaat caagcataaa gacataataa atgttaacat ctttcctttg tgtctgttta     120
aaaaatgttt cttattacag aaaatttcaa atagatttaa agtagagaaa tgcacgtgat     180
gaaaccccat gcacttgcca ttccatttca atagttatta cttcacggaa gagttggcaa     240
tgatgagaac atgtcaaaca cctggaaatt cctgtctcat tcaacagact cactaaataa     300
aatcagcaag gtcaatgagt caacagaatc acttactgat gagggtacag acatgaatga     360
aggacaacta ctgggagact ttgagattga gtccaaacag ctggaagcag agtcttggag     420
tcggataata gacagcaagt ttctaaaaca gcaaaagaaa gatgtggtca aacggcaaga     480
agtaatatat gagttgatgc agacagagtt tcatcatgtc cgcactctca agatcatgag     540
tggtgtgtac agccagggga tgatggcaga tctgcttttt gagcagcaga tggtagaaaa     600
gctgttcccc tgtttggatg agctgatcag tatccatagc caattcttcc agaggattct     660
ggagcggaag aaggagtctc tggtggataa aagtgaaaag aactttctca tcaagaggat     720
aggggatgtg cttgtaaatc agttttcagg tgagaatgca gaacgtttaa agaagacata     780
tggcaagttt tgtgggcaac ataaccagtc tgtaaactac ttcaaagacc tttatgccaa     840
ggataagcgt tttcaagcct ttgtaaagaa gaagatgagc agttcagttg ttagaaggct     900
tggaattcca gagtgcatat tgcttgtaac tcagcggatt accaagtacc cagttttatt     960

ccaaagaata ttgcagtgta ccaaagacaa tgaagtggag caggaagatc tagcacagtc    1020
cttgagcctg gtgaaggatg tgattggagc tgtagacagc aaagtggcaa gttatgaaaa    1080
gaaagtgcgt ctcaatgaga tttatacaaa gacagatagc aagtcaatca tgaggatgaa    1140
gagtggtcag atgtttgcca aggaagattt gaaacggaag aagcttgtac gtgatgggag    1200
tgtgtttctg aagaatgcag caggaaggtt gaaagaggtt caagcagttc ttctcactga    1260
cattttagtt ttccttcaag aaaaagacca gaagtacatc tttgcatcat tggaccagaa    1320
gtcaacagtg atctctttaa agaagctgat tgtgagagaa gtggcacatg aggagaaagg    1380
tttattcctg atcagcatgg ggatgacaga tccagagatg gtagaagtcc atgccagctc    1440
caaagaggaa cgaaacagct ggattcagat cattcaggac acaatcaaca cccctttccgg    1500
gaatggatgg aggtgcttta actgatgcga gtctcacaga gtcctatttc agcaccagct    1560
ttattggagt caatggattt ggaagccctg tagaaacaaa gtatcccttg atgcagtaca    1620
ctctggttgt cttccaacgt cttcgggtct gcacctctgg attcagcaat accaaaaaca    1680
ccggccccca aacttcccac ctacttgaac caataaactt cctcttgtgt ttaaact       1737
```

<210> 3
<211> 1051
<212> DNA
<213> Human

<220>
<221> misc_feature
<222> (23)..(23)
<223> n=any nucleotide

<220>
<221> misc_feature
<222> (26)..(26)
<223> n=any nucleotide

<400> 3

```
agtaaatcag gtaggaaatc acnttngwyt twkcttccag tagtcattac caccttttac     60
tgcacaattc acaagcatgt tttcctggtg aattggactg aaaattacat tttgacaact    120
ttttttcctt ttatccccaa cttttgccag aaagcagaaa aatgctctat ttttataaag    180
aaagattaaa ttctccaatg atattttaaa aaatatcaac ctacatgccc tttagaatgt    240
taaataacaa tgactatttt aagactcgaa gacccactat tttgagtatt ttttatagac    300
tttaaatact gggttttttt cctccttcaa tctcaggctt ttctccatct tttaagcggc    360
ctctgtkact ccctttgtc cataggtgtt gcgtgcctct catctgtggg gaagtattat    420
ttaataaaat ttatgttaca ggataacttt attttaatgg gcatgggctt gtctatacac    480
aaaggtatgt atattttcat tataaaaaac cagtttaaaa ttttttctta ttttaattgt    540

ttgaaatttt tctagagcca aagaagctct ttaaagaagt tgtttcttcc aaagaacaaa    600
gccaggttaa tgacattcaa ttctaaatga tacattggaa ttgtgccttt tctaccacac    660
tggattaaat aaacttgtca aaatatggtt ttgtcatttt ctgagacact tggtaatttg    720
ctgttctttc tttcatgtgc ccgttagtac atttggccaa ctgamcdkct gtaacaggga    780
ggtctcatgt ttgttagtag atacgcaggt aaggraactt gaattcatcc tctcctcggc    840
tcaggtcttt gcttttcttt taatatatgc atataaaata gtaggcattt gattctgcsa    900
aggcactaga ctacttgaaw ttaacattct gtccagaggg ataataccag gctttccttt    960
tccttcatct gttgtaagtt tcaggtcctt gacagaaata gctggactat tccaaatttg   1020
ctcagtgcat caaatgtgma aagggagcgg a                                  1051
```

<210> 4
<211> 1098
<212> DNA
<213> Human

<400> 4

```
ggccgcgtcg aagccgaaat gccgccaccc cggaccggcc gaggccttct ctggctgggt     60
ctggttctga gctccgtctg cgtcgccctc ggatccgaaa cgcaggccaa ctcgaccaca    120
gatgctctga acgttcttct catcatcgtg gatgacctgc gcccctccct gggctgttat    180
ggggataagc tggtgaggtc cccaaatatt gaccaactgg catcccacag cctcctcttc    240
cagaatgcct ttgcgcagca agcagtgtgc gccccgagcc gcgtttcttt cctcactggc    300
aggagacctg acaccacccg cctgtacgac ttcaactcct actggagggt gcacgctgga    360
aacttctcca ccatccccca gtacttcaag gagaatggct atgtgaccat gtcggtggga    420
aaagtctttc accctggtac tgctccatgt ccagagtctg ggttctcttg gtttgtggtg    480
tctgaatcca gcattcccat cctggggatg gggctgtctt tgcagagccc tcttctggct    540
gggcgagtcc ctcgctagtc agtgcttcct ttctaaaaaa ctgacttgtc aacccagcca    600
cgttttcacc caaagtgaaa aagggtagaa agagctttgc ttcctttcag aaaccactga    660
gggtgtgctg ttgggtcttt cagctcctcg ggtggtaggg aggacacagg ctggggaggt    720
ggcagtgttg ggtggagtcc agctcagggc cccaccctct cccctgcagg gtacctgtca    780
gtaaacctag gggtggggtg aggacacctg agggcctccc gtgtggccag cattgctgtt    840
gctgacttat tgcccaatga gggggctgtg cttaggtagg ggctgctatc cactctggaa    900
attaagtcag aaagatgagt gtaatctggt acccaggatc tatagggtcc cagagacgga    960
cattccttac ctcaaatgct gcctgataaa ctggctctct ttaatgccaa tatggggata   1020
gtgaaaagca aacaaacttt aaaacttttt atttatagaa gtaatgcatg agtatttgag   1080
aaaaaaaaaa aaaaaaa                                                  1098
```

<210> 5
<211> 2110
<212> DNA
<213> Human

<400> 5

```
cgagccccgc cgcaagctga gcgcctccgc ccgccaggcg cgccggcgcc gggccatgta      60
ctcggggaac cgcagcggcg gccacggcta ctgggacggc ggcggggccg cgggcgctga     120
ggggccggcg ccggcgggga cactgagccc cgcgcccctc ttcagccccg gcacctacga     180
gcgcctggcg ctgctgctgg gctccattgg gctgctgggc gtcggcaaca acctgctggt     240
gctcgtcctc tactacaagt tccagcggct ccgcactccc actcacctcc tcctggtcaa     300
catcagcctc agcgacctgc tggtgtccct cttcggggtc acctttacct tcgtgtcctg     360
cctgaggaac ggctgggtgt gggacaccgt gggctgcgtg tgggacgggt ttagcggcag     420
cctcttcggg attgtttcca ttgccaccct aaccgtgctg gcctatgaac gttacattcg     480
cgtggtccat gccagagtga tcaatttttc ctgggcctgg agggccatta cctacatctg     540
gctctactca ctggcgtggg caggagcacc tctcctggga tggaacaggt acatcctgga     600
cgtacacgga ctaggctgca ctgtggactg gaaatccaag gatgccaacg attcctcctt     660
tgtgcttttc ttatttcttg gctgcctggt ggtgcccctg ggtgtcatag cccattgcta     720
tggccatatt ctatattcca ttcgaatgct tcgttgtgtg gaagatcttc agacaattca     780
agtgatcaag attttaaaat atgaaaagaa actggccaaa atgtgctttt taatgatatt     840
caccttcctg gtctgttgga tgccttatat cgtgatctgc ttcttggtgg ttaatggtca     900
tggtcacctg gtcactccaa caatatctat tgtttcgtac ctctttgcta aatcgaacac     960
tgtatacaat ccagtgattt atgtcttcat gatcagaaag tttcgaagat cccttttgca    1020
gcttctgtgc ctccgactgc tgaggtgcca gaggcctgct aaagacctac cagcagctgg    1080
aagtgaaatg cagatcagac ccattgtgat gtcacagaaa gatggggaca ggccaaagaa    1140
aaaagtgact ttcaactctt cttccatcat ttttatcatc accagtgatg aatcactgtc    1200
agttgacgac agcgacaaaa ccattggggt ccaaagtttg atgttaatcc aagttcgtcc    1260
tttgtaggaa tgaaggatgg caacgaaagg tggggcctta aattggatgc cacttttgga    1320
ctttcatcat cctcctgaag aagaagtgtc tggaataccc gttctatgta atatcaacag    1380
aaccttgtgg tccagcagga aatccgaatt gcccatatgc tcttgggcct caggaagagg    1440
ttgaacaaaa acaaattctt ttaattcaac gggtgcttta cataatgaaa aaaccacttg    1500
tgcacacgat gggcatctaa catcatcatc ttctaatgtg ttggagattt tcatttcaaa    1560
```

```
tatatttttt aaattactct attttccaaa acacgtaatg catttttctc gaaaatacct   1620
tactgtaaaa ataactgtcg cgtacacatg tgtgaagtag ctagaacata ctgaattttt   1680
tttgtactgt tggactctat tcagtgtcat gtcctatatc tgatcaagtt atcaaggaga   1740
taattctaga atgaaaaaga aaatcctctt gttggaaaca aaagacgttt tatatgtgca   1800
gtatgacaaa gaggagtttc agagacaact ttgaatcctt gtcagcctgg agaccagcac   1860
cagaggaatc tacaaggcaa actcccatat atttgcttcc cccaaattgc tgcccctaca   1920
gactcaaagc tcttttctt tgttttgttg tttctctaaa aatttactgt tctttgtcga   1980
tgctatataa gccagggagt tctaagacgc cagctctttg agatttgctc attcccctgt   2040
atttcccaca tatatattac atatacccgc taataaattt atgtttgttt taaaaaaaaa   2100
aaaaaaaaaa                                                          2110
```

<210> 6
<211> 2416
<212> DNA
<213> Human

<400> 6

```
gaacagtgtt accttggagc ctacaatgag aggtatttca aaatgagtga agcatgactc     60
tcacagatga aggcctagac gcaggatctt taatggaaaa acacttgggc cacttcaaga    120
cgacaaacgc tcactgggca aaacaccttc actgaaaaga gacctcatat tatgcaaaaa    180
aaatcttaag aggcctctgc cttcagaagt tacaagatga tcaattcaac ctccacacag    240
cctccagatg aatcctgctc tcagaacctc ctgatcactc agcagatcat tcctgtgctg    300
tactgtatgg tcttcattgc gggaatccta ctcaatggag tgtcaggatg gatattcttt    360
tacgtgccca gctctaagag tttcatcatc tatctcaaga acattgttat tgctgacttt    420
gtgatgagcc tgacttttcc tttcaagatc cttggtgact caggccttgg tccctggcag    480
ctgaacgtgt ttgtgtgcag ggtctctgcc gtgctcttct acgtcaacat gtacgtcagc    540
attgtgttct ttgggctcat cagctttgac aggtattata aaattgtaaa gcctctttgg    600
acttctttca tccagtcagt gagttacagc aaacttctgt cagtgatagt atggatgctc    660
atgctcctcc ttgctgttcc aaatattatt ctcaccaacc agagtgttag ggaggttaca    720
caaataaaat gtatagaact gaaaagtgaa ctgggacgga agtggcacaa agcatcaaac    780
tacatcttcg tggccatctt ctggattgtg tttcttttgt taatcgtttt ctatactgct    840
atcacaaaga aaatctttaa gtcccacctt aagtcaagtc ggaattccac ttcggtcaaa    900
aagaaatcta gccgcaacat attcagcatc gtgtttgtgt tttttgtctg ttttgtacct    960
taccatattg ccagaatccc ctacacaaag agtcagaccg aagctcatta cagctgccag   1020
```

tcaaaagaaa tcttgcggta tatgaaagaa ttcactctgc tactatctgc tgcaaatgta 1080

tgcttggacc ctattattta tttctttcta tgccagccgt ttagggaaat cttatgtaag 1140

aaattgcaca ttccattaaa agctcagaat gacctagaca tttccagaat caaaagagga 1200

aatacaacac ttgaaagcac agatactttg tgagttccta ccctcttcca aagaaagacc 1260

acgtgtgcat gttgtcatct tcaattacat aacagaaatc aataagatat gtgccctcat 1320

cataaatatc atctctagca ctgccatcca atttagttca ataaaattca aatataagtt 1380

tccatgcttt tttgtaacat caaagaaaac atacccatca gtaatttctc taatactgac 1440

ctttctattc tctattaata aaaaattaat acatacaatt attcaattct attatattaa 1500

aataagttaa agtttataac cactagtctg gtcagttaat gtagaaattt aaatagtaaa 1560

taaaacacaa cataatcaaa gacaactcac tcaggcatct tctttctcta aataccagaa 1620

tctagtatgt aattgttttc aacactgtcc ttaaagacta acttgaaagc aggcacagtt 1680

tgatgaaggg ctagagagct gtttgcaata aaaagtcagg tttttttcct gatttgaaga 1740

agcaggaaaa gctgacaccc agacaatcac ttaagaaacc ccttattgat gtatttcatg 1800

gcactgcaaa ggaagaggaa tattaattgt atacttagca agaaaatttt ttttttctga 1860

tagcactttg aggatattag atacatgcta aatatgtttt ctacaaagac ttacgtcatt 1920

taatgagcct ggggttctgg tgttagaata tttttaagta ggctttactg agagaaacta 1980

aatattggca tacgttatca gcaacttccc ctgttcaata gtatgggaaa aataagatga 2040

ctgggaaaaa gacacaccca caccgtagaa catatattaa tctactggcg aatgggaaag 2100

gagaccattt tcttagaaag caaataaact tgattttttt aaatctaaaa tttacattaa 2160

tgagtgcaaa ataacacata aaatgaaaat tcacacatca cattttctg gaaaacagac 2220

ggattttact tctggagaca tggcatacgg ttactgactt atgagctacc aaaactaaat 2280

tctttctctg ctattaactg gctagaagac attcatctat ttttcaaatg ttctttcaaa 2340

acatttttat aagtaatgtt tgtatctatt tcatgcttta ctgtctatat actaataaag 2400

aaatgtttta atactg 2416

<210> 7
<211> 3763
<212> DNA
<213> Human

<400> 7

```
acttccaagt tctggtacaa ggcggatatt tcaagagaac aagccatcgc catgttgaag     60
gacaaggagc cgggctcatt cattgttcga gacagccatt ccttccgagg ggcttatggc    120
ctggccatga aggtggccac gcccccacct tcagtcctgc agctgaacaa gaaagctgga    180
```

```
gatttggcca atgaactcgt ccggcacttt ttgatcgagt gtaccccgaa gggagtgcgg     240
ttgaaagggt gctcgaatga accatatttc gggagcctga cggccttggt gtgccagcat     300
tccatcacgc ccttggcctt gccgtgcaag ctgcttatcc cagagagaga tccattggag     360
gaaatagcag aaagttctcc ccagacggca gccaattcag cagctgagct gttgaagcag     420
ggggcagcct gcaacgtgtg gtacttgaac tctgtggaga tggagtccct caccggccac     480
caggcgatcc agaaggccct gagcatcacc ctggtccagg agcctccacc tgtgtccaca     540
gttgtgcact tcaaggtgtc agcccagggc atcaccctga cagacaatca gaggaagctc     600
ttcttccgga ggcattaccc cgtgaacagt gtgattttct gtgccttgga cccacaagac     660
aggaagtgga tcaaagatgg cccttcctca aaagtctttg gatttgtggc ccggaagcag     720
ggcagtgcca cggataatgt gtgccacctg tttgcagagc atgaccctgt gcagcctgcc     780
agtgccattg tcaacttcgt atcaaaggtc atgattggtt ccccaaagaa ggtctgagaa     840
ctcccctccc tccctggacc caccgatgcc tctcgaagcc ctggagacag ccgttgggtg     900
agggtggggc ccccactttt taccaaacta gtaaacctga cattccaggc ccatgagggg     960
aaagaggatc ttccagctct gcaaaaacaa gaacaaacaa catcaccgtg aattggcctt    1020
tcctgaaagt gacttatctg acacatctct gtagccacat gctttttggg tagaagaagc    1080
tgggcatggg tgcaccccac cccctagggt ccccatggga aagggacatg caaggaaaca    1140
gcacagaaca cgaggtggtc cccatgtccc tggcacacta gcattctggg ggatgaggaa    1200
tccccagccc ttgaggcaga ggtgccgagt gactgccatg cttcgcccgt ccgcatgggc    1260
gcttctgtcc agctgcaccc gaggccgggg gtttccctca cctcggtctt cccaagatgg    1320
agatgctaac gaaactgaga agggggcgta tgtttgacga aggtttgtgc aagtcaggcc    1380
cttctggaac acagcagggc ctacaacgag gggcctttgc gatgggctgt gaggatgggg    1440
gtggtgggaa gaattggcca cgttagagac cccatgccac cccaccatgg tgagtgctct    1500
gtgcctcctg ctcacctgtg gtgagctggg cgagctgggc gagctgggcg agctgggctg    1560
gggagagcct gtgaggaccg agaggagaaa tgagaagaag gaacaaaaat attatttcta    1620
tgtaatttat attttactta tgccaaatta tttatgataa tttgccattg ctatactgta    1680
ccagtgtcaa atgctgcagc ctgccaagct gtgattttgt gaggcttgtc cctatgtagg    1740
atgcaccgca ggcccctggc cactgaaaga gtgtgcagtg gactgtgggt ctcccatatg    1800
cggtgccgcc caaaggtggc tttgcctcaa gcaacctacc ctgatgtttt actcattgga    1860
atgtttttcc ccgattgtgg atgacttctt ttctgatgga gagagtccag gagggatgga    1920
aaactcctgg atttaagctc agcatccccc acatgggctt ttcgatcatc ttcaggcctg    1980
```

```
aagctgcacg acctgaagtt cgcctgcatt tatcagccct tttgtgctgc tccttgccac   2040
cttggggttc ctgctgggga ccatgtgtgg ttgtggcatg tgtgagcaga agggaggatg   2100
aggaaaaaga gaagaaaccc cggtactgac aagctgtttt tgagtgccac tgtttgccat   2160
catctaagcc actgaatcaa gtgtatttca ggcttatttc aacattccaa tgccctggtt   2220
ttcctgcttg aatctgttcg tggtcaaagg tttgggggaa tttgtgaccc tggaccatcc   2280
ccagagtgaa agatggagct gggccacatc agaataaggc cttggcccca tcctctcaca   2340
gcctaggtgc tctgcaggca tactgactgt cctgattgcg atccagcccg aaattccctc   2400
ctctgctttc aaaagtcaaa tcccccattc ttaggccaca ctggtgtcac aagctcctgt   2460
cagggagctg gggtttggga atgtgctttg tgaactctgc tttaaagtga ggggccgagg   2520
aaaacttaga aacaggcaga gttggaagca gccaaatcac agtgggtgtt gtgtgtgtgt   2580
gcgtgtgtgc atgcgtgcgt gtatgcgtgt gtgaaagcag gtggaccatt ccacttttta   2640
gctcctattg atgcaccaaa ccaagtgcct catttctgtg ccaaatgttt gccttggtcg   2700
tcgtggacct ccttctctaa cttgaggtgg catgactgtc aggaggtgct ggcattttca   2760
gcagatcctc atgtgttgac cctgatgtct ttagcagagg cctctagcat ctcggttttt   2820
catccactgc aggaatgtgg ccacagggag cagaggtttg tactttcccc aagaggtcct   2880
catcctgaga cggtctctac ccatgtttaa cccaaagagt gcaggccagg ttccttatcc   2940
ttctgatgaa ggatgagaga gctcatttag aagtcagagc aaactagggt ctcagtattg   3000
agaaacgcag cctgccaggg aatcacagag acatcggggt gcccgcgatg gccctcatga   3060
agccatgcct cgacggcatt caggaagccc tgcaaacgtg ctttttgaac tcattggcca   3120
ggtgtgattt ttacacaagg taaacgtggt caagggcatc ggggaatttg ctccaagcag   3180
atagctccct ctgaggaacc aaaggaagca agtttccatg atttctgaag agctggtata   3240
ggaagtttct ttcttccttt tgtgttacat gtgcattaaa cagaacaagc tgtgtgtcat   3300
cacagattgt actgtgggct cagaaaccgt gagagagccc ccaccgtgga caccggctct   3360
atggccacag gaaaaggaac gtttccaggc attttgtctc cagggctccc gctggacagg   3420
cacgtactgc cccggggagt aaatgcggag agttcacgaa ctgtgcccaa cgcatgttat   3480
agccagggtc ctactaacta ctcagtaaaa gaacgtattg ttgtattcct ccagtgttaa   3540
gctatagcca tgttaaaagt cactgtgcat ttattctcag catcaaatac cttgtaacgt   3600
cttctctgcc ttgttagtgc atatttttac ttttctgata ctgtaaagaa tatatccagt   3660
atgtaaatga atgttctata aatcttttgt atagtcattt tctctgctcc ttaaatatca   3720
tctctattca gagtataata aaattatgaa cttggtaagc ctc                     3763
```

<210> 8
<211> 1684
<212> DNA
<213> Human

<400> 8

```
cgacggagtg ccaggagcac taacagtacc cttagcttgc tttcctcctc cctccttttt     60
attttcaagt tcctttttat ttctccttgc gtaacaacct tcttcccttc tgcaccactg    120
cccgtaccct tacccgcccc gccacctcct tgctacccca ctcttgaaac cacagctgtt    180
ggcagggtcc ccagctcatg ccagcctcat ctcctttctt gctagccccc aaagggcctc    240
caggcaacat gggggcccca gtcagagagc cggcactctc agttgccctc tggttgagtt    300
ggggggcagc tctgggggcc gtggcttgtg ccatggctct gctgacccaa caaacagagc    360
tgcagagcct caggagagag gtgagccggc tgcaggggac aggaggcccc tcccagaatg    420
gggaagggta tccctggcag agtctcccgg agcagagttc cgatgccctg gaagcctggg    480
agaatgggga gagatcccgg aaaaggagag cagtgctcac ccaaaaacag aagaatgact    540
ccgatgtgac agaggtgatg tggcaaccag ctcttaggcg tgggagaggc ctacaggccc    600
aaggatatgg tgtccgaatc caggatgctg gagtttatct gctgtatagc caggtcctgt    660
ttcaagacgt gactttcacc atgggtcagg tggtgtctcg agaaggccaa ggaaggcagg    720
agactctatt ccgatgtata agaagtatgc cctcccaccc ggaccgggcc tacaacagct    780
gctatagcgc aggtgtcttc catttacacc aaggggatat tctgagtgtc ataattcccc    840
gggcaagggc gaaacttaac ctctctccac atggaacctt cctggggttt gtgaaactgt    900
gattgtgtta taaaaagtgg ctcccagctt ggaagaccag ggtgggtaca tactggagac    960
agccaagagc tgagtatata aaggagaggg aatgtgcagg aacagaggcg tcttcctggg   1020
tttggctccc cgttcctcac ttttcccttt tcattcccac cccctagact ttgattttac   1080
ggatatcttg cttctgttcc ccatggagct ccgaattctt gcgtgtgtgt agatgagggg   1140
cgggggacgg gcgccaggca ttgttcagac ctggtcgggg cccactggaa gcatccagaa   1200
cagcaccacc atctagcggc cgctcgaggg aagcacccgc cggttggccg aagtccacga   1260
agccgccctc tgctagggaa aacccctggt tctccatgcc acacctctct ccaggtgccc   1320
tctgcctctt caccccacaa gaagccttat cctacgtcct tctctccatc tatcggaccc   1380
cagtttccat cactatctcc agagatgtag ctattatgcg cccgtctaca gggggtgccc   1440
gacgatgacg gtgccttcgc agtcaaatta ctcttcgggt cccaaggttt ggctttcacg   1500
cgctccattg ccccggcgtg gcaggccatt ccaagccctt ccgggctgga actggtgtcg   1560
gaggagcctc gggtgtatcg tacgccctgg tgttggtgtt gcctcactcc tctgagctct   1620
tctttctgat caagccctgc ttaaagttaa ataaaataga atgaatgata aaaaaaaaaa   1680
```

aaaa 1684

<210> 9
<211> 6719
<212> DNA
<213> Human

<400> 9

tcggcgggaa gcggcgatcc tgccaccggg aggtgtggaa gagccgggta gattctggct 60

acattggaga ttggttgctt tctaaaactg aaggagaagc ccatgaagag atggtggatt 120

ctcactgagt tttgactagc ggaagaaaag agagagttca agtggatggc cttgaggact 180

tgaaaagctg agatatgatg attttgaagt catttcacat cgaagccatg atttaaatat 240

cggcgttaag atttcaacaa gaaaaactta agcttccttg gattcccacg tcaaaggaaa 300

gtttcaagct ttcagaagga gttctcactc gaagataaag aacagctcgc taaccacgaa 360

agaggaatcg atgctcagct tttagttgca cttcctaaag ttgcagaatt aagacaaatc 420

tttgaaccaa agaagaaaga attcttagaa atgaaaagaa aagaaagaat tgccaggcgc 480

ctggaaggga ttgaaaatga cactcagccc atcctcttgc agagctgcac aggattggtg 540

actcaccgcc tgctggagga agacacccct cgatacatga gagccagcga ccctgccagc 600

ccccacatcg gccgatcaaa tgaagaggag gaaacttctg attcttctct agaaaagcaa 660

actcgatcca aatactgcac agaaacctcc ggtgtccacg gtgactcacc ctatggttcg 720

ggtaccatgg acacccacag tctggagtcc aaagccgaaa gaattgcaag gtacaaagca 780

gaaagaaggc gacagctggc agagaagtat gggctgactc tggatcccga ggccgactcc 840

gagtatttat cccgctatac caagtccagg aaggagcctg atgctgtcga gaagcgggga 900

ggaaaaagtg acaaacagga agagtcaagc agagatgcga gttctctgta ccccgggacc 960

gagacgatgg ggctcaggac ctgtgccggt gaatccaagg actatgccct ccatgtgggt 1020

gacggctctt ccgacccgga ggtgctgctg aacatagaaa accaaagacg aggtcaagag 1080

ctgagtgcca cccggcaggc ccatgacctg tccccagcag ccgagagttc ctcgaccttc 1140

tctttctctg ggcgagactc ctccttcact gaagtgccac ggtcccccaa gcacgcccac 1200

agctcctccc tgcagcaggc agcctcccgg agcccctcct ttggtgaccc acagctatcc 1260

cctgaggccc gacccaggtg cacttcacat tcagaaacgc caactgtcga tgatgaagaa 1320

aaggtggatg aacgagccaa gctgagcgtc gccgccaaga ggttgctttt cagggagatg 1380

gaaaaatctt ttgatgaaca aaatgttcca aagcgacgct caagaaacac agctgtggag 1440

cagaggctac gccgtctgca ggacaggtcc ctcacccagc ccatcaccac tgaagaggtg 1500

gtcatcgcag ccacattgca ggcctctgct caccaaaagg ccttagccaa ggaccagaca 1560

```
aatgagggca aagagcttgc tgagcaagga gaacctgatt cctccactct aagcttggcc   1620
gaaaagttgg ccttgtttaa caaattgtcc cagccagtct caaaagcgat ttctacccgg   1680
aacagaatag acacgagaca gaggagaatg aacgctcgct atcaaactca gccagtcaca   1740
ctgggagagg tggagcaggt gcagagtgga aagctcattc ctttctcacc tgccgtgaac   1800
acatcagtgt ctaccgtagc atccacggtt gctccaatgt atgccggaga tcttcgcaca   1860
aagccacctc ttgaccacaa tgcaagtgcc actgactata agttttcttc ttcaatagaa   1920
aattcggact ctccagttag aagcattctg aaatcgcaag cttggcagcc tttggtagag   1980
ggtagcgaga acaagggaat gttgagagaa tatggagaga cagaaagcaa gagagctttg   2040
acaggtcgag acagtgggat ggagaagtat gggtcctttg aggaagcaga agcatcctac   2100
cccatcctga accgagccag ggaaggagac agccataagg aatctaaata tgctgttccc   2160
agaagaggaa gcctggaacg ggcgaaccct cccatcaccc acctcgggga tgaaccgaag   2220
gaattttcca tggctaaaat gaatgcacaa ggaaacttgg acttgaggga caggctgccc   2280
tttgaagaga aggtggaggt ggagaatgtt atgaaaagga agttttcact aagagcggca   2340
gagttcgggg agcccacttc cgagcagacg gggacagctg ctgggaaaac tattgctcaa   2400
accacagccc ccgtgtcctg gaagccccag gattcttcgg aacagccaca ggagaagctc   2460
tgcaagaatc catgtgcgat gtttgctgct ggagagatca aaacgccgac aggggagggc   2520
cttcttgact cacccagcaa aaccatgtct attaaagaaa gattggcact gttgaagaaa   2580
agcggggagg aagattggag aaacagactc agcaggaggc aggagggcgg caaggcgccg   2640
gccagcagcc tgcacaccca ggaagcaggg cggtccctca tcaagaagcg ggtcacagaa   2700
agtcgagaga gccaaatgac gattgaggag aggaagcagc tcatcactgt gagagaggag   2760
gcctggaaga cgagaggcag aggagcggcc aacgactcga cccagttcac tgtggctggc   2820
aggatggtga agaaaggttt ggcgtcacct actgccataa ccccagtagc ctcagccatt   2880
tgcggtaaaa caagaggcac cacacccgtt tccaaacccc tggaagatat cgaagccaga   2940
ccagatatgc agttagaatc ggacctgaag ttggacaggc tggaaacctt tctaagaagg   3000
ctgaataaca aagttggcgg gatgcacgaa acggtgctca ctgtcaccgg caaatctgtg   3060
aaggaggtga tgaagccaga tgatgatgaa acctttgcca aattttaccg cagcgtggat   3120
tataatatgc caagaagtcc tgtggagatg gatgaggact tcgatgtcat tttcgatcct   3180
tatgcaccca aattgacgtc ttccgtggcc gagcacaagc gggcagttag gcccaagcgc   3240
cgggttcagg cctccaaaaa ccccctgaaa atgctggcgg caagagaaga tctccttcag   3300
gaatacactg agcagagatt aaacgttgcc ttcatggagt caaagcggat gaaagtagaa   3360
```

aagatgtctt ccaactccaa cttctcagaa gtcaccctgg cgggtttagc cagtaaagaa 3420
aacttcagca acgtcagcct gcggagcgtc aacctgacgg aacagaactc taacaacagc 3480
gccgtgccct acaagaggct gatgctgttg cagattaaag gaagaagaca tgtgcagacc 3540
aggctggtgg aacctcgagc ttcggcgctc aacagtgggg actgcttcct cctgctctct 3600
ccccactgct gcttcctgtg ggtaggagag tttgcaaacg tcatagaaaa ggcgaaggcc 3660
tcagaacttg caactttaat tcagacaaag agggaacttg gttgtagagc tacttatatc 3720
caaaccattg aagaaggaat taatacacac actcatgcag ccaaagactt ctggaagctt 3780
ctgggtggcc aaaccagtta ccaatctgct ggagacccaa aagaagatga actctatgaa 3840
gcagccataa tagaaactaa ctgcatttac cgtctcatgg atgacaaact tgttcctgat 3900
gacgactact gggggaaaat tccgaagtgc tcccttctgc aacccaaaga ggtactggtg 3960
tttgattttg gtagtgaagt ttacgtatgg catgggaaag aagtcacatt agcacaacga 4020
aaaatagcat ttcagctggc aaagcactta tggaatggaa cctttgacta tgagaactgt 4080
gacatcaatc ccctggatcc tggagaatgc aatccgctta tccccagaaa aggacagggg 4140
cggcccgact gggcgatatt tgggagactt actgaacaca atgagacgat tttgttcaaa 4200
gagaagtttc tggattggac ggaactgaag agatcgaatg agaagaaccc cggggaactt 4260
gcccagcaca aggaagaccc caggactgat gtcaaggcat acgatgtgac acggatggtg 4320
tccatgcccc agacgacagc aggcaccatc ctggacggag tgaacgtcgg ccgtggctat 4380
ggcctggtgg aaggacacga caggaggcag tttgagatca ccagcgtttc cgtggatgtc 4440
tggcacatcc tggaattcga ctatagcagg ctccccaaac aaagcatcgg gcagttccat 4500
gaggggggatg cctatgtggt caagtggaag ttcatggtga gcacggcagt gggaagtcgc 4560
cagaagggag agcactcggt gagggcagcc ggcaaagaga agtgcgtcta cttcttctgg 4620
caaggccggc actccaccgt gagtgagaag ggcacgtcgg cgctgatgac ggtggagctg 4680
gacgaggaaa gggggcccca ggtccaggtt ctccagggaa aggagccccc ctgtttcctg 4740
cagtgtttcc agggggggat ggtggtgcac tcggggaggc gggaagagga agaagaaaat 4800
gtgcaaagtg agtggcggct gtactgcgtg cgtggagagg tgcccgtgga agggaatttg 4860
ctggaagtgg cctgtcactg tagcagcctg aggtccagaa cttccatggt ggtgcttaac 4920
gtcaacaagg ccctcatcta cctgtggcac ggatgcaaag cccaggccca cacgaaggag 4980
gtcggaagga ccgctgcgaa caagatcaag gaacaatgtc ccctggaagc aggactgcat 5040
agtagcagca aagtcacaat acacgagtgt gatgaaggct ccgagccact cggattctgg 5100
gatgccttag gaaggagaga caggaaagcc tacgattgca tgcttcaaga tcctggaagt 5160
tttaacttcg cgccccgcct gttcatcctc agcagctcct ctggggattt tgcagccaca 5220

gagtttgtgt accctgcccg agccccctct gtggtcagtt ccatgccctt cctgcaggaa 5280
gatctgtaca gcgcgcccca gccagcactt ttccttgttg acaatcacca cgaggtgtac 5340
ctctggcaag gctggtggcc catcgagaac aagatcactg gttccgcccg catccgctgg 5400
gcctccgacc ggaagagtgc gatggagact gtgctccagt actgcaaagg aaaaaatctc 5460
aagaaaccag cccccaagtc ttaccttatc cacgctggtc tggagcccct gacattcacc 5520
aatatgtttc ccagctggga gcacagagag gacatcgctg agatcacaga gatggacacg 5580
gaagtttcca atcagatcac cctcgtggaa gacgtcttag ccaagctctg taaaaccatt 5640
tacccgctgg ccgacctcct ggccaggcca ctcccggagg gggtcgatcc tctgaagctt 5700
gagatctatc tcaccgacga agacttcgag tttgcactag acatgacgag ggatgaatac 5760
aacgccctgc ccgcctggaa gcaggtgaac ctgaagaaag caaaaggcct gttctgagtg 5820
gggagacgcc agaggagcct cacggtcacg tccaacaaca ccactgcacc agggaaatgg 5880
atatatattt ttggactggt gtttttcaca aagtattttt caatcagagt tttcagaacc 5940
tgacattgtt aaagatactg cttgtcccgg agttgtgtat tttgtaaatg ttcaagggaa 6000
ctgtttggaa acttctttcc accattcagg aggttatcag aattaataaa agtatctgtt 6060
atgtgcactt aagccgcagc tgctatagat agcactgcct tcttgttcca gctaggcaat 6120
gccttttttt ttttttttga agcagttctc tttataaagt gttattttga tagtttgtgg 6180
attctaaaat atatatatat ttatataaac accatataag tcaaatatgt atttaacaaa 6240
gcaatatgta ttcattcact ttcaagattt gttttggtgt caaaataaca tgaaaaggta 6300
gatggagttg cttctgttga attagctctg ccaccaatat gtatcttcat acacgtttgg 6360
aaatgtttcc tgcagcatta ggtatgactt gttctgagta ctgcttccgg tgctaaaatg 6420
aacaaagaat ttgtacttaa tggcatggac tctggagaat ctatgcgaat caacctttct 6480
accttaatat ctccccaaaa atgtatagtg ccttgttttt atgtacagtt tatatacaga 6540
aaagtttgct ctgcattttt gatgatggtt tggaacatta tctacaattt tactctcaaa 6600
tagtcaaaat aaaaacatct caatttctaa taccggttgt aaacaaacag tacacatgtc 6660
attttgtgat ataggactcc caaataaaag tatcagaata aacacaacaa ttaactggt 6719

<210> 10
<211> 1460
<212> DNA
<213> Human

<400> 10

gcacacggga agatatcaga aacatcctag gatcaggaca ccccagatct tctcaactgg 60
aaccacgaag gctgtttctt ccacacagca ctttgatctc catttaagca ggcacctctg 120

tcctgcgttc cggagctgcg ttcccgatgg tcctcctttg gctcacgctg ctcctgatcg 180
ccctgccctg tctcctgcaa acgaaggaag atccaaaccc accaatcacg aacctaagga 240
tgaaagcaaa ggctcagcag ttgacctggg accttaacag aaatgtgacc gatatcgagt 300
gtgttaaaga tgccgactat tctatgccgg cagtgaacaa tagctattgc cagtttggag 360
caatttcctt atgtgaagtg accaactaca ccgtccgagt ggccaaccca ccattctcca 420
cgtggatcct cttccctgag aacagtggga agccttgggc aggtgcggag aatctgacct 480
gctggattca tgacgtggat ttcttgagct gcagctgggc ggtaggcccg gggccccccg 540
cggacgtcca gtacgacctg tacttgaacg ttgccaacag gcgtcaacag tacgagtgtc 600
ttcactacaa aacggatgct cagggaacac gtatcgggtg tcgtttcgat gacatctctc 660
gactctccag cggttctcaa agttcccaca tcctggtgcg gggcaggagc gcagccttcg 720
gtatcccctg cacagataag tttgtcgtct tttcacagat tgagatatta actccaccca 780
acatgactgc aaagtgtaat aagacacatt cctttatgca ctggaaaatg agaagtcatt 840
tcaatcgcaa atttcgctat gagcttcaga tacaaaagag aatgcagcct gtaatcacag 900
aacaggtcag agacagaacc tccttccagc tactcaatcc tggaacgtac acagtacaaa 960
taagagcccg ggaaagagtg tatgaattct tgagcgcctg gagcaccccc cagcgcttcg 1020
agtgcgacca ggaggagggc gcaaacacac gtgcctggcg gacgtcgctg ctgatcgcgc 1080
tggggacgct gctggccctg gtctgtgtct tcgtgatctg cagaaggtat ctggtgatgc 1140
agagactctt tccccgcatc cctcacatga aagaccccat cggtgacagc ttccaaaacg 1200
acaagctggt ggtctgggag gcgggcaaag ccggcctgga ggagtgtctg gtgactgaag 1260
tacaggtcgt gcagaaaact tgagactggg gttcagggct tgtgggggtc tgcctcaatc 1320
tccctggccg ggccaggcgc ctgcacagac tggctgctgg acctgcgcac gcagcccagg 1380
aatggacatt cctaacgggt ggtgggcatg ggagatgcct gtgtaatttc gtccgaagct 1440
gccaggaaga agaacagaac 1460

<210> 11
<211> 4318
<212> DNA
<213> Human

<400> 11

```
gcttcccggc tgccaggcta cagaactcgc ctcgccactc ctgagacatg gacaacgccg      60
ttgacggact ggacaaagct tctatagcaa actcagatgg ccccacagca ggttcccaaa     120
cacctcccttt caaaagaaag gggaaactat ccaccattgg taaaatcttt aagccttgga    180
aatggaggaa aaagaagacc agcgacaaat ttagagaaac ctcggcagta ttagaaagga     240
```

agatatccac acgacaaagt agagaggagc tgataagaag gggagtgctt aaggaattgc 300
ctgatcaaga tggagatgta acagttaact ttgaaaattc aaacgggcac atgataccca 360
tcggagagga atctacccga gaggaaaatg tagtaaagtc tgaagaaggt aatggctctg 420
tatctgaaaa aacaccacct ctggaggaac aggcagaaga taagaaagag aacactgaaa 480
accactctga aacaccggca gctcctgctc tacctccttc tgctcctcct aagcctagat 540
ccaaacctaa acccaaaaaa tcacctgtgc ctccgaaagg ggccactgct ggggccagcc 600
acaaaggtga tgaagtgcct cccattaaaa aaaataccaa ggctcctggt aagcaggccc 660
ccgtccctcc acccaagcca gcaagccgaa acacgacccg agaggctgct ggctcctctc 720
attcaaaaaa aacaactggc tctaaagcat cagcttcgcc atccacttca tccacctcat 780
ctcgtcccaa agcttcaaag gagacagttt ctagcaaagc agggacagtg gggaccacca 840
agggcaagag aaaaactgac aagcagccaa taacttctca cctgtcctca gacacaacaa 900
cttctggcac atccgacctg aaaggagagc ctgcagagac cagagtggag agtttcaaac 960
tcgaacagac tgtccctgga gctgaggagc agaacacagg caaattcaag tccatggtcc 1020
ctccaccccc tgtggctcca gcaccttctc ctctggcccc ccctctccct cttgaggatc 1080
agtgcattac tgcctcagac actccagttg tcctcgtcag cgttggagct gacctgcccg 1140
tctctgcctt agacccaagt cagcttcttt gggctgaaga gccgacgaac agaaccactc 1200
tctactcagg cactggctta agtgttaaca gagaaaatgc aaaatgtttc acaaccaaag 1260
aggagctggg gaagacagtg cctcagctac tgactcctgg gctgatgggc gaatcttcag 1320
aatcctttag tgcctcagaa gatgaaggcc acagggaata ccaagccaat gactctgact 1380
cggacgggcc tatcttgtac accgatgatg aggacgaaga cgaagatgag gatggcagtg 1440
gagaaagtgc tttggcaagt aaaatacgcc ggagggatac tcttgctatc aaacttggca 1500
acagaccatc taagaaagaa ctagaggaca aaaacatctt gcagcgtaca tctgaagaag 1560
agaggcagga aatccgacaa caaattggaa ccaagttagt caggaggctg agccagaggc 1620
ccacaactga agaattagag caaagaaaca tcctaaaaca aaagaatgaa gaagaggaac 1680
aagaagcaaa aatggaactt aaacgcagac tcagcagaaa gctcagcctg agacccacag 1740
tggcagagct acaagctcga aggatcctgc gatttaacga gtatgtagaa gtcacggatt 1800
ctcctgacta tgaccgccga gcagacaagc cctgggccag gttaacacct gcagacaagg 1860
cagcaataag gaaagaacta aatgaattta aaagcacaga aatggaagtt catgaagaga 1920
gtcgacagtt tacaaggttt catcgtccat aacgaagagt gagactattt ggaaacagag 1980
actgatcatc tttgggggaa gccctgcttc ctgaaaacct gatattgcac tgggatttga 2040

atgtgtgtgt ttccgtttaa cttgtggtga aggaagtgtg tgactcagct tggctgggaa 2100

gtgctcctca cctgtgtggc ccagatggct ccaacaagca aaaggaagga tgcagtgact 2160

cttgctgccc agaatgcaca gcgatggtaa gagacaccgt ggatattctt catcagaagc 2220

tttaatcaaa gaagatgagc agaagacaat cactggctcc ctgttaccag aaagccaaat 2280

tttataatgc gggtgaaaga aaaatataga ttaaaattgc tgggcactga aaaggggtgg 2340

gagttggaaa tcagaaagaa agagaaatat ttcattatgt taatgaagaa aagagaaaat 2400

tgaagcataa atgtattttt gaagtagtta ttcggtagct gagaacttat taagggattt 2460

aggaatttaa cctcctgatg tggattcctc aaagacttga tcggagaata ctcttgtttc 2520

accattatgt tggtacagta tagtaccatt attttatgtt gtgccagtga atccagttgc 2580

cagaaataag tctgaatgtt ttcatgcatc tttttcttaa atgcaagagc ttctactgac 2640

tcttaacaag catgcttacc caaattttgt tgcatgcttt taagtagcat tagctatgca 2700

cacttgacat ttttaaatat ccctttccta gtgtaccagc cttcacagga taagaggact 2760

gggaataaag tcagatgtaa tgtgacccta gaaaactgta gcaagcttca gaaataaatc 2820

ttttgatctt tccctatctt gattagatcc aaagtcaaag caaccatact tcacctagag 2880

aagacagtat tggcaatcat gacacctgta ataaaaactt gaatccaaag tcaaaactta 2940

agcaagatac aaatgtgctg cctgcagtta gtcctgcatg ggaataagga ctagttattt 3000

ttttagtgct gcattttttt aaagttggat tgctgctatt taaaaaaaaa aaaaggacac 3060

agatcaaaaa aacacccaaa ggcttaacag aacatggaaa gttcccacta agttgtgtgt 3120

gaggaaatcc tcatcttgta ttttaagaat ctgcagatgg catccataga tacagtacag 3180

tatttactgt caatgcaaaa gaatcagtga ggttaaagta gtggaagttt tccctatgag 3240

cctacagtct gtattgttta cattaagaaa ctctctatta ttgtgttgct attttccatg 3300

gagtcacagg cacactatga cttctgctcc caaatacata tgaccatatg taaataccac 3360

cttgcatttc attgttcttc acaagtgctt tgtgcccctt taattaaaaa cacatgaaat 3420

gaataacaaa acaagaccca aatagagttt attttattct cagagtttgg actacaattt 3480

tctaagatgt ttctggttca agactgtcat tttctatttc aaccgaaaag taagcattta 3540

acccggtgaa taaatgtaga tcctgccatt cattggtatt ttaaagaacc acttgaaatc 3600

ggatcatttt atttaaaaat aaaaaatgtt aatgccagtt ggcctactat aaaaagccag 3660

gaccatgtta gaattaggaa aagtaaccag cattactgca cctcttgtta gactctgtgc 3720

attaataaaa cacacctaga gtctgttgtc atttcactaa taggataaga caaatttttt 3780

gctttgaaaa aatttttctg catgcccagg tgtctgtctc tggctgaggt tttgtctatt 3840

ttacagtgtt tcaatccagc cataaaaatt aacttgtgat tttttttttc ccaaagtcat 3900

gcttttcctt aattatattt ttattttatt attttagtgt cttgagaaaa ataccaagag 3960
atataatgtt tcttttaatt gtctatgctt aatcatcttt aaacactttt taaatttcta 4020
accacaagac ctctctataa tggtaaatgt aagacatcac cattttatca ctcaaagtat 4080
gttattgaaa gtttctattt ggttgataaa aggaacaatt ttttcccact tttgatgcct 4140
gtgatgcaat tttttattgc ctacaatgag atacacttag tacaaaaaat gaaaatctgg 4200
tatttcaaaa ttgcatttct tgtataatag gtcagattta ttaactactc atactttttc 4260
tttacactaa tcgatacatt tagaaaaaat tttctaagtt agagacaagt ttaaaagt 4318

<210> 12
<211> 3015
<212> DNA
<213> Human

<400> 12

gggagcaaat tcattccctt gctgggttgc tgggctctgg cactgcgctg gccctcgttg 60
gaggtctgca gaagtgcaca gaaagtgtgg gcaatggact ggccatttcc acagttttgg 120
aaacttttaa aaatccatat tcttgctggg attacagatg tgagccatcg tgcccagccc 180
acccactttg ttaatcatga tcagagcagg ggtgccattc taccaccttg gcacacctgc 240
ctagaggagt gagtggcgct gggagcagac tgaggaacat cttagctgag cagtgaatcc 300
agcactgttc taaggaagct cagatggtct gtgactcatc cctgtcctct ggactgaatc 360
tgaggccttg gaactcaccc agggtcttag tggaggcaca gctctgagct atgggtgaag 420
agcatctgaa cccatctaca cattgaccaa acacaggcag gttccagtct gcgggggaga 480
tggagaaggt gccaccatgc tgctgcacaa atcccaggat agctggggaa tgagaatcca 540
gcactttggg cctaggacct ggaaggagag gtcttttggg agaggtatgg ggacatctga 600
aggaggactt cttggaaggg gaggggctgg agcagaggca agagtgattt tgtcagatgg 660
agagaaggtt ctccacatgg gcggggctgg cagaggcaga tgcccagaca ggtcacaact 720
ctctaggtgc ccaattacct gccctcggtg agctcagcca tcggcgggga ggtgccccag 780
cgctacgtgt ggcgtttctg catcggcctg cactcggcgc ctcgcttctt ggtggccttc 840
gcctactgga accactacct cagctgcacc tccccgtgtt cctgctatcg cccgctctgc 900
cgcctcaact tcggcctcaa tgtcgtggag aacctcgcgt tgctagtgct cacttatgtc 960
tcctcctccg aggacttcag tgggtgcctg gatgagggag gagtggggaa gtgttccctg 1020
aggggacggg cctgccccta ccacattcgg accttcctac ttcgtggtgt gggcactccc 1080
tgcaatgtgg ctcccaatcc tctttccctc cagccatcca cgaaaatgct ttcattgtgt 1140
tcattgcctc atccctcggg cacatgctcc tcacctgcat tctctggcgg ttgaccaaga 1200

agcacacagt aagtcaggag gtacggtcta tccctagcgg gggctccaag gcagcccaga 1260
agaaaatcaa ggacatctgt cctcaggatt cgggtaatgg tgaggtggga actgagttct 1320
aatgggaacc ctggcagagg ggtgctgggg ttggggctgg ggcttgggaa caaactgagg 1380
gtggttggtc agaatctagg actatagcac tgtgtttggg tagtgtggga gactggagaa 1440
tagaagagat ggaggctgca aatggggata gaatgaggag tcgcatctag gcggcagtga 1500
gttaggaaca gtgtcagagg actggtctgt cataattcca gcgccccacc catgtacatg 1560
ggtttctttt cccccacagt atttggaggt ggggttggtg ggtgactcca tgtaactttc 1620
atactccatc cccctgaagt ggaagcggca ggaataccac tgctcctcgg cccatcactc 1680
ccccagaggc agggacgtga acacatagtc gtcattcttg ctgcccctgc ctgtgctccc 1740
ttccatgacc gctagtggga gccaagggag ataaggccca gcccttagga gttagggctg 1800
agaggggagc ccacgctctc atacccagca agctgcagag tgatcagaca gcccattccc 1860
taggatcgca agtcctacag ctggaaacag cggctcttca tcatcaactt catctccttc 1920
ttctcggcgc tggctgtcta ctttcggcac aacatgtatt gtgaggctgg agtgtacacc 1980
atctttgcca tcctggagta cactgttgtc ttaaccaaca tggcgttcca catgacggcc 2040
tggtgggact tcgggaacaa ggagctgctc ataacctctc agcctgagga aaagcgattc 2100
tgaacccttc agtcctgctt gggaggacgc agcccactgc ccagaaacaa gaaacacgat 2160
accattctgg ccttccccac cccacatcct ctcttggcct tactgaagat gggggaaggg 2220
taagaaggaa gggtgtaggc caaggctcac cccagtgctg ctggcttctc ctctccaccc 2280
ctcatatggg cgtggggtcc tcaaacatca cctttacctg agaggcccca agaagctgag 2340
ctggcagaga gctctaccat ttggtgctaa aaaaaaaaaa acgtcctgag gttcatgacc 2400
accatccagt ttctggcctt tacacagtca cctttcactg aggtcaggag cccctgagca 2460
gtggctgctc cctgacaacc acagccattt ctctgcgcgg gggtcattca taggactaat 2520
gtatttcatg atctactgtg cacatccagg cctgtggcca cagtcccctg ctaaagttgc 2580
tcaggtgttc tagtcctgac ttcacctttt tgatttggtg tgtgccctag ggtatgtacc 2640
cttccccatc tgagcctcgg tgtgtccatg tgtctggtgg gggatgggtg gactgtatga 2700
tttccaagga ctctaccagt cagtggttct gatgtcatcg ggtggaggtg gtgttctata 2760
cctaaaggat gacctgctcc agaaacagca ccagcacagc atgtattttc ttctcttctg 2820
aaagttctgg cttgtagacc cctcccctcc tttgcaaagg tatgggatag aggggtcaga 2880
tgcagatctc tactgtaaaa tgggctccct ggtatctcct gtcttcccta ctgctccaaa 2940
ccctaaattt tggttgtaca ttttatttga aaggaaaata aatttttttt ttgggccaaa 3000

aaaaaaaaaa aaaaa 3015

<210> 13
<211> 558
<212> DNA
<213> Human

<220>
<221> misc_feature
<222> (252)..(252)
<223> n=any nucleotide

<400> 13

tttttataa ttaaataaat aactttattt atgatacttc agattttata aatgccaact 60

acatagcaaa gtcccataaa ttttaaccga gacacaacca ccaatcaagg ccctttgaaa 120

aatcaaagat aataaagtgt caagggagaa aagttttgtt ctcccaacag agaatttaat 180

caatgaactt acttctctga tcatttaagg ttcacaatgg gaaaacttct tttcttttaa 240

gggtaccctg anatcaatta acagtcaatc aacccacatt tagaaaaata aaccccatag 300

ttaagaaaaa tatatcaaat gcccttcaca cccataatgt aacaaaactt tgacaccaga 360

atgaaaaact tctcagtctc tgtaccaaac caggtgttag tccacccatg agcaagttcg 420

cctgaataac agcatctata caccatgatg tagctgagaa ctacagctga tatcctttta 480

ttgcctgtgg ctcggactga agccaatact ataaatacca cagtgaattc caatggtgaa 540

gagcagcagt gtagatgt 558

<210> 14
<211> 5481
<212> DNA
<213> Human

<400> 14

```
ccgctggtcc gagctgtctg gcctcagttt ccctccgact tttctccgct ctgccagccc      60
tcactgctgc ccgtcattgt tctcgcagtt agatgggggt gctttgtgac ggctgccaag     120
ttggggtgtg ttctctttat tccgtttttc aaacagaaca aggcctccaa ggctgacccc     180
agacaaccca ccccctcgga ccctaattca ccttattgca ctgatttttt ttatcaagtc     240
gtattttatt gtacaggagc cacgccctga tttcttaaag gcgccttgca ctctggccat     300
gtgttatctc tgcagccggt gtgtgggagg cctcttgtga gccagttgtt ttcccgcctc     360
caccaccccc ctcgaagatt tagggatgcc agggggggaag aaggtggctg gggtggcag      420
cagcggtgcc actccaacgt ccgctgcggc caccgccccc tctggggtca ggcgtttgga     480
gaccagcgaa ggaacctcag cccagagaga tgaggagcca gaagaggaag gggaagagga     540
cctgcgagac ggaggcgtcc ccttctttgt caaccggggt gggctacctg tggatgaggc     600
```

```
cacctgggaa aggatgtgga aacacgtggc caagatccac cccgatggag agaaggtggc    660
gcaacggatc cgtggggcca cagacctgcc caagatcccc ataccgagtg tgcctacgtt    720
ccagccgtct acacctgtcc ctgagcgcct ggaagctgtg cagcgctata tcagagagct    780
gcagtacaat cacacaggga cacagttctt tgaaattaag aagagcagac ctctgacagg    840
gctgatggac ctggccaagg aaatgaccaa agaggccctg ccaatcaaat gcctggaagc    900
cgtgatcctg ggaatttacc tcaccaacag catgcccacc ctggagcgct tccccatcag    960
cttcaagacc tacttctcag ggaactactt ccgccacatc gtgctggggg tgaacttcgc   1020
gggccgctac ggtgcgctgg gcatgagtcg gcgcgaggac ctgatgtaca agccgcccgc   1080
cttccgcacg ctcagcgagc tcgtgctgga cttcgaggcc gcctacggcc gctgctggca   1140
cgtgctcaag aaggtgaagc tgggccagag cgtgtcacac gacccgcaca gcgtggagca   1200
gatcgagtgg aagcactcgg tgctggacgt ggagcgcctg ggccgcgatg acttccgcaa   1260
ggagctggag cgccacgccc gcgacatgcg gctcaagatt ggcaaaggga cgggccctcc   1320
ctctcccacc aaggaccgga agaaggatgt ttcttccccg cagcgggccc agtccagccc   1380
ccaccgcagg aacagccgca gtgaaagacg gccctcgggt gacaagaaga cttccgagcc   1440
caaagccatg ccagacctta acgggtacca gatccgggtc tgaggcggat gccagcaccc   1500
caggccccac ccactcttgg gggccaggat ccacctgctg gaaccagcct tatgcatggg   1560
gaaggcgggg ctggtgacaa ggcagggcaa gaggctgcag gaagagtgtg ttccagctca   1620
gcccccccaag ctgctctcgc tcccactgag ccaagccccc taactttggg cctagaggcc   1680
gttagtattt tatttggagt ttttaactct acaactgaag tttaaggtat ttggggaaaa   1740
cttagtccaa atggatctgc tgatggtggg aaggccagtg cttaacaaat ccatgtgtca   1800
tggggccagg tgagggaaac tgctggttct gctggtgcct ctgcccctgg cttctctctg   1860
ggagttgggt gcatcttatc agtgggaaat ctcccagcct taccaggcct gtgatggggg   1920
gtgggggtgg ggtggagatg tttctccagt tctgcctgcc ctggcagaat cttgacccag   1980
ggaaagggaa gcagggtagg agtccttctg agaaaggtct gtgtagccca ttaaccagga   2040
gcttggcaca ggccacatct gccccaagag catgagctcg tggctcagga gagccttcag   2100
gcccttgagg cccccatggg cagtgctgtg tgggcagagg aggggtgata tgagagcgag   2160
cccagggaag gacctctggg caaaaagttc ccaggcccta actgcgtcta cttgctcagt   2220
cccagctctg cctgttgctc tagcccacag gctccctgcg gagggtctgg gcttggcgga   2280
ggacccagaa tggcactgag gccagcatgg ctgtgggagt tgagcaaacc ctgggtgcca   2340
gtccaggagc tgtggctgga catggggtga tggggcggga tgcttgggcc tgggtctctc   2400
```

cgccagcagt gccaggagcc cttgctgggg aaatcaagac cagactagga tgcttctgcc 2460

ccaggcctgc cttccattct ttaacagccc atcttggctt ggggttgcaa tgatggctgg 2520

gccagtcact tgtggcaggg catcagggcc ctggcaggga agaacctagg cacctggggt 2580

tgtccccagc ctgcccgtca gcatgagata cccagtggga aagtgagagg atgcggagag 2640

gttggcagag ccaggggtag gttctggagg ctcaagcaac aaggaggtgc aggtaaaggg 2700

tgcagtgcag ccactgaggg acagctggga actgggggat gcaagtgaga aagggatgtg 2760

ggggagagtt caggatcagg ctgcttgagg agtaatgggt tacctacagc agagacgaga 2820

tggctgtttg tcaggaggcg gtagaaaggt agaaaggatt cagattgtgg aagggtaaat 2880

ggatgagatg accattaagg ttttgtttta ctgagaggct gtaagtctgc cagggacagc 2940

tgtcagtaag gctgcagaag ggctgggggg ctacacaagg aagagcagaa ctagggttgt 3000

aagctcaaat gacgagcaaa cggtgagaag gaggcctgaa gggctgtgtg gggactatgg 3060

ctacctgaag agggcacacc ctgttaaagg ggccacagct gctcagctgt tcttacagtg 3120

ccggccctgt gttgccagat tgtctgcttt gtcagaggcc agaattctga ctttttatgt 3180

gaaataggat ttttaaatgc tggtgatcac ttaaaaaaaa cttaaaaacc caatactggc 3240

aaaagaggat gccagtttgc aatccctgaa gtagagagag ctcgtgctgg ggagaagtcc 3300

accaagatgc tttgaggcgg gctgtagcaa agtcctgttt ctcagagctg ggctgggctg 3360

gggtaggatc cttgcagctg aggaggagga aaagccactg agtctcctcc cagagcggga 3420

caaaccagag gccctttgca gttgctgggt caccagcggg ggtggcgcat ggagtacaga 3480

cagtgtagct cttggcctgc cagggagacg gatggtgcct ttgaagcaaa gaggaaggga 3540

aggcagaacc agggatgcct ttgctgatga gagtgcctgt cagggaaggc gccacaggct 3600

ggcagctctt caaaccagca gcgcttgacc cagagccaga tccagcatgg cctggccaga 3660

ggcaccctgg gaggccagct ggtcagtccc ttgcctcccc aagttccccc tggggtcaat 3720

gagccctggg aggatgccta acctaactcc agccagacta ataggggcat ggtgaccctt 3780

gactcaccat cccatcccag ctttcaggga gtgggggtag tgtggtctcc atgttcctac 3840

tatgcctaag aagagatggc tcaccttggg aggtgccagg ctgaaactag gtcctttccg 3900

ggtctggatg ctgccgctca ggagcagggg cgtggcctca gctgcctctg ggagcttccc 3960

gggaatgaca gggtttgagg ggagtagata tgagagggag ccgctcctgg ttctggagtc 4020

ttaggaggtt ccaacttgca ggatcctttc ccagagccct ccatggagaa aacagcaaaa 4080

tgaagccctt acctgcttgc tgtctgcaag ggagggagcc gagccccagc tgataatccc 4140

ccagcactca cccttcctga gctgagactt cggggctgtg gagaccagca caggacatag 4200

tggtgctttt taaatttatt tttaactgtt tctcatatgt agcaacccct cctcccctcc 4260

tgggcatgtt tacacaggct ctgctctggg ggctggcctg gctgtgaggt ttctggggag 4320
gcagagaggc agggactttg gggccttagt caccatccat ggtatcacct catctcactt 4380
cctgtgaggg acagggcctg gctgatgtga tcccagctcc ccccagttca ggactgtctt 4440
tcagctcctt tgcccctgga ggtgggggct gctggctgag gaggggtcaa ggtgagttca 4500
agaaagctac ctgtggaaaa tggaccaggt tggggggtg attgcaaagt ctccccaaag 4560
cctggctcct catgctcagt gccaggggca gaacactggg gagccaggta tagagagcct 4620
tcctgtcata actgccagtc ctcttcctcc aaggcctctg catattctca tgttcccctc 4680
acccatcatg ccagccaccc ctatccctct tctagcaggg ccaagatggg gacagcagca 4740
gccctctggc cttgggatgt gatgataaag caagcctagg gccagggttt ggggagcaga 4800
gagagccaag aagttgacca cgtgtgattt ccagcccttc ccactgggac ttgacttccc 4860
aggtcaagga gtccgtctca ttctggctgg tcgagtgacc agaggcctgt gtgaatgtgt 4920
gcacctgctt ttcctgcctg gaatgttttc tggctcagct gcagcaacat ctgtgagccc 4980
agtgtctgcc ctgtgtccct gggctcgctc caagtgcagg aacatacatg cagggcccaa 5040
catgatgatg gtgtgaaggg caggaaacag tcctctgaag gagtggggag gtgggcagtc 5100
tgcccccgcc aggtaccatc gcctcctgcc agcttcctta gaccaggcag ggctgccatg 5160
gtgctagctg caagtccatc agtattgacc gtctcgctcc atcttggtcc tccggagtcc 5220
caagtttcct tttcatcaaa tctgacaaga gagaagaaac atgggtgtgc ttggcccaca 5280
gggcctggtg gtgatggacc tccccgctcc ctcaagctct ggatggctgc agtgttgtac 5340
tagactttgt tcaggctgtt ctcatctcag tattgcccct tcctttcact ttcacacttc 5400
atctcattcc tgttgtcact ttccccgaaa cgaataaagt ctccccagct cctgctgtgt 5460
aggctgggca gaaaccacaa c 5481

<210> 15
<211> 743
<212> DNA
<213> Human

<220>
<221> misc_feature
<222> (531)..(531)
<223> n=any nucleotide

<220>
<221> misc_feature
<222> (655)..(655)
<223> n=any nucleotide

<220>
<221> misc_feature

<222> (657)..(657)
<223> n=any nucleotide

<220>
<221> misc_feature
<222> (658)..(658)
<223> n=any nucleotide

<220>
<221> misc_feature
<222> (665)..(665)
<223> n=any nucleotide

<220>
<221> misc_feature
<222> (667)..(667)
<223> n=any nucleotide

<220>
<221> misc_feature
<222> (672)..(672)
<223> n=any nucleotide

<220>
<221> misc_feature
<222> (673)..(673)
<223> n=any nucleotide

<220>
<221> misc_feature
<222> (702)..(702)
<223> n=any nucleotide

<220>
<221> misc_feature
<222> (736)..(736)
<223> n=any nucleotide

<400> 15

```
tttggcatgc cactaactat tttttatttg caactacatg atggcacaca attctcttaa      60
acaacgacat aaaatagatt tccttgtata taaataactt acatacgctc cataaagtaa     120
attctcaaag gtgctagaac aaatcgtcca cttctacagt gttctcgtat ccaacagagt     180
tgatgcacaa tatataaata ctcaagtcca atattaaaaa cttaggcact tgactaactt     240
taataaaatt tctcaaacta tatcaatatc taaagggcat atatttttta agaaagatta     300
ttctcaataa cttctataaa aataagtttg atggtttggc ccatctaact tcactactat     360
tagtaagaac ttttaacttt taatgtgtag taaggtttat tctacctttt tctcaacatg     420
acaccaacac aatcaaaaac gaagttagtg aggtgctaac atgtgaggat taatccagtg     480
attccggtca caatgcattc caggaggagg tacccatgtc actggaattg ngcgatatgg     540
tttatttttt cttccctgat ttggataacc aaatggaaca ggaggaggat agtgattctg     600
atggccattc cctcgataca ttcctggctt ttttctgggc aagggtgccc acatngnnag     660

agtgnanata tnnagtctga aatctggtac acaggacttg cngctgcagt caccgaactg     720
ggttcacttt catttncttt act                                             743
```

<210> 16
<211> 4701
<212> DNA
<213> Human

<400> 16

gcggccgcca cacccagcac cacaggcacc aagtccaaca cgcccacatc ctccgtgccc 60
tcggccgccg tcacacccct caacgagagc ctgcagcccc tgggggacta tggcgtgggc 120
tccaagaaca gcaagcgtgc ccgggagaag cgcgacagcc gcaacatgca agtacaggtc 180
acccaggaga tgcgcaacgt cagtataggc atgggcagca gtgacgagtg gtctgatgtt 240
caagacatta ttgactccac gccagagctg gacatgtgtc cagagacccg cctggaccgc 300
acaggaagca gcccaaccca gggcatcgtg aacaaagctt tcggcatcaa caccgactcc 360
ctgtaccatg agctgtcgac ggcagggtct gaggtcatcg gggatgtgga cgaaggggcc 420
gacctcctag gggagttctc aggaatgggc aaagaagtgg ggaatctgct actggaaaac 480
tcacagcttc tggaaaccaa aaacgccttg aatgtggtga agaatgacct gattgccaag 540
gtcgaccagc tgtccgggga gcaggaggtg ctgaggggcg agttggaggc tgctaagcag 600
gccaaagtca agctggaaaa ccgtatcaag gagctggaag aggaactgaa aagagtgaag 660
tccgaggcca tcatcgcccg ccgtgaaccc aaagaagagg cggaggatgt aagcagctat 720
ctctgtacag aatcggacaa aatccccatg gcccagcgcc gccgcttcac gcgggtggag 780
atggcccgtg tgctcatgga gcggaaccag tacaaggagc ggctgatgga gctgcaggag 840
gctgtgcggt ggactgagat gatcagagcg tcccgagagc acccatccgt ccaggagaag 900
aagaagtcga ccatctggca gttcttcagc cgcctcttca gctcttcctc cagccccccct 960
ccggccaagc gcccctatcc ctcggtgaac atccactaca agtcacccac cactgccggc 1020
ttcagccagc gccgcaacca tgccatgtgc ccgatctcgg caggcagccg gcccctggaa 1080
ttcttccctg acgacgactg cacgtcctcc gcccgtcgag agcagaagcg cgagcagtac 1140
cgccaggtgc gtgagcacgt gcgtaacgac gacggccgtc tgcaggcctg cggctggagc 1200
ctgcccgcca agtacaagca gctgagtccc aacgggggcc aggaggacac gcggatgaag 1260
aacgtgccgg tgccggtgta ctgccgccct ctggtggaga aggaccccac catgaagctg 1320
tggtgtgccg cgggcgtcaa cctgagcggg tggaggccca atgaggacga cgctgggaat 1380
ggagtcaagc cagcgccagg ccgcgatccc ctgacctgcg accgcgaagg agacggcgag 1440
cccaagagcg cccacgcgtc tcccgagaag aagaaggcca aggagctccc tgaaatggac 1500

gccacctcca gccgggtgtg gatcctgacc agcaccctga ccaccagcaa ggtggtgatc 1560
atcgacgcca accagccggg cacggtggtg gaccagttca ccgtctgcaa cgcgcacgtg 1620
ctgtgcatct ccagcatccc cgcggccagc gacagcgact accctcccgg ggagatgttc 1680
ctggacagcg acgtgaaccc agaggacccg ggcgcagatg gcgtgctggc cggtatcacc 1740
ctggtgggct gtgccacccg ctgcaacgtg ccgcggagca actgctcctc ccgaggggac 1800
accccagtgc tagacaaggg gcagggggag gtggccacca tcgccaacgg gaaggtcaac 1860
ccgtcccagt ccacagagga ggccacagag gccacggagg tgccagaccc tgggcccagc 1920
gagccagaga cagccacatt gcggcccggg cctctcacag agcacgtctt cactgaccca 1980
gccccgaccc cgtcctctgg cccccagcct ggcagcgaga acgggccaga gcctgacagc 2040
agcagcacac ggccagagcc agagcccagc ggggacccca cgggagcagg cagcagtgct 2100
gcacccacca tgtggctggg agcccagaac ggctggctct atgtgcactc ggctgtggcc 2160
aactggaaga agtgcctgca ctccatcaag ctgaaggatt ctgtgctgag cctggtgcat 2220
gtcaaaggcc gtgtgctggt ggctctggcg gacgggaccc tggccatctt ccaccgtggt 2280
gaagatggcc agtgggatct gagcaactat cacctaatgg acctgggcca cccgcaccac 2340
tccatccgct gcatggctgt tgtgtacgac cgcgtgtggt gtggctacaa gaacaaggtg 2400
cacgtcatcc agcccaagac catgcagata gagaagtcat ttgacgccca cccgcggcgg 2460
gagagccagg tgcggcagct ggcgtggatc ggcgatggcg tatgggtgtc catccgcctg 2520
gactccaccc tgaggctcta ccatgcacac acgcaccagc atctacagga cgtggacatt 2580
gagccctacg tcagcaagat gctaggcact ggcaagctgg gtttctcctt cgtacgcatc 2640
acggccctgc ttgtcgcggg cagccggctc tgggtgggca ccggcaacgg agtggtcatc 2700
tccatccccc tgacagagac tgtggtcctg caccgaggcc agctcctggg gctccgagcc 2760
aataagacat cccccacctc tggggagggc gcccgtcccg gggcatcat ccacgtgtat 2820
ggcgatgaca gcagtgacag ggcggccagc agcttcatcc cctactgctc catggcccag 2880
gcccagctat gcttccatgg gcaccgcgat gccgtgaagt tctttgtctc ggtgccaggg 2940
aacgtgctgg ccaccctgaa tggcagtgtg ctggacagcc cagccgaggg ccctgggcca 3000
gctgcccctg cctcggaggt cgagggccag aagctgcgga acgtgctggt gctgagcggc 3060
ggggagggct acatcgactt ccgcattgga gacggagagg acgacgagac ggaggagggc 3120
gcaggggaca tgagccaggt gaagcccgtg ctgtccaagg cagagcgcag tcacatcatc 3180
gtgtggcagg tgtcctacac ccccgagtga agctgctgcc ctgcctggcc cgacctgtac 3240
ataggacccc cgaccacctg acccccgccc ggcccgcggg gtagccagcc aggcgccgcc 3300

```
gcccctcttc taacctctca acctgcagct ttcacctgag tctggcccct ccagcgggca   3360
gggagtgcgg ggatgcggat cagctgggag gaggagggga ggggtgcttc cacccgaggg   3420
gaagatgctc tcgggacagt ttcccgggca gctcctggcc agcttccagc ccagagtcct   3480
caagtccagg gcaccttggg cccagcgcag gcagaatccg aggtggtcct ggctctaccc   3540
tgggcctcct actccccagc acccctggag gaggcagggg ctccccgccg ccgaggctgc   3600
ctgccctggg cccacctctg catgctgctc atggggccac cctgcctcct gggccctcac   3660
tctgcctagg ggagctgggc caggcactag cctttgccca gggaggtggg cctcaggctg   3720
cccaggtgcc tgcaccccag ccggccttct ctggggcctc cccgtcgtca agcctctatc   3780
ctgtctgtcc ccaccccagc tgtcccctgc ccagggagct ggcataaaag cacgaggccc   3840
ggctccctgg ggcagctgct tgagaacaga gactgctacc ccatcctgcc catgcaggca   3900
ggctcttgcc agccccgttc tgacccgtgt ccccccaggc tctgcctggg cagaagactc   3960
accttggagg agtgggccct ggagtcctgt ccctcccaga agcccccagg gtgggatttc   4020
tcaggctgcc agggcaggcc caggcctcag gaagaagggg aggcccctgg cctctccggg   4080
atcagtccta ggacacaggc tcagcctcag gttgatgggg gatgatgtgc tcccggggcc   4140
tgcctcctgc acggggctcc acggagccca gctcccagac acgctactaa gtgcctaggg   4200
ttgcccgctg tggcctgctc ccagggagca acagagaggc caccaagcag aggcccgtgg   4260
ggctgaggat ggagccgccc ccagccgact ccaagcccgc agagggcaga cgccaccctg   4320
gactgctctc cctgcccagc tgggcctctc tggcctattc ctaccttcca ggcccactgc   4380
actcctgtct gggaggccct tatgagggca gcccagcccc cgcacccacc cccaaccaga   4440
gaagcacaga tcttggggag ctgccccaca agccccgctg gccaccgagg gctgcagccg   4500
ctgcgctgcc ggcttctccc caccaccctg ccacctccac tgtgatgtat gtccgctccc   4560
tcgtctgttc ccccaggatc tcgaagtgac tccgggctga gcagtggggc ggctggggga   4620
ggggtgacga ttctcctcag gctttggccc tgcaagcaaa cccacatatc tgctctgtat   4680
gtaataaatg tcttaacgtc g                                              4701
```

<210> 17
<211> 1445
<212> DNA
<213> Human

<400> 17

```
ctgccccaca cacactaacc caaccatctt ggggtggact ccctgccagc ccaactgttg      60
tattttcagt tcttccagtg tgaatcagtt aatattctcg ggaacgaggg agaggttgat     120
cctatgagga aatcaaccac agtgaaaagg cttgggccgc ttttgttttc gcctcctttt     180
gttgaacaaa tttgatttcc ggagtcagtc attttactgt caagacattt cttcggcatt     240
ctgcaacagt ttccaacatg gctagatcca tcagaaactg aagccgtgga gaacgctctc     300
ggggcctttg ccacttcttg gagtagaagc cgacagagag ctgtttggaa acttctcctt     360
cacacaccag ttgaagacta ggctttggag gttttcaaag cagacggtgc ttggatgggc     420
agggagaagt aacattctgc aaatcgccgt cagaggtcct gaggacacag acctacctgg     480
cttgcattcc ccttgctgaa tggcgtgtgc tgcagctgcc cactgagggc tcttttccct     540
gggattctgg acttcagagt aggacagcag gctgggaaga tgctgagttc catcaagtgc     600
gtgttggtgg gcgactctgc tgtggggaaa acctctctgt tggtgcgctt cacctccgag     660
accttcccgg aggcctacaa gcccacagtg tacgagaaca caggggtgga cgtcttcatg     720
gatggcatcc agatcagcct gggcctctgg gacacagccg gcaatgacgc cttcagaagc     780
atccggcccc tgtcctacca gcaggcagac gtggtgctga tgtgctactc tgtggccaac     840
cataactcat tcctgaactt gaagaacaag tggattggtg aaattaggag caacttgccc     900
tgtacccctg tgctggtggt ggccacccag actgaccagc gggagatggg gccccacagg     960
gcctcctgcg tcaatgccat ggaagggaag aaactggccc aggatgtcag agccaagggc    1020
tacctggagt gctcagccct tagcaatcgg ggagtacagc aggtgtttga gtgcgccgtc    1080
cgaactgccg tcaaccaggc caggagacga aacagaagga ggctcttctc catcaatgag    1140
tgcaagatct tctaaacccc aagagacttc acacaacact tatgtatgca ccccaaagac    1200
taatggggag agggagggcc gggaagccag gaaagcttgg tgttttctct gggtacaccc    1260
caagcagcgt ctccgttttg gatacagtta ttgatgaggc ttggccactg gatgttttca    1320
ctaactacac tctacaagtg aactccttgc ccaggccagt tagaaaatcc cttggggaac    1380
tgtgatgaat attccatctt tgattaaaaa agtgaaatag tctccataaa aaaaaaaaaa    1440
aaaaa                                                                1445
```

<210> 18
<211> 2429
<212> DNA
<213> Human

<400> 18

```
ggcggtgcac gccggctgcc cgctgcccgc gatgcccatg cagggcggcg cgcagagtcc    60
cgaggaggag ctgagggccg cggtgctgca gctgcgcgag accgtcgtgc agcagaagga   120
gacgctggcc agcgcgaggg ccatccgcga gctcacgggc aagctagcgc gctgcgaggg   180
gctggcgggc ggcaaggcgc gcggcgcggg ggccacgggc aaggacacta tgggcgacct   240
gccgcgggac cccggccacg tcgtggagca gctcagccgc tcgctgcaga ccctcaagga   300
```

ccgcctggag agcctcgagc accagctcag agcaaacgtg tccaatgctg ggctgcccgg 360
cgacttccgc gaggtgctcc agcagcggct gggggagctg gagaggcagc ttctgcgcaa 420
ggtggcagag ctggaggacg agaagtccct gctgcacaat gagacctcgg ctcaccggca 480
gaagaccgag agcaccctga acgcgctgct gcagagggtc accgagctgg agcgaggcaa 540
tagcgccttt aagtcaccag atgcgttcaa ggtgtccctc ccactccgca caaactacct 600
atacggcaag atcaagaaga cgctgcctga gctgtacgcc ttcaccatct gcctgtggct 660
gcggtccagc gcctcaccag gcattggcac ccccttctcc tatgcggtgc cagggcaggc 720
caacgagatc ttgctgatcg agtggggcaa caaccccatc gagctgctca tcaacgacaa 780
ggttgcgcag ctgcccctgt ttgtcagtga cggcaagtgg caccacatct gtgtcacctg 840
gacgacacgg gatggcatgt gggaggcatt ccaggacgga gagaagctgg gcactgggga 900
gaacctggcc ccctggcacc ccatcaagcc cgggggcgtg ctgatccttg gacaagagca 960
ggacaccgtg gggggtaggt ttgatgccac tcaggcattt gtcggggagc tcagccagtt 1020
caacatatgg gaccgcgtcc ttcgcgcaca agaaattgtc aacatcgcca actgctccac 1080
aaacatgccg ggcaacatca tcccgtgggt ggacaataac gtcgatgtgt tcggaggggc 1140
ctccaagtgg cccgtggaga cgtgtgagga ggctctcctt gacttgtagc cgccttctcc 1200
tctgtccagg aggccgggat caggctgttg ccatggaagt tcagggccat agactgcccc 1260
acttaaactc ttgtcagtct gggctcaggg ttcccagagc tcattcccca ggaatctcta 1320
agaccagggc tggggcagtg tctgtcactg gcttgtttgt tccctaccaa tattctgttg 1380
ctgtttgaag tagtgccagg gtcccctggg aagatgcccc caagacacct gccccaagtg 1440
ggtggatatc tgccttcctg ctgcaagtgg aggcaggtcc agcagcccct cttcagagcc 1500
cctgtaaatg ctatcgcagc ctgagtcctg ccgccttcca gttccttggt gtcccgtgca 1560
cccccttctgt ctgtcccctt tcatgctgtg cagccgtccc gctggagtgg ccatgtccct 1620
tgtgcattga gtgcatcccc gctggtgact aagctcgcag caagcgctac ccccgatctg 1680
caaaagggcc tctccctttg tgttctatac attgtgaatc ttcccgtctg aagaacgccc 1740
agcctgccca gacaaagccc cgccttcccc aaagcagagg ggctgtctgt gtctccagaa 1800
aggggacatc gggggggggag gggggctcag aaaggagaag ggctgtgatc tccggtccct 1860
tcccccatca tccttcctta gactgatgct ttgactgaat catcactagc tatggcatta 1920
aaaggcctct cttctcatct ggtgccaaag gttccgttgc agctttttac aaccatccgg 1980
tgtggtttgg aggatttgtt tttttttttt cccaacagaa aagaacagcc attagaagaa 2040
ggctcccatt ttctgatgtt ccgccccact gtgaagagtg tgctcgtttt aaattcatgt 2100
tgattcttgt aagcactgga ctgtcttcat caagtatttc ccctacagaa ctcctcaaga 2160

aaaacagaga tcatttggct agagattgtc tgagtgactc caagctactc actgtattgg 2220

acgggagtag taatttattt taaagataaa gtgactaagt ggggaaattt ataaagctaa 2280

atattatata ttttattttt catacatgtt tgaagtgcaa atctgtggat attccatttg 2340

taggaccaag tcgacatgcc catcctgaca ttgtatgcta cgagaactct tctgatgatg 2400

gaatttcgat taaagtgcac tgaaagatg 2429

<210> 19
<211> 21
<212> DNA
<213> Primer

<400> 19
ggtcagatgt ttgccaagga a 21

<210> 20
<211> 24
<212> DNA
<213> Primer

<400> 20
tcttcagaaa cacactccca tcac 24

<210> 21
<211> 20
<212> DNA
<213> Probe

<400> 21
tgaaacggaa gaagcttgta 20

<210> 22
<211> 8081
<212> DNA
<213> Human

<400> 22

```
gtctaaaact ctcttttctc ttggctcttc ttattcgagt gatgaggagg aggagttgca      60
taattcacgg cccttccaca gtaccttcca caataccagt gctaatctga ctgagagtat     120
aacagaagag aactataatt tcctgccaca tagcccctcc aagaaagatt ctgaatggaa     180
gagtggaaca aaagtcagtc gtacattcag ctacatcaag aataaaatgt ctagcagcaa     240
gaagagcaaa gaaaaggaaa aagaaaaaga taagattaag gagaaggaga aagattctaa     300
agacaaggag aaagataaga agactgtcaa cgggcacact ttcagttcca ttcctgttgt     360
gggtcccatc agctgtagcc agtgtatgaa gcccttcacc aacaaagatg cctatacttg     420
tgcaaattgc agtgcttttg tccacaaagg ctgccgagaa agtctagcct cctgtgcaaa     480
```

ggtcaaaatg aagcagccca aagggagcct tcaggcacat gacacatcat cactgcccac 540

ggtcattatg agaaacaagc cctcacagcc caaggagcgt cctcggtccg cagtcctcct 600

ggtggatgaa accgctacca ccccaatatt tgccaataga cgatcccagc agagtgtctc 660

gctctccaaa agtgtctcca tacagaacat tactggagtt ggcaatgatg agaacatgtc 720

aaacacctgg aaattcctgt ctcattcaac agactcacta aataaaatca gcaaggtcaa 780

tgagtcaaca gaatcactta ctgatgaggg agtaggtaca gacatgaatg aaggacaact 840

actgggagac tttgagattg agtccaaaca gctggaagca gagtcttgga gtcggataat 900

agacagcaag tttctaaaac agcaaaagaa agatgtggtc aaacggcaag aagtaatata 960

tgagttgatg cagacagagt ttcatcatgt ccgcactctc aagatcatga gtggtgtgta 1020

cagccagggg atgatggcgg atctgctttt tgagcagcag atggtagaaa agctgttccc 1080

ctgtttggat gagctgatca gtatccatag ccaattcttc cagaggattc tggagcggaa 1140

gaaggagtct ctggtggata aaagtgaaaa gaactttctc atcaagagga taggggatgt 1200

gcttgtaaat cagttttcag gtgagaatgc agaacgttta aagaagacat atggcaagtt 1260

ttgtgggcaa cataaccagt ctgtaaacta cttcaaagac ctttatgcca aggataagcg 1320

ttttcaagcc tttgtaaaga agaagatgag cagttcagtt gttagaaggc ttggaattcc 1380

agagtgcata ttgcttgtaa ctcagcggat taccaagtac ccagttttat tccaaagaat 1440

attgcagtgt accaaagaca atgaagtgga gcaggaagat ctagcacagt ccttgagcct 1500

ggtgaaggat gtgattggag ctgtagacag caaagtggca agttatgaaa agaaagtgcg 1560

tctcaatgag atttatacaa agacagatag caagtcaatc atgaggatga agagtggtca 1620

gatgtttgcc aaggaagatt tgaaacggaa gaagcttgta cgtgatggga gtgtgtttct 1680

gaagaatgca gcaggaaggt tgaaagaggt tcaagcagtt cttctcactg acattttagt 1740

tttccttcaa gaaaaagacc agaagtacat ctttgcatca ttggaccaga agtcaacagt 1800

gatctcttta aagaagctga ttgtgagaga agtggcacat gaggagaaag gtttattcct 1860

gatcagcatg gggatgacag atccagagat ggtagaagtc catgccagct ccaaagagga 1920

acgaaacagc tggattcaga tcattcagga cacaatcaac accctgaaca gagatgaaga 1980

tgaaggaatt cctagtgaga atgaggaaga aaagaaaatg ttggacacca gagcccgaga 2040

attaaaagaa caacttcacc agaaggacca aaaaatccta ctcttgttgg aagagaagga 2100

gatgattttc cgggacatgg ctgagtgcag cacccctctc ccagaggatt gctccccaac 2160

acatagccct agagttctct tccgctccaa cacagaagag gctctcaaag gaggaccttt 2220

aatgaaaagt gcaataaatg aggtggagat ccttcagggt ttggtgagtg gaaatctggg 2280

aggcacactt gggccgactg tcagcagccc cattgagcaa gatgtggtcg gtcccgtttc 2340

cctgccccgg agagcagaga cctttggagg atttgacagc catcagatga atgcttcaaa 2400
aggaggcgag aaggaagagg gagatgatgg ccaagatctt aggagaacgg aatcagatag 2460
tggcctaaaa aagggtggaa atgctaacct ggtatttatg cttaaaagaa acagtgagca 2520
ggttgtccag agcgttgttc atctctacga gctcctcagc gctctgcagg gtgtggtgct 2580
gcagcaggac agctacattg aggaccagaa actggtgctg agcgagaggg cgctcactcg 2640
cagcttgtcc cgcccgagct ccctcattga gcaggagaag cagcgcagcc tggagaagca 2700
gcgccaggac ctggccaacc tgcagaagca gcaggcccag tacctcgagg agaagcgcag 2760
gcgcgagcgt gagtgggaag ctcgtgagag ggagctgcgg gagcgggagg ccctcctggc 2820
ccagcgcgag gaggaggtgc agcaggggca gcaggacctg gaaaaggagc gggaggagct 2880
ccagcagaag aagggcacat accagtatga cctggagcga ctgcgtgctg cccagaaaca 2940
gcttgagagg gaacaggagc agctgcgccg ggaggcagag cggctcagcc agcggcagac 3000
agaacgggac ctgtgtcagg tttcccatcc acataccaag ctgatgagga tcccatcgtt 3060
cttccccagt cctgaggagc ccccctcgcc atctgcacct tccatagcca aatcagggtc 3120
attggactca gaactttcag tgtccccaaa aaggaacagc atctctcgga cacacaaaga 3180
taaggggcct tttcacatac tgagttcaac cagccagaca aacaaaggac cagaagggca 3240
gagccaggcc cctgcgtcca cctctgcctc tacccgcctg tttgggttaa caaagccaaa 3300
ggaaaagaag gagaaaaaaa agaagaacaa aaccagccgc tctcagcccg gtgatggtcc 3360
cgcgtcagaa gtatcagcag agggtgaaga gatcttctgc tgaccctctt cctctctgct 3420
gaggcagctg cctcctgatc ctggccagcc cacctctcct gctgtccccg cgtgcacaag 3480
tctcttacac tggacgccca ctgctcctca gcgtccagtc ctcctgggcg gccccaggtc 3540
ctggacaata agcaacagat gatattgagt gtcgggtggg gaaggaggcc cagactctgc 3600
ttcggccatg atttgtgact gcccaggact ctcaggttgg gctggcccta ctcaggatta 3660
cactgaaagt aatggcctcg taagtacagg tgatggtttt ggacacgtca ggaattccta 3720
aaggctgaaa gagtgtatcc aagtaaggtc tgaacctccg aatgcctttt atttggggga 3780
acacaaaacc aaacagcaga tgttttggac ttgatctgtg tacgtacatg ggacctgtc 3840
tgcatataca cacggggaat gccagaagaa ggcccagtct gcaccaggcg tctggtcaac 3900
ttagcacaag ggcagtgcct ggacggaccc ggagcccccg catatcagca gttcacccag 3960
tactcctcag agactggttt ccctctaaac ccatcccggg cacataccac ccgtgttttg 4020
catgtatttc tcatttcatt ttagggatga caaacatttg tgaaaccagt gagagaaggc 4080
ttgatgtgta taaaagacgt gatgtgcacc acctcgatct cggtgtttca ggcactaaag 4140

```
caacaaaaca acccatagta tctcattctg tcatcagatc cagaagaaat atcctggttt   4200
tccagcatgt ttacccacat gttttggcca tggataaagt gaagaggcct actcaccatt   4260
atccctgcag cgtgacacct tttgattgtc actgaccact cagaaggggc cacggcctcc   4320
tggctgtgtt cctgagcccc cgtcgtgcct ctcccagaca gcagctgtct ggcccttgct   4380
gggtgagggc acaccactgc caggggtcag cctcgcaccc aggccaggca gaagctgtgc   4440
tctgaagcta ggacagctgg ctgagaagtg ggttcaggcg aagggtgaag ccatgtgtag   4500
cagttcctgc cagtgcagat ctggagagga gctggcccgg aaggcgtggt tgtgaaagcg   4560
cccttcttat gttaggaggc cttggcaaaa ttggatttct tcaaaaatac atgtaaaggt   4620
ctgttgttga attgtactct gcccctggaa gcagatacag atggctgcct gctgctcggc   4680
tttgcttttg cttttcccac cgtgttttca tctttgttca cttgaggctt tccccagctg   4740
gtgtgtgcag gacagttcat ggtaatgttg ccctctgagg ccccgtacac cagaagggag   4800
gccctggaaa attttgtgct tccaacgtgg ccttcaattc ttgctttttt gcccctcgga   4860
agcatggggc ttttgagcac acttaaaaaa agaaaaatct gtaacttggt gcttattgat   4920
gaattgcaag ctggccttgc agatggagat atttatcttt cagtttattt gaaagaggtc   4980
tggtttaaaa tttgtagcct acatttgttt tatttattgt atttgtgtgt ttgtgtttgt   5040
ttttttttaa gggtgagcca ggtctagccc aacagtctaa actatccagt caataccgag   5100
tgaagtggca gccagcactg ttcactctgt gtcttttgaa gtgccttgaa ggcccagatg   5160
aaattttaaa gggagggggt ccatgtcctt ccctccccca ccccgcctca ttctttaatc   5220
aaaggatgtc ttctcccttg tttgagaatg aagaaactcg ccacctctga cctacctttg   5280
cctttttctg tcatggagaa tactcaccct tcagaaacag accaaaggcc aaaacctgct   5340
gatttttcta ttgaaaatat gtccccttgc aaagacccta aacaaaaagt taagtttctt   5400
tctttcacct atttgtacaa ctccaagtta cagctgaatc tgtcgtgact ttcctgagat   5460
ctacccgggg cttggctgtc tgttctgggc actggctccg agttcccctc ctgggatttg   5520
caggagggca gtactgaacc tgcattcttc tccttgtaaa tgtaggccgg gtgcccctgt   5580
tctccgggtt tggaacaata cgaggttggt gctgatggga tttacttgcg tacgtgctct   5640
tcacaaaaac accgtggatg ctgaagttag agcacgtcgc cacagagctt gacatcaatg   5700
ttagagggtc tcttactccc cgcccagctg tgatgtttca tctgctttgg ttgttttggt   5760
ggtctttttt aaaaatagag atttcacatc tgcccagacc ccactcaaaa cgatttggtc   5820
aggttctggt tggacaagtt taaaatcaaa gtagtgcccg gaattccctc aaaccaccca   5880
acttcatcca ggaatacagt ctgcagtgca gcaacagaac cgcttaccaa gaactgtgct   5940
tacatacctt tgtcatctct cttccccccct tggaagttgt cctcaggggg atttgttcct   6000
```

gtcctgggga tttacctggg atggtggctg cctgtgcttt tgctcatggc cttgacagtg 6060
ctctagttgc tggatctaat ggcctgtctt ggtttctatc acatgagaag gggttgtttt 6120
tttggggtga ctcggactga attccccata ctgtttccac gccgggacac catgttctcc 6180
atcaagctaa agaaatcacg tgcctgaaac tgtgcttaag ttttggggga aagatggagt 6240
tcctatccag agcccccaga tttccagaat cgagtgagct tcctggaagg agactgcgtc 6300
ttctctcaat tccagtcatc tcagtcgttg tcgttaggtg acatgtgcac tttaaatgct 6360
ctcatcggtt ggcttcattt tcaagacaat caaatgtatt gactgtgttt tcttcttaga 6420
aaatggagag ggttaaaaac atgcaaactg ccactttcaa cctttgccag tattccctct 6480
acccccgtga gagctatctg gggggaagaa tccttaccaa ggtttttttg gaaaggtacg 6540
aatcttaact tttttcccct tctgtgtctc agggtaatac tattcagagt cgcccctttg 6600
ctcattttct cccgtatttg ttaccttcct gaggcctcag tattagtcgt gagcacaaag 6660
ttttgagacc tttggcgttg tttcttgatg tgggagggga ggtgttagtg catgcaaggg 6720
ttgaactaga tagaccctgc cttagtagag ggtgggacta taaccttaga ggccagaact 6780
tgatccagaa gttgctgtcc acagaagtgc tttctatttc atcatttttg tttctagggc 6840
tctttttctg tagccaggtc ttcccaagga ttttagtatt tgcattggag ttgaggttta 6900
ctctaatgat ggtggcccag ctgtgcccag aggacagcca ggcaggccct gggagggagt 6960
ttagaaagac agtcctggtg aatgggcttc aagtggtcac aaagagggtg gctgtgaggt 7020
gaccccagac actgcagaac gatgtgcacc ctctgcgttt tggatgtcct tggaatgtgg 7080
gagcctagaa ataaccctgt ggatggaatt ggggcagcgg ctgctggaga tctgtgtgcc 7140
ttgccttcct tcagcaggac cgtctaggtg cgcagccacc tatggatgcg tcccagccag 7200
ccccgtcgct ctcgtccatc ctcagagaca aagaagaggg cagggagttt gggcttggtt 7260
ttgaactttc ctttcaatgt agcaaagcat tcctagttaa ccagagcctt ggaatctact 7320
gcctgctggc caggctttaa aatgaaaagt gttttaatgc tgccataaaa gggaggcggg 7380
ggggaggaag ggaaaataaa ggcatctttc caagtactca tctaatttaa ttgtcaaaag 7440
attgataggc catgaattac ttctccatct cactaagggt taaaggcgtg caacccccca 7500
ctggctgtgt cccctgccac cgaagtgagt gacctgccct acaaccaggt gggaccacct 7560
gtgctgcagt ccggaggggc ttctgcagga agcactcacc ccccacacct tccccggcct 7620
gagcttcccc tacctttcgt caccacctga gggcatgagc acaggccatg ggcgtgcct 7680
ggtgagtctg cctgtggttc aggcttagcc tgtggtctcc tgtgtgctgc tgcccgcatg 7740
ggatgcgcag gggaggcgtg gggatccgca ggagggtggt tgggatacac cggatacctc 7800

tgctctcatt gcttgtttgc aaatgctcta tggacatttg tgtgctaaat cctattaaat 7860
aaaaaagacg ggttaaaacc cagatgctgt atattcattt gtaattatgt ataaagtgaa 7920
gcagttttaa actgtaaaga ttttttcag tgtgttttct cgaattttgc cacaacatac 7980
tggcttcgta ttttatttat ctttctttct agttaccagc ttcagaccct tgtaaagtct 8040
ccctcagccc tttcaaaaaa taataaattt cctgtgaagt t 8081

<210> 23
<211> 13290
<212> DNA
<213> Human

<400> 23

gaagcgcctg tgctctgccg agactgccgt gcccattgct cgcctcggtc gccgccgctt 60
tagccgcctc cgggggagcg gccgcctatt gtctttctcc gcggcgaagg tgaagagttg 120
tcccagctcg gcccgcgggg gagccccggg agccgcacgt gtcctgggtc atgaaactta 180
atccacagca agctccctta tatggtgatt gtgttgttac agtgctgctt gctgaagagg 240
acaaagctga agatgatgta gtgttttact tggtattttt gggttccacc ctccgtcact 300
gtacaagtac tcggaaggtc agttctgata cattggagac cattgctcct ggtcatgatt 360
gttgtgaaac agtgaaggtg cagctctgtg cttccaaaga gggccttccc gtgtttgtgg 420
tggctgaaga agactttcat ttcgtccagg atgaagcgta tgatgcagct caattcctag 480
caaccagtgc tggaaatcag caggctttga actttacccg ttttcttgac cagtcaggac 540
ccccatctgg ggatgtgaat tcccttgata agaagttggt gctggcattc aggcacctga 600
agctgcccac ggagtggaat gtattgggga cagatcagag tttgcatgat gctggcccgc 660
gagagacatt gatgcatttt gctgtgcggc tgggactgct gaggttgacg tggttcctgt 720
tgcagaagcc aggtggccgc ggagctctca gtatccacaa ccaggaaggg gcgacgcctg 780
tgagcttggc cttggagcga ggctatcaca agctgcacca gcttctaacc gaggagaatg 840
ctggagaacc agactcctgg agcagtttat cctatgaaat accgtatgga gactgttctg 900
tgaggcatca tcgagagttg gacatctata cattaacctc tgagtctgat tcacatcatg 960
aacacccatt tcctggagac ggttgcactg gaccaatttt taaacttatg aacatccaac 1020
agcaactaat gaaaacaaac ctcaagcaga tggacagtct tatgccctta atgatgacag 1080
cacaggatcc ttccagtgcc ccagagacag atggccagtt tcttccctgt gcaccggagc 1140
ccacggaccc tcagcgactt tcttcttctg aagagactga gagcactcag tgctgcccag 1200
ggagccctgt tgcacagact gaaagtccct gtgatttgtc aagcatagtt gaggaggaga 1260
atacagaccg ttcctgtagg aagaaaaata aaggcgtgga aagaaaaggg gaagaggtgg 1320

agccagcacc tattgtggac tctggaactg tatctgatca agacagctgc cttcagagct 1380
tgcctgattg tggagtaaag ggcacggaag gcctttcgtc ctgtggaaac agaaatgaag 1440
aaactggaac aaaatcttct ggaatgccca cagaccagga gtccctgagc agtggagatg 1500
ctgtgcttca gagagacttg gtcatggagc caggcacagc ccagtattcc tctggaggtg 1560
aactgggagg catttcaaca acaaatgtca gtaccccaga cactgcaggg gaaatggaac 1620
atgggctcat gaacccagat gccactgttt ggaagaatgt gcttcaggga ggggaaagta 1680
caaaggaaag atttgagaac tctaatattg gcacagctgg agcctctgac gtgcacgtca 1740
caagtaagcc tgtggataaa atcagtgttc caaactgtgc ccctgctgcc agttccctgg 1800
atggtaacaa acctgctgag tcttcacttg catttagtaa tgaagaaacc tccactgaaa 1860
aaacagcaga aacggaaact tcacgaagtc gtgaggagag tgctgatgct ccagtagatc 1920
agaattctgt ggtgattcca gctgctgcaa aagacaagat ttcagatgga ttagaacctt 1980
atactctctt agcagcaggc ataggtgagg caatgtcacc ctcagattta gcccttcttg 2040
ggctggaaga agatgtaatg ccacaccaga actcagaaac aaattcatct catgctcaaa 2100
gccaaaaggg caaatcctca cccatttgtt ctacaactgg agacgataaa ctttgtgcag 2160
actctgcatg tcaacagaac acagtgactt ctagtggcga tttggttgca aaactgtgtg 2220
ataacatagt tagcgagtcc gaaagcacca cagcaaggca acccagctca caagatccac 2280
ccgatgcctc ccactgtgaa gacccacagg ctcatacagt cacctctgac cctgtaaggg 2340
atacccagga acgtgcggat ttttgtcctt tcaaagtggt ggataacaaa ggccaacgaa 2400
aagatgtgaa actagataaa cctttaacaa atatgcttga ggtggtttca catccacatc 2460
cagttgtccc taaaatggag aaagaactgg tgccagacca ggcagtaata tcagacagta 2520
ctttctctct ggcaaacagt ccaggcagtg aatcagtaac caaggatgac gcactttctt 2580
ttgtcccctc ccagaaagaa aagggaacag caactcctga actacataca gctacagatt 2640
atagagatgg cccagatgga aattcgaatg agcctgatac gcggccacta gaagacaggg 2700
cagtaggcct gtccacatcc tccactgctg cagagcttca gcacgggatg gggaatacca 2760
gtctcacagg acttggtgga gagcatgagg gtcccgcccc tccagcaatc ccagaagctc 2820
tgaatatcaa ggggaacact gactcttccc tgcaaagtgt gggtaaggcc actttggctt 2880
tagattcagt tttgactgaa gaaggaaaac ttctggtggt ttcagaaagc tctgcagctc 2940
aggaacaaga taaggataaa gcggtgacct gttcctctat taaggaaaat gctctctctt 3000
caggaacttt gcaggaagag cagagaacac cacctcctgg acaagatact caacaatttc 3060
atgaaaaatc aatctcagct gactgtgcca aggacaaagc acttcagcta agtaattcac 3120
cgggtgcatc ctctgccttt cttaaggcag aaactgaaca taacaaggaa gtggccccac 3180

aagtctcact gctgactcaa ggtggggctg cccagagcct ggtgccacca ggagcaagtc 3240
tggccacaga gtcaaggcag gaagccttgg gggcagagca caacagctcc gctctgttgc 3300
catgtctgtt gccagatggg tctgatgggt ccgatgctct taactgcagt cagccttctc 3360
ctctggatgt tggagtgaag aacactcaat cccagggaaa aactagtgcc tgtgaggtga 3420
gtggagatgt gacggtggat gttacagggg ttaatgctct acaaggtatg gctgagccca 3480
gaagagagaa tatatcacac aacacccaag acatcctgat tccaaacgtc ttgttgagcc 3540
aagagaagaa tgccgttcta ggtttgccag tggctctaca ggacaaagct gtgactgacc 3600
cacagggagt tggaacccca gagatgatac ctcttgattg ggagaaaggg aagctggagg 3660
gagcagacca cagctgtacc atgggtgacg ctgaggaagc ccaaatagac gatgaagcac 3720
atcctgtcct actgcagcct gttgccaagg agctccccac agacatggag ctctcagccc 3780
atgatgatgg ggccccagct ggtgtgaggg aagtcatgcg agccccgcct tcaggcaggg 3840
aaaggagcac tccctctcta ccttgcatgg tctctgccca ggacgcacct ctgcctaagg 3900
gggcagactt gatagaggag gctgccagcc gtatagtgga tgctgtcatc gaacaagtca 3960
aggccgctgg agcactgctt actgaggggg aggcctgtca catgtcactg tccagccctg 4020
agttgggtcc tctcactaaa ggactagaga gtgcttttac agaaaaagtg agtactttcc 4080
cacctgggga gagcctacca atgggcagta ctcctgagga agccacgggg agccttgcag 4140
gatgttttgc tggaagggag gagccagaga agatcatttt acctgtccag gggcctgagc 4200
cagcagcaga aatgccagac gtgaaagctg aagatgaagt ggattttaga gcaagttcaa 4260
tttctgaaga agtggctgta gggagcatag ctgctacact gaagatgaag caaggcccaa 4320
tgacccaggc gataaaccga gaaaactggt gtacaataga gccatgccct gatgcagcat 4380
ctcttctggc ttccaagcag agcccagaat gtgagaactt cctggatgtt ggactgggca 4440
gagagtgtac ctcaaaacaa ggtgtactta aaagagaatc tgggagtgat tctgacctct 4500
ttcactcacc cagtgatgac atggacagca tcatcttccc aaagccagag gaagagcatt 4560
tggcctgtga tatcaccgga tccagttcat ccaccgatga cacggcttca ctggaccgac 4620
attcttctca tggcagtgat gtgtctctct cccagatttt aaagccaaac aggtcaagag 4680
atcggcaaag ccttgatgga ttctacagcc atgggatggg agctgagggt cgagaaagtg 4740
agagtgagcc tgctgaccca ggcgacgtgg aggaggagga gatggacagt atcactgaag 4800
tgcctgcaaa ctgctctgtc ctaaggagct ccatgcgctc tctttctccc ttccggaggc 4860
acagctgggg gcctgggaaa aatgcagcca gcgatgcaga aatgaaccac cggagttcaa 4920
tgcgagttct tggggatgtt gtcaggagac ctcccattca taggagaagt ttcagtctag 4980

aaggcttgac aggaggagct ggtgtcggaa acaagccatc ctcatctcta gaagtaagct 5040
ctgcaaatgc cgaagagctc agacacccat tcagtggtga ggaacgggtt gactctttgg 5100
tgtcactttc agaagaggat ctggagtcag accagagaga acataggatg tttgatcagc 5160
agatatgtca cagatctaag cagcagggat ttaattactg tacatcagcc atttcctctc 5220
cattgacaaa atccatctca ttaatgacaa tcagccatcc tggattggac aattcacggc 5280
ccttccacag taccttccac aataccagtg ctaatctgac tgagagtata acagaagaga 5340
actataattt cctgccacat agcccctcca agaaagattc tgaatggaag agtggaacaa 5400
aagtcagtcg tacattcagc tacatcaaga ataaaatgtc tagcagcaag aagagcaaag 5460
aaaaggaaaa agaaaaagat aagattaagg agaaggagaa agattctaaa gacaaggaga 5520
aagataagaa gactgtcaac gggcacactt tcagttccat tcctgttgtg ggtcccatca 5580
gctgtagcca gtgtatgaag cccttcacca acaaagatgc ctatacttgt gcaaattgca 5640
gtgcttttgt ccacaaaggc tgccgagaaa gtctagcctc ctgtgcaaag gtcaaaatga 5700
agcagcccaa agggagcctt caggcacatg acacatcatc actgcccacg gtcattatga 5760
gaaacaagcc ctcacagccc aaggagcgtc ctcggtccgc agtcctcctg gtggatgaaa 5820
ccgctaccac cccaatattt gccaatagac gatcccagca gagtgtctcg ctctccaaaa 5880
gtgtctccat acagaacatt actggagttg gcaatgatga gaacatgtca aacacctgga 5940
aattcctgtc tcattcaaca gactcactaa ataaaatcag caaggtcaat gagtcaacag 6000
aatcacttac tgatgaggga gtaggtacag acatgaatga aggacaacta ctgggagact 6060
ttgagattga gtccaaacag ctggaagcag agtcttggag tcggataata gacagcaagt 6120
ttctaaaaca gcaaaagaaa gatgtggtca aacggcaaga agtaatatat gagttgatgc 6180
agacagagtt tcatcatgtc cgcactctca agatcatgag tggtgtgtac agccagggga 6240
tgatggcgga tctgcttttt gagcagcaga tggtagaaaa gctgttcccc tgtttggatg 6300
agctgatcag tatccatagc caattcttcc agaggattct ggagcggaag aaggagtctc 6360
tggtggataa aagtgaaaag aactttctca tcaagaggat aggggatgtg cttgtaaatc 6420
agttttcagg tgagaatgca gaacgtttaa agaagacata tggcaagttt tgtgggcaac 6480
ataaccagtc tgtaaactac ttcaaagacc tttatgccaa ggataagcgt tttcaagcct 6540
ttgtaaagaa gaagatgagc agttcagttg ttagaaggct tggaattcca gagtgcatat 6600
tgcttgtaac tcagcggatt accaagtacc cagttttatt ccaaagaata ttgcagtgta 6660
ccaaagacaa tgaagtggag caggaagatc tagcacagtc cttgagcctg gtgaaggatg 6720
tgattggagc tgtagacagc aaagtggcaa gttatgaaaa gaaagtgcgt ctcaatgaga 6780
tttatacaaa gacagatagc aagtcaatca tgaggatgaa gagtggtcag atgtttgcca 6840

aggaagattt gaaacggaag aagcttgtac gtgatgggag tgtgtttctg aagaatgcag 6900
caggaaggtt gaaagaggtt caagcagttc ttctcactga cattttagtt ttccttcaag 6960
aaaaagacca gaagtacatc tttgcatcat tggaccagaa gtcaacagtg atctctttaa 7020
agaagctgat tgtgagagaa gtggcacatg aggagaaagg tttattcctg atcagcatgg 7080
ggatgacaga tccagagatg gtagaagtcc atgccagctc caaagaggaa cgaaacagct 7140
ggattcagat cattcaggac acaatcaaca ccctgaacag agatgaagat gaaggaattc 7200
ctagtgagaa tgaggaagaa aagaaaatgt tggacaccag agcccgagaa ttaaaagaac 7260
aacttcacca gaaggaccaa aaaatcctac tcttgttgga agagaaggag atgattttcc 7320
gggacatggc tgagtgcagc acccctctcc cagaggattg ctccccaaca catagcccta 7380
gagttctctt ccgctccaac acagaagagg ctctcaaagg aggaccttta atgaaaagtg 7440
caataaatga ggtggagatc cttcagggtt tggtgagtgg aaatctggga ggcacacttg 7500
ggccgactgt cagcagcccc attgagcaag atgtggtcgg tcccgtttcc ctgccccgga 7560
gagcagagac ctttggagga tttgacagcc atcagatgaa tgcttcaaaa ggaggcgaga 7620
aggaagaggg agatgatggc caagatctta ggagaacgga atcagatagt ggcctaaaaa 7680
agggtggaaa tgctaacctg gtatttatgc ttaaaagaaa cagtgagcag gttgtccaga 7740
gcgttgttca tctctacgag ctcctcagcg ctctgcaggg tgtggtgctg cagcaggaca 7800
gctacattga ggaccagaaa ctggtgctga gcgagagggc gctcactcgc agcttgtccc 7860
gcccgagctc cctcattgag caggagaagc agcgcagcct ggagaagcag cgccaggacc 7920
tggccaacct gcagaagcag caggcccagt acctcgagga gaagcgcagg cgcgagcgtg 7980
agtgggaagc tcgtgagagg gagctgcggg agcgggaggc cctcctggcc cagcgcgagg 8040
aggaggtgca gcaggggcag caggacctgg aaaaggagcg ggaggagctc cagcagaaga 8100
agggcacata ccagtatgac ctggagcgac tgcgtgctgc ccagaaacag cttgagaggg 8160
aacaggagca gctgcgccgg gaggcagagc ggctcagcca gcggcagaca gaacgggacc 8220
tgtgtcaggt ttcccatcca cataccaagc tgatgaggat cccatcgttc ttccccagtc 8280
ctgaggagcc cccctcgcca tctgcacctt ccatagccaa atcagggtca ttggactcag 8340
aactttcagt gtccccaaaa aggaacagca tctctcggac acacaaagat aaggggcctt 8400
ttcacatact gagttcaacc agccagacaa acaaaggacc agaagggcag agccaggccc 8460
ctgcgtccac ctctgcctct acccgcctgt ttgggttaac aaagccaaag gaaaagaagg 8520
agaaaaaaaa gaagaacaaa accagccgct ctcagcccgg tgatggtccc gcgtcagaag 8580
tatcagcaga gggtgaagag atcttctgct gaccctcttc ctctctgctg aggcagctgc 8640

ctcctgatcc tggccagccc acctctcctg ctgtccccgc gtgcacaagt ctcttacact 8700
ggacgcccac tgctcctcag cgtccagtcc tcctgggcgg ccccaggtcc tggacaataa 8760
gcaacagatg atattgagtg tcgggtgggg aaggaggccc agactctgct tcggccatga 8820
tttgtgactg cccaggactc tcaggttggg ctggccctac tcaggattac actgaaagta 8880
atggcctcgt aagtacaggt gatggttttg gacacgtcag gaattcctaa aggctgaaag 8940
agtgtatcca agtaaggtct gaacctccga atgcctttta tttgggggaa cacaaaacca 9000
aacagcagat gttttggact tgatctgtgt acgtacatgg ggacctgtct gcatatacac 9060
acggggaatg ccagaagaag gcccagtctg caccaggcgt ctggtcaact tagcacaagg 9120
gcagtgcctg gacggacccg gagcccccgc atatcagcag ttcacccagt actcctcaga 9180
gactggtttc cctctaaacc catcccgggc acataccacc cgtgttttgc atgtatttct 9240
catttcattt tagggatgac aaacatttgt gaaaccagtg agagaaggct tgatgtgtat 9300
aaaagacgtg atgtgcacca cctcgatctc ggtgtttcag gcactaaagc aacaaaacaa 9360
cccatagtat ctcattctgt catcagatcc agaagaaata tcctggtttt ccagcatgtt 9420
tacccacatg ttttggccat ggataaagtg aagaggccta ctcaccatta tccctgcagc 9480
gtgacacctt ttgattgtca ctgaccactc agaaggggcc acggcctcct ggctgtgttc 9540
ctgagccccc gtcgtgcctc tcccagacag cagctgtctg gcccttgctg ggtgagggca 9600
caccactgcc aggggtcagc ctcgcaccca ggccaggcag aagctgtgct ctgaagctag 9660
gacagctggc tgagaagtgg gttcaggcga agggtgaagc catgtgtagc agttcctgcc 9720
agtgcagatc tggagaggag ctggcccgga aggcgtggtt gtgaaagcgc ccttcttatg 9780
ttaggaggcc ttggcaaaat tggatttctt caaaaataca tgtaaaggtc tgttgttgaa 9840
ttgtactctg cccctggaag cagatacaga tggctgcctg ctgctcggct ttgcttttgc 9900
ttttcccacc gtgttttcat ctttgttcac ttgaggcttt ccccagctgg tgtgtgcagg 9960
acagttcatg gtaatgttgc cctctgaggc cccgtacacc agaagggagg ccctggaaaa 10020
ttttgtgctt ccaacgtggc cttcaattct tgcttttttg cccctcggaa gcatggggct 10080
tttgagcaca cttaaaaaaa gaaaaatctg taacttggtg cttattgatg aattgcaagc 10140
tggccttgca gatggagata tttatctttc agtttatttg aaagaggtct ggtttaaaat 10200
ttgtagccta catttgtttt atttattgta tttgtgtgtt tgtgtttgtt ttttttaag 10260
ggtgagccag gtctagccca acagtctaaa ctatccagtc aataccgagt gaagtggcag 10320
ccagcactgt tcactctgtg tcttttgaag tgccttgaag gcccagatga aattttaaag 10380
ggagggggtc catgtccttc cctcccccac cccgcctcat tctttaatca aaggatgtct 10440
tctcccttgt ttgagaatga agaaactcgc cacctctgac ctacctttgc ctttttctgt 10500

catggagaat actcaccctt cagaaacaga ccaaaggcca aaacctgctg atttttctat 10560
tgaaaatatg tccccttgca aagaccctaa acaaaaagtt aagtttcttt ctttcaccta 10620
tttgtacaac tccaagttac agctgaatct gtcgtgactt tcctgagatc tacccggggc 10680
ttggctgtct gttctgggca ctggctccga gttcccctcc tgggatttgc aggagggcag 10740
tactgaacct gcattcttct ccttgtaaat gtaggccggg tgcccctgtt ctccgggttt 10800
ggaacaatac gaggttggtg ctgatgggat ttacttgcgt acgtgctctt cacaaaaaca 10860
ccgtggatgc tgaagttaga gcacgtcgcc acagagcttg acatcaatgt tagagggtct 10920
cttactcccc gcccagctgt gatgtttcat ctgctttggt tgttttggtg gtctttttta 10980
aaaatagaga tttcacatct gcccagaccc cactcaaaac gatttggtca ggttctggtt 11040
ggacaagttt aaaatcaaag tagtgcccgg aattccctca aaccacccaa cttcatccag 11100
gaatacagtc tgcagtgcag caacagaacc gcttaccaag aactgtgctt acataccttt 11160
gtcatctctc ttcccccctt ggaagttgtc ctcaggggga tttgttcctg tcctggggat 11220
ttacctggga tggtggctgc ctgtgctttt gctcatggcc ttgacagtgc tctagttgct 11280
ggatctaatg gcctgtcttg gtttctatca catgagaagg ggttgttttt ttggggtgac 11340
tcggactgaa ttccccatac tgtttccacg ccgggacacc atgttctcca tcaagctaaa 11400
gaaatcacgt gcctgaaact gtgcttaagt tttgggggaa agatggagtt cctatccaga 11460
gcccccagat ttccagaatc gagtgagctt cctggaagga gactgcgtct tctctcaatt 11520
ccagtcatct cagtcgttgt cgttaggtga catgtgcact ttaaatgctc tcatcggttg 11580
gcttcatttt caagacaatc aaatgtattg actgtgtttt cttcttagaa aatggagagg 11640
gttaaaaaca tgcaaactgc cactttcaac ctttgccagt attccctcta cccccgtgag 11700
agctatctgg ggggaagaat ccttaccaag gtttttttgg aaaggtacga atcttaactt 11760
ttttcccctt ctgtgtctca gggtaatact attcagagtc gcccctttgc tcattttctc 11820
ccgtatttgt taccttcctg aggcctcagt attagtcgtg agcacaaagt tttgagacct 11880
ttggcgttgt ttcttgatgt gggaggggag gtgttagtgc atgcaagggt tgaactagat 11940
agaccctgcc ttagtagagg gtgggactat aaccttagag gccagaactt gatccagaag 12000
ttgctgtcca cagaagtgct ttctatttca tcatttttgt ttctagggct ctttttctgt 12060
agccaggtct tcccaaggat tttagtattt gcattggagt tgaggtttac tctaatgatg 12120
gtggcccagc tgtgcccaga ggacagccag gcaggccctg ggagggagtt tagaaagaca 12180
gtcctggtga atgggcttca agtggtcaca aagagggtgg ctgtgaggtg accccagaca 12240
ctgcagaacg atgtgcaccc tctgcgtttt ggatgtcctt ggaatgtggg agcctagaaa 12300

```
taaccctgtg gatggaattg gggcagcggc tgctggagat ctgtgtgcct tgccttcctt  12360
cagcaggacc gtctaggtgc gcagccacct atggatgcgt cccagccagc cccgtcgctc  12420
tcgtccatcc tcagagacaa agaagagggc agggagtttg ggcttggttt tgaactttcc  12480
tttcaatgta gcaaagcatt cctagttaac cagagccttg gaatctactg cctgctggcc  12540
aggctttaaa atgaaaagtg ttttaatgct gccataaaag ggaggcgggg gggaggaagg  12600
gaaaataaag gcatctttcc aagtactcat ctaatttaat tgtcaaaaga ttgataggcc  12660
atgaattact tctccatctc actaagggtt aaaggcgtgc aaccccccac tggctgtgtc  12720
ccctgccacc gaagtgagtg acctgcccta caaccaggtg ggaccacctg tgctgcagtc  12780
cggaggggct tctgcaggaa gcactcaccc cccacacctt ccccggcctg agcttcccct  12840
acctttcgtc accacctgag ggcatgagca caggccatgg ggcgtgcctg gtgagtctgc  12900
ctgtggttca ggcttagcct gtggtctcct gtgtgctgct gcccgcatgg gatgcgcagg  12960
ggaggcgtgg ggatccgcag gagggtggtt gggatacacc ggatacctct gctctcattg  13020
cttgtttgca aatgctctat ggacatttgt gtgctaaatc ctattaaata aaaaagacgg  13080
gttaaaaccc agatgctgta tattcatttg taattatgta taaagtgaag cagttttaaa  13140
ctgtaaagat ttttttcagt gtgttttctc gaattttgcc acaacatact ggcttcgtat  13200
tttatttatc tttctttcta gttaccagct tcagaccctt gtaaagtctc cctcagccct  13260
ttcaaaaaat aataaatttc ctgtgaagtt                                    13290
```

<210> 24
<211> 13302
<212> DNA
<213> Human

<400> 24

```
gaagcgcctg tgctctgccg agactgccgt gcccattgct cgcctcggtc gccgccgctt     60
tagccgcctc cgggggagcg gccgcctatt gtctttctcc gcggcgaagg tgaagagttg    120
tcccagctcg gcccgcgggg gagccccggg agccgcacgt gtcctgggtc atgaaactta    180
atccacagca agctccctta tatggtgatt gtgttgttac agtgctgctt gctgaagagg    240
acaaagctga agatgatgta gtgttttact tggtattttt gggttccacc ctccgtcact    300
gtacaagtac tcggaaggtc agttctgata cattggagac cattgctcct ggtcatgatt    360
gttgtgaaac agtgaaggtg cagctctgtg cttccaaaga gggccttccc gtgtttgtgg    420
tggctgaaga agactttcat ttcgtccagg atgaagcgta tgatgcagct caattcctag    480
caaccagtgc tggaaatcag caggctttga actttacccg ttttcttgac cagtcaggac    540
ccccatctgg ggatgtgaat tcccttgata agaagttggt gctggcattc aggcacctga    600
```

agctgcccac ggagtggaat gtattgggga cagatcagag tttgcatgat gctggcccgc 660
gagagacatt gatgcatttt gctgtgcggc tgggactgct gaggttgacg tggttcctgt 720
tgcagaagcc aggtggccgc ggagctctca gtatccacaa ccaggaaggg gcgacgcctg 780
tgagcttggc cttggagcga ggctatcaca agctgcacca gcttctaacc gaggagaatg 840
ctggagaacc agactcctgg agcagtttat cctatgaaat accgtatgga gactgttctg 900
tgaggcatca tcgagagttg gacatctata cattaacctc tgagtctgat tcacatcatg 960
aacacccatt tcctggagac ggttgcactg gaccaatttt taaacttatg aacatccaac 1020
agcaactaat gaaaacaaac ctcaagcaga tggacagtct tatgccctta atgatgacag 1080
cacaggatcc ttccagtgcc ccagagacag atggccagtt tcttccctgt gcaccggagc 1140
ccacggaccc tcagcgactt tcttcttctg aagagactga gagcactcag tgctgcccag 1200
ggagccctgt tgcacagact gaaagtccct gtgatttgtc aagcatagtt gaggaggaga 1260
atacagaccg ttcctgtagg aagaaaaata aaggcgtgga aagaaaaggg gaagaggtgg 1320
agccagcacc tattgtggac tctggaactg tatctgatca agacagctgc cttcagagct 1380
tgcctgattg tggagtaaag ggcacggaag gcctttcgtc ctgtggaaac agaaatgaag 1440
aaactggaac aaaatcttct ggaatgccca cagaccagga gtccctgagc agtggagatg 1500
ctgtgcttca gagagacttg gtcatggagc caggcacagc ccagtattcc tctggaggtg 1560
aactgggagg catttcaaca acaaatgtca gtaccccaga cactgcaggg gaaatggaac 1620
atgggctcat gaacccagat gccactgttt ggaagaatgt gcttcaggga ggggaaagta 1680
caaaggaaag atttgagaac tctaatattg gcacagctgg agcctctgac gtgcacgtca 1740
caagtaagcc tgtggataaa atcagtgttc caaactgtgc ccctgctgcc agttccctgg 1800
atggtaacaa acctgctgag tcttcacttg catttagtaa tgaagaaacc tccactgaaa 1860
aaacagcaga aacggaaact tcacgaagtc gtgaggagag tgctgatgct ccagtagatc 1920
agaattctgt ggtgattcca gctgctgcaa aagacaagat ttcagatgga ttagaacctt 1980
atactctctt agcagcaggc ataggtgagg caatgtcacc ctcagattta gcccttcttg 2040
ggctggaaga agatgtaatg ccacaccaga actcagaaac aaattcatct catgctcaaa 2100
gccaaaaggg caaatcctca cccatttgtt ctacaactgg agacgataaa ctttgtgcag 2160
actctgcatg tcaacagaac acagtgactt ctagtggcga tttggttgca aaactgtgtg 2220
ataacatagt tagcgagtcc gaaagcacca cagcaaggca acccagctca caagatccac 2280
ccgatgcctc ccactgtgaa gacccacagg ctcatacagt cacctctgac cctgtaaggg 2340
atacccagga acgtgcggat ttttgtcctt tcaaagtggt ggataacaaa ggccaacgaa 2400
aagatgtgaa actagataaa cctttaacaa atatgcttga ggtggtttca catccacatc 2460

```
cagttgtccc taaaatggag aaagaactgg tgccagacca ggcagtaata tcagacagta   2520
ctttctctct ggcaaacagt ccaggcagtg aatcagtaac caaggatgac gcactttctt   2580
ttgtcccctc ccagaaagaa aagggaacag caactcctga actacataca gctacagatt   2640
atagagatgg cccagatgga aattcgaatg agcctgatac gcggccacta gaagacaggg   2700
cagtaggcct gtccacatcc tccactgctg cagagcttca gcacgggatg gggaatacca   2760
gtctcacagg acttggtgga gagcatgagg gtcccgcccc tccagcaatc ccagaagctc   2820
tgaatatcaa ggggaacact gactcttccc tgcaaagtgt gggtaaggcc actttggctt   2880
tagattcagt tttgactgaa gaaggaaaac ttctggtggt ttcagaaagc tctgcagctc   2940
aggaacaaga taaggataaa gcggtgacct gttcctctat taaggaaaat gctctctctt   3000
caggaacttt gcaggaagag cagagaacac cacctcctgg acaagatact caacaatttc   3060
atgaaaaatc aatctcagct gactgtgcca aggacaaagc acttcagcta agtaattcac   3120
cgggtgcatc ctctgccttt cttaaggcag aaactgaaca taacaaggaa gtggccccac   3180
aagtctcact gctgactcaa ggtggggctg cccagagcct ggtgccacca ggagcaagtc   3240
tggccacaga gtcaaggcag gaagccttgg gggcagagca caacagctcc gctctgttgc   3300
catgtctgtt gccagatggg tctgatgggt ccgatgctct taactgcagt cagccttctc   3360
ctctggatgt tggagtgaag aacactcaat cccagggaaa aactagtgcc tgtgaggtga   3420
gtggagatgt gacggtggat gttacagggg ttaatgctct acaaggtatg gctgagccca   3480
gaagagagaa tatatcacac aacacccaag acatcctgat tccaaacgtc ttgttgagcc   3540
aagagaagaa tgccgttcta ggtttgccag tggctctaca ggacaaagct gtgactgacc   3600
cacagggagt tggaacccca gagatgatac ctcttgattg ggagaaaggg aagctggagg   3660
gagcagacca cagctgtacc atgggtgacg ctgaggaagc ccaaatagac gatgaagcac   3720
atcctgtcct actgcagcct gttgccaagg agctccccac agacatggag ctctcagccc   3780
atgatgatgg ggccccagct ggtgtgaggg aagtcatgcg agccccgcct tcaggcaggg   3840
aaaggagcac tccctctcta ccttgcatgg tctctgccca ggacgcacct ctgcctaagg   3900
gggcagactt gatagaggag gctgccagcc gtatagtgga tgctgtcatc gaacaagtca   3960
aggccgctgg agcactgctt actgaggggg aggcctgtca catgtcactg tccagccctg   4020
agttgggtcc tctcactaaa ggactagaga gtgcttttac agaaaaagtg agtactttcc   4080
cacctgggga gagcctacca atgggcagta ctcctgagga agccacgggg agccttgcag   4140
gatgttttgc tggaagggag gagccagaga agatcatttt acctgtccag gggcctgagc   4200
cagcagcaga aatgccagac gtgaaagctg aagatgaagt ggattttaga gcaagttcaa   4260
```

tttctgaaga agtggctgta gggagcatag ctgctacact gaagatgaag caaggcccaa 4320

tgacccaggc gataaaccga gaaaactggt gtacaataga gccatgccct gatgcagcat 4380

ctcttctggc ttccaagcag agcccagaat gtgagaactt cctggatgtt ggactgggca 4440

gagagtgtac ctcaaaacaa ggtgtactta aaagagaatc tgggagtgat tctgacctct 4500

ttcactcacc cagtgatgac atggacagca tcatcttccc aaagccagag gaagagcatt 4560

tggcctgtga tatcaccgga tccagttcat ccaccgatga cacggcttca ctggaccgac 4620

attcttctca tggcagtgat gtgtctctct cccagatttt aaagccaaac aggtcaagag 4680

atcggcaaag ccttgatgga ttctacagcc atgggatggg agctgagggt cgagaaagtg 4740

agagtgagcc tgctgaccca ggcgacgtgg aggaggagga gatggacagt atcactgaag 4800

tgcctgcaaa ctgctctgtc ctaaggagct ccatgcgctc tctttctccc ttccggaggc 4860

acagctgggg gcctgggaaa aatgcagcca gcgatgcaga aatgaaccac cggagtatga 4920

gctggtgccc ctctggtgtg cagtactctg ctggcctgag tgctgacttt aattacagaa 4980

gtttcagtct agaaggcttg acaggaggag ctggtgtcgg aaacaagcca tcctcatctc 5040

tagaagtaag ctctgcaaat gccgaagagc tcagacaccc attcagtggt gaggaacggg 5100

ttgactcttt ggtgtcactt tcagaagagg atctggagtc agaccagaga gaacatagga 5160

tgtttgatca gcagatatgt cacagatcta agcagcaggg atttaattac tgtacatcag 5220

ccatttcctc tccattgaca aaatccatct cattaatgac aatcagccat cctggattgg 5280

acaattcacg gcccttccac agtaccttcc acaataccag tgctaatctg actgagagta 5340

taacagaaga gaactataat ttcctgccac atagcccctc caagaaagat tctgaatgga 5400

agagtggaac aaaagtcagt cgtacattca gctacatcaa gaataaaatg tctagcagca 5460

agaagagcaa agaaaaggaa aaagaaaaag ataagattaa ggagaaggag aaagattcta 5520

aagacaagga gaaagataag aagactgtca acgggcacac tttcagttcc attcctgttg 5580

tgggtcccat cagctgtagc cagtgtatga agcccttcac caacaaagat gcctatactt 5640

gtgcaaattg cagtgctttt gtccacaaag gctgccgaga aagtctagcc tcctgtgcaa 5700

aggtcaaaat gaagcagccc aaagggagcc ttcaggcaca tgacacatca tcactgccca 5760

cggtcattat gagaaacaag ccctcacagc ccaaggagcg tcctcggtcc gcagtcctcc 5820

tggtggatga aaccgctacc accccaatat ttgccaatag acgatcccag cagagtgtct 5880

cgctctccaa aagtgtctcc atacagaaca ttactggagt tggcaatgat gagaacatgt 5940

caaacacctg gaaattcctg tctcattcaa cagactcact aaataaaatc agcaaggtca 6000

atgagtcaac agaatcactt actgatgagg gagtaggtac agacatgaat gaaggacaac 6060

tactgggaga ctttgagatt gagtccaaac agctggaagc agagtcttgg agtcggataa 6120

tagacagcaa gtttctaaaa cagcaaaaga aagatgtggt caaacggcaa gaagtaatat 6180

atgagttgat gcagacagag tttcatcatg tccgcactct caagatcatg agtggtgtgt 6240

acagccaggg gatgatggcg gatctgcttt ttgagcagca gatggtagaa aagctgttcc 6300

cctgtttgga tgagctgatc agtatccata gccaattctt ccagaggatt ctggagcgga 6360

agaaggagtc tctggtggat aaaagtgaaa agaactttct catcaagagg ataggggatg 6420

tgcttgtaaa tcagttttca ggtgagaatg cagaacgttt aaagaagaca tatggcaagt 6480

tttgtgggca acataaccag tctgtaaact acttcaaaga cctttatgcc aaggataagc 6540

gttttcaagc ctttgtaaag aagaagatga gcagttcagt tgttagaagg cttggaattc 6600

cagagtgcat attgcttgta actcagcgga ttaccaagta cccagtttta ttccaaagaa 6660

tattgcagtg taccaaagac aatgaagtgg agcaggaaga tctagcacag tccttgagcc 6720

tggtgaagga tgtgattgga gctgtagaca gcaaagtggc aagttatgaa aagaaagtgc 6780

gtctcaatga gatttataca aagacagata gcaagtcaat catgaggatg aagagtggtc 6840

agatgtttgc caaggaagat ttgaaacgga agaagcttgt acgtgatggg agtgtgtttc 6900

tgaagaatgc agcaggaagg ttgaaagagg ttcaagcagt tcttctcact gacattttag 6960

ttttccttca agaaaaagac cagaagtaca tctttgcatc attggaccag aagtcaacag 7020

tgatctcttt aaagaagctg attgtgagag aagtggcaca tgaggagaaa ggtttattcc 7080

tgatcagcat ggggatgaca gatccagaga tggtagaagt ccatgccagc tccaaagagg 7140

aacgaaacag ctggattcag atcattcagg acacaatcaa caccctgaac agagatgaag 7200

atgaaggaat tcctagtgag aatgaggaag aaaagaaaat gttggacacc agagcccgag 7260

aattaaaaga acaacttcac cagaaggacc aaaaaatcct actcttgttg gaagagaagg 7320

agatgatttt ccgggacatg gctgagtgca gcacccctct cccagaggat tgctccccaa 7380

cacatagccc tagagttctc ttccgctcca acacagaaga ggctctcaaa ggaggacctt 7440

taatgaaaag tgcaataaat gaggtggaga tccttcaggg tttggtgagt ggaaatctgg 7500

gaggcacact tgggccgact gtcagcagcc ccattgagca agatgtggtc ggtcccgttt 7560

ccctgccccg gagagcagag acctttggag gatttgacag ccatcagatg aatgcttcaa 7620

aaggaggcga gaaggaagag ggagatgatg gccaagatct taggagaacg gaatcagata 7680

gtggcctaaa aaagggtgga aatgctaacc tggtatttat gcttaaaaga aacagtgagc 7740

aggttgtcca gagcgttgtt catctctacg agctcctcag cgctctgcag ggtgtggtgc 7800

tgcagcagga cagctacatt gaggaccaga aactggtgct gagcgagagg gcgctcactc 7860

gcagcttgtc ccgcccgagc tccctcattg agcaggagaa gcagcgcagc ctggagaagc 7920

agcgccagga cctggccaac ctgcagaagc agcaggccca gtacctcgag gagaagcgca 7980
ggcgcgagcg tgagtgggaa gctcgtgaga gggagctgcg ggagcgggag gccctcctgg 8040
cccagcgcga ggaggaggtg cagcaggggc agcaggacct ggaaaaggag cgggaggagc 8100
tccagcagaa gaagggcaca taccagtatg acctggagcg actgcgtgct gcccagaaac 8160
agcttgagag ggaacaggag cagctgcgcc gggaggcaga gcggctcagc cagcggcaga 8220
cagaacggga cctgtgtcag gtttcccatc cacataccaa gctgatgagg atcccatcgt 8280
tcttccccag tcctgaggag cccccctcgc catctgcacc ttccatagcc aaatcagggt 8340
cattggactc agaactttca gtgtccccaa aaaggaacag catctctcgg acacacaaag 8400
ataaggggcc ttttcacata ctgagttcaa ccagccagac aaacaaagga ccagaagggc 8460
agagccaggc ccctgcgtcc acctctgcct ctacccgcct gtttgggtta acaaagccaa 8520
aggaaaagaa ggagaaaaaa aagaagaaca aaaccagccg ctctcagccc ggtgatggtc 8580
ccgcgtcaga agtatcagca gagggtgaag agatcttctg ctgaccctct tcctctctgc 8640
tgaggcagct gcctcctgat cctggccagc ccacctctcc tgctgtcccc gcgtgcacaa 8700
gtctcttaca ctggacgccc actgctcctc agcgtccagt cctcctgggc ggccccaggt 8760
cctggacaat aagcaacaga tgatattgag tgtcgggtgg ggaaggaggc ccagactctg 8820
cttcggccat gatttgtgac tgcccaggac tctcaggttg ggctggccct actcaggatt 8880
acactgaaag taatggcctc gtaagtacag gtgatggttt tggacacgtc aggaattcct 8940
aaaggctgaa agagtgtatc caagtaaggt ctgaacctcc gaatgccttt tatttggggg 9000
aacacaaaac caaacagcag atgttttgga cttgatctgt gtacgtacat ggggacctgt 9060
ctgcatatac acacggggaa tgccagaaga aggcccagtc tgcaccaggc gtctggtcaa 9120
cttagcacaa gggcagtgcc tggacggacc cggagccccc gcatatcagc agttcaccca 9180
gtactcctca gagactggtt tccctctaaa cccatcccgg gcacatacca cccgtgtttt 9240
gcatgtattt ctcatttcat tttagggatg acaaacattt gtgaaaccag tgagagaagg 9300
cttgatgtgt ataaaagacg tgatgtgcac cacctcgatc tcggtgtttc aggcactaaa 9360
gcaacaaaac aacccatagt atctcattct gtcatcagat ccagaagaaa tatcctggtt 9420
ttccagcatg tttacccaca tgttttggcc atggataaag tgaagaggcc tactcaccat 9480
tatccctgca gcgtgacacc ttttgattgt cactgaccac tcagaagggg ccacggcctc 9540
ctggctgtgt tcctgagccc ccgtcgtgcc tctcccagac agcagctgtc tggcccttgc 9600
tgggtgaggg cacaccactg ccaggggtca gcctcgcacc caggccaggc agaagctgtg 9660
ctctgaagct aggacagctg gctgagaagt gggttcaggc gaagggtgaa gccatgtgta 9720
gcagttcctg ccagtgcaga tctggagagg agctggcccg gaaggcgtgg ttgtgaaagc 9780

gcccttctta tgttaggagg ccttggcaaa attggatttc ttcaaaaata catgtaaagg 9840
tctgttgttg aattgtactc tgcccctgga agcagataca gatggctgcc tgctgctcgg 9900
ctttgctttt gcttttccca ccgtgttttc atctttgttc acttgaggct ttccccagct 9960
ggtgtgtgca ggacagttca tggtaatgtt gccctctgag gccccgtaca ccagaaggga 10020
ggccctggaa aattttgtgc ttccaacgtg gccttcaatt cttgcttttt tgcccctcgg 10080
aagcatgggg cttttgagca cacttaaaaa aagaaaaatc tgtaacttgg tgcttattga 10140
tgaattgcaa gctggccttg cagatggaga tatttatctt tcagtttatt tgaaagaggt 10200
ctggtttaaa atttgtagcc tacatttgtt ttatttattg tatttgtgtg tttgtgtttg 10260
ttttttttta agggtgagcc aggtctagcc caacagtcta aactatccag tcaataccga 10320
gtgaagtggc agccagcact gttcactctg tgtcttttga agtgccttga aggcccagat 10380
gaaattttaa agggaggggg tccatgtcct tccctccccc accccgcctc attctttaat 10440
caaaggatgt cttctccctt gtttgagaat gaagaaactc gccacctctg acctaccttt 10500
gccttttttct gtcatggaga atactcaccc ttcagaaaca gaccaaaggc caaaacctgc 10560
tgatttttct attgaaaata tgtccccttg caaagaccct aaacaaaaag ttaagtttct 10620
ttctttcacc tatttgtaca actccaagtt acagctgaat ctgtcgtgac tttcctgaga 10680
tctacccggg gcttggctgt ctgttctggg cactggctcc gagttcccct cctgggattt 10740
gcaggagggc agtactgaac ctgcattctt ctccttgtaa atgtaggccg ggtgcccctg 10800
ttctccgggt ttggaacaat acgaggttgg tgctgatggg atttacttgc gtacgtgctc 10860
ttcacaaaaa caccgtggat gctgaagtta gagcacgtcg ccacagagct tgacatcaat 10920
gttagagggt ctcttactcc ccgcccagct gtgatgtttc atctgctttg gttgttttgg 10980
tggtcttttt taaaaataga gatttcacat ctgcccagac cccactcaaa acgatttggt 11040
caggttctgg ttggacaagt ttaaaatcaa agtagtgccc ggaattccct caaaccaccc 11100
aacttcatcc aggaatacag tctgcagtgc agcaacagaa ccgcttacca agaactgtgc 11160
ttacatacct ttgtcatctc tcttcccccc ttggaagttg tcctcagggg gatttgttcc 11220
tgtcctgggg atttacctgg gatggtggct gcctgtgctt ttgctcatgg ccttgacagt 11280
gctctagttg ctggatctaa tggcctgtct tggtttctat cacatgagaa ggggttgttt 11340
ttttggggtg actcggactg aattccccat actgtttcca cgccgggaca ccatgttctc 11400
catcaagcta aagaaatcac gtgcctgaaa ctgtgcttaa gttttggggg aaagatggag 11460
ttcctatcca gagcccccag atttccagaa tcgagtgagc ttcctggaag gagactgcgt 11520
cttctctcaa ttccagtcat ctcagtcgtt gtcgttaggt gacatgtgca ctttaaatgc 11580

tctcatcggt tggcttcatt ttcaagacaa tcaaatgtat tgactgtgtt ttcttcttag 11640
aaaatggaga gggttaaaaa catgcaaact gccactttca acctttgcca gtattccctc 11700
tacccccgtg agagctatct gggggaaga atccttacca aggtttttt ggaaaggtac 11760
gaatcttaac tttttcccc ttctgtgtct cagggtaata ctattcagag tcgcccttt 11820
gctcattttc tcccgtattt gttaccttcc tgaggcctca gtattagtcg tgagcacaaa 11880
gttttgagac ctttggcgtt gtttcttgat gtgggagggg aggtgttagt gcatgcaagg 11940
gttgaactag atagaccctg ccttagtaga gggtgggact ataaccttag aggccagaac 12000
ttgatccaga agttgctgtc cacagaagtg ctttctattt catcatttt gtttctaggg 12060
ctctttttct gtagccaggt cttcccaagg attttagtat ttgcattgga gttgaggttt 12120
actctaatga tggtggccca gctgtgccca gaggacagcc aggcaggccc tgggagggag 12180
tttagaaaga cagtcctggt gaatgggctt caagtggtca caaagagggt ggctgtgagg 12240
tgaccccaga cactgcagaa cgatgtgcac cctctgcgtt ttggatgtcc ttggaatgtg 12300
ggagcctaga aataaccctg tggatggaat tggggcagcg gctgctggag atctgtgtgc 12360
cttgccttcc ttcagcagga ccgtctaggt gcgcagccac ctatggatgc gtcccagcca 12420
gccccgtcgc tctcgtccat cctcagagac aaagaagagg gcagggagtt tgggcttggt 12480
tttgaacttt cctttcaatg tagcaaagca ttcctagtta accagagcct tggaatctac 12540
tgcctgctgg ccaggcttta aaatgaaaag tgttttaatg ctgccataaa agggaggcgg 12600
gggggaggaa gggaaaataa aggcatcttt ccaagtactc atctaattta attgtcaaaa 12660
gattgatagg ccatgaatta cttctccatc tcactaaggg ttaaaggcgt gcaacccccc 12720
actggctgtg tcccctgcca ccgaagtgag tgacctgccc tacaaccagg tgggaccacc 12780
tgtgctgcag tccggagggg cttctgcagg aagcactcac cccccacacc ttccccggcc 12840
tgagcttccc ctacctttcg tcaccacctg agggcatgag cacaggccat ggggcgtgcc 12900
tggtgagtct gcctgtggtt caggcttagc ctgtggtctc ctgtgtgctg ctgcccgcat 12960
gggatgcgca ggggaggcgt ggggatccgc aggagggtgg ttgggataca ccggatacct 13020
ctgctctcat tgcttgtttg caaatgctct atggacattt gtgtgctaaa tcctattaaa 13080
taaaaaagac gggttaaaac ccagatgctg tatattcatt tgtaattatg tataaagtga 13140
agcagtttta aactgtaaag attttttca gtgtgttttc tcgaattttg ccacaacata 13200
ctggcttcgt attttattta tctttctttc tagttaccag cttcagaccc ttgtaaagtc 13260
tccctcagcc ctttcaaaaa ataataaatt tcctgtgaag tt 13302

<210> 25
<211> 8442
<212> DNA
<213> Human

<400> 25

atgaaactta atccacagca agctccctta tatggtgatt gtgttgttac agtgctgctt 60
gctgaagagg acaaagctga agatgatgta gtgttttact tggtattttt gggttccacc 120
ctccgtcact gtacaagtac tcggaaggtc agttctgata cattggagac cattgctcct 180
ggtcatgatt gttgtgaaac agtgaaggtg cagctctgtg cttccaaaga gggccttccc 240
gtgtttgtgg tggctgaaga agactttcat ttcgtccagg atgaagcgta tgatgcagct 300
caattcctag caaccagtgc tggaaatcag caggctttga actttacccg ttttcttgac 360
cagtcaggac ccccatctgg ggatgtgaat tcccttgata agaagttggt gctggcattc 420
aggcacctga agctgcccac ggagtggaat gtattgggga cagatcagag tttgcatgat 480
gctggcccgc gagagacatt gatgcatttt gctgtgcggc tgggactgct gaggttgacg 540
tggttcctgt tgcagaagcc aggtggccgc agagctctca gtatccacaa ccaggaaggg 600
gcgacgcctg tgagcttggc cttggagcga ggctatcaca agctgcacca gcttctaacc 660
gaggagaatg ctggagaacc agactcctgg agcagtttat cctatgaaat accgtatgga 720
gactgttctg tgaggcatca tcgagagttg gacatctata cattaacctc tgagtctgat 780
tcacatcatg aacacccatt tcctggagac ggttgcactg gaccaatttt taaacttatg 840
aacatccaac agcaactaat gaaaacaaac ctcaagcaga tggacagtct tatgccctta 900
atgatgacag cacaggatcc ttccagtgcc ccagagacag atggccagtt tcttccctgt 960
gcaccggagc ccacggaccc tcagcgactt tcttcttctg aagagactga gagcactcag 1020
tgctgcccag ggagccctgt tgcacagact gaaagtcccc gtgatttgtc aagcatagtt 1080
gaggaggaga atacagaccg ttcctgtagg aagaaaaata aaggcgtgga aagaaaaggg 1140
gaagaggtgg agccagcacc tattgtggac tctggaactg tatctgatca agacagctgc 1200
cttcagagct tgcctgattg tggagtaaag ggcacggaag gcctttcgtc ctgtggaaac 1260
agaaatgaag aaactggaac aaaatcttct ggaatgccca cagaccagga gtccctgagc 1320
agtggagatg ctgtgcttca gagagacttg gtcatggagc caggcacagc ccagtattcc 1380
tctggaggtg aactgggagg catttcaaca acaaatgtca gtaccccaga cactgcaggg 1440
gaaatggaac atgggctcat gaacccagat gccactgttt ggaagaatgt gcttcaggga 1500
ggggaaagta caaaggaaag atttgagaac tctaatattg gcacagctgg agcctctgac 1560
gtgcacgtca caagtaagcc tgtggataaa atcagtgttc caaactgtgc ccctgccgcc 1620
agttccctgg atggtaacaa acctgctgag tcttcacttg catttagtaa tgaagaaacc 1680
tccactgaaa aaacagcaga aacggaaact tcacgaagtt gtgaggagag tgctgatgct 1740

```
ccagtagatc agaattctgt ggtgattcca gctgctgcaa aagacaagat ttcagatgga   1800
ttagaacctt atactctctt agcagtaggc ataggtgaga caatgtcacc cccagattta   1860
gcccttcttg ggctggaaga agatgtaatg ccacaccaga actcagaaac aaattcatct   1920
catgctcaaa gccaaaaggg caaatcctca cccatttgtt ctacaactgg agacgataaa   1980
ctttgtgcag actctgcatg tcaacagaac acagtgactt ctagtggcga tttggttgca   2040
aaactgtgtg ataacatagt tagcgagtcc gaaagcacca cagcaaggca acccagctca   2100
caagatccac ccgatgcctc ccactgtgaa gacccacagg ctcatacagt cacctctgac   2160
cctgtaaggg atacccagga acgtgcggat tttgtccttt tcaaagtggt ggataacaaa   2220
ggccaacgaa aagatgtgaa actagataaa cctttaacaa atatgcttga ggtggtttca   2280
catccacatc cagttgtccc taaaatggag aaagaactgg tgccagacca ggcagtaata   2340
tcagacagta ctttctctct ggcaaacagt ccaggcagtg aatcagtaac caaggatgac   2400
gcactttctt ttgtcccctc ccagaaagaa aagggaacag caactcctga actacataca   2460
gctacagatt atagagatgg cccagatgga aattcgaatg agcctgatac gcggccacta   2520
gaagacaggg cagtaggcct gtccacatcc tccactgctg cagagcttca gcacgggatg   2580
gggaatacca gtctcacagg acttggtgga gagcatgagg gtcccgcccc tccagcaatc   2640
ccagaagctc tgaatatcaa ggggaacact gactcttccc tgcaaagtgt gggtaaggcc   2700
actttggctt tagattcagt tttgactgaa gaaggaaaac ttctggtggt ttcagaaagc   2760
tctgcagctc aggaacaaga taaggataaa gcggtgacct gttcctctat taaggaaaat   2820
gctctctctt caggaacttt gcaggaagag cagagaacac cacctcctgg acaagatact   2880
caacaatttc atgaaaaatc aatctcagct gactgtgcca aggacaaagc acttcagcta   2940
agtaattcac cgggtgcatc ctctgccttt cttaaggcag aaactgaaca taacaaggaa   3000
gtggccccac aagtctcact gctgactcaa ggtggggctg cccagagcct ggtgccacca   3060
ggagcaagtc tggccacaga gtcaaggcag gaagccttgg gggcagagca caacagctcc   3120
gctctgttgc catgtctgtt gccagatggg tctgatgggt ccgatgctct taactgcagt   3180
cagccttctc ctctggatgt tggagtgaag aacactcaat cccagggaaa aactagtgcc   3240
tgtgaggtga gtggagatgt gacggtggat gttacagggg ttaatgctct acaaggtatg   3300
gctgagccca gaagagagaa tatatcacac aacacccaag acatcctgat tccaaacgtc   3360
ttgttgagcc aagagaagaa tgccgttcta ggtttgccag tggctctaca ggacaaagct   3420
gtgactgacc cacagggagt tggaacccca gagatgatac ctcttgattg ggagaaaggg   3480
aagctggagg gagcagacca cagctgtacc atgggtgacg ctgaggaagc ccaaatagac   3540
```

gatgaagcac atcctgtcct actgcagcct gttgccaagg agctccccac agacatggag 3600
ctctcagccc atgatgatgg ggccccagct ggtgtgaggg aagtcatgcg agccccgcct 3660
tcaggcaggg aaaggagcac tccctctcta ccttgcatgg tctctgccca ggacgcacct 3720
ctgcctaagg gggcagactt gatagaggag gctgccagcc gtatagtgga tgctgtcatc 3780
gaacaagtca aggccgctgg agcactgctt actgaggggg aggcctgtca catgtcactg 3840
tccagccctg agttgggtcc tctcactaaa ggactagaga gtgcttttac agaaaaagtg 3900
agtactttcc cacctgggga gagcctacca atgggcagta ctcctgagga agccacgggg 3960
agccttgcag gatgttttgc tggaagggag gagccagaga agatcatttt acctgtccag 4020
gggcctgagc cagcagcaga aatgccagac gtgaaagctg aagatgaagt ggattttaga 4080
gcaagttcaa tttctgaaga agtggctgta gggagcatag ctgctacact gaagatgaag 4140
caaggcccaa tgacccaggc gataaaccga gaaaactggt gtacaataga gccatgccct 4200
gatgcagcat ctcttctggc ttccaagcag agcccagaat gtgagaactt cctggatgtt 4260
ggactgggca gagagtgtac ctcaaaacaa ggtgtactta aaagagaatc tgggagtgat 4320
tctgacctct ttcactcacc cagtgatgac atggacagca tcatcttccc aaagccagag 4380
gaagagcatt tggcctgtga tatcaccgga tccagttcat ccaccgatga cacggcttca 4440
ctggaccgac attcttctca tggcagtgat gtgtctctct cccagatttt aaagccaaac 4500
aggtcaagag atcggcaaag ccttgatgga ttctacagcc atgggatggg agctgagggt 4560
cgagaaagtg agagtgagcc tgctgaccca ggcgacgtgg aggaggagga gatggacagt 4620
atcactgaag tgcctgcaaa ctgctctgtc ctaaggagct ccatgcgctc tctttctccc 4680
ttccggaggc acagctgggg gcctgggaaa aatgcagcca gcgatgcaga aatgaaccac 4740
cggagttcaa tgcgagttct tggggatgtt gtcaggagac ctcccattca taggagaagt 4800
ttcagtctag aaggcttgac aggaggagct ggtgtcggaa acaagccatc ctcatctcta 4860
gaagtaagct ctgcaaatgc cgaagagctc agacacccat tcagtggtga ggaacgggtt 4920
gactctttgg tgtcactttc agaagaggat ctggagtcag accagagaga acataggatg 4980
tttgatcagc agatatgtca cagatctaag cagcagggat ttaattactg tacatcagcc 5040
atttcctctc cattgacaaa atccatctca ttaatgacaa tcagccatcc tggattggac 5100
aattcacggc ccttccacag taccttccac aataccagtg ctaatctgac tgagagtata 5160
acagaagaga actataattt cctgccacat agcccctcca agaaagattc tgaatggaag 5220
agtggaacaa aagtcagtcg tacattcagc tacatcaaga ataaaatgtc tagcagcaag 5280
aagagcaaag aaaaggaaaa agaaaaagat aagattaagg agaaggagaa agattctaaa 5340
gacaaggaga aagataagaa gactgtcaac gggcacactt tcagttccat tcctgttgtg 5400

ggtcccatca gctgtagcca gtgtatgaag cccttcacca acaaagatgc ctatacttgt 5460
gcaaattgca gtgcttttgt ccacaaaggc tgccgagaaa gtctagcctc ctgtgcaaag 5520
gtcaaaatga agcagcccaa agggagcctt caggcacatg acacatcatc actgcccacg 5580
gtcattatga gaaacaagcc ctcacagccc aaggagcgtc ctcggtccgc agtcctcctg 5640
gtggatgaaa ccgctaccac cccaatattt gccaatagac gatcccagca gagtgtctcg 5700
ctctccaaaa gtgtctccat acagaacatt actggagttg gcaatgatga gaacatgtca 5760
aacacctgga aattcctgtc tcattcaaca gactcactaa ataaaatcag caaggtcaat 5820
gagtcaacag aatcacttac tgatgaggga gtaggtacag acatgaatga aggacaacta 5880
ctgggagact ttgagattga gtccaaacag ctggaagcag agtcttggag tcggataata 5940
gacagcaagt ttctaaaaca gcaaaagaaa gatgtggtca aacggcaaga agtaatatat 6000
gagttgatgc agacagagtt tcatcatgtc cgcactctca agatcatgag tggtgtgtac 6060
agccagggga tgatggcaga tctgcttttt gagcagcaga tggtagaaaa gctgttcccc 6120
tgtttggatg agctgatcag tatccatagc caattcttcc agaggattct ggagcggaag 6180
aaggagtctc tggtggataa aagtgaaaag aactttctca tcaagaggat aggggatgtg 6240
cttgtaaatc agttttcagg tgagaatgca gaacgtttaa agaagacata tggcaagttt 6300
tgtgggcaac ataaccagtc tgtaaactac ttcaaagacc tttatgccaa ggataagcgt 6360
tttcaagcct ttgtaaagaa gaagatgagc agttcagttg ttagaaggct tggaattcca 6420
gagtgcatat tgcttgtaac tcagcggatt accaagtacc cagttttatt ccaaagaata 6480
ttgcagtgta ccaaagacaa tgaagtggag caggaagatc tagcacagtc cttgagcctg 6540
gtgaaggatg tgattggagc tgtagacagc aaagtggcaa gttatgaaaa gaaagtgcgt 6600
ctcaatgaga tttatacaaa gacagatagc aagtcaatca tgaggatgaa gagtggtcag 6660
atgtttgcca aggaagattt gaaacggaag aagcttgtac gtgatgggag tgtgtttctg 6720
aagaatgcag caggaaggtt gaaagaggtt caagcagttc ttctcactga cattttagtt 6780
ttccttcaag aaaaagacca gaagtacatc tttgcatcat tggaccagaa gtcaacagtg 6840
atctctttaa agaagctgat tgtgagagaa gtggcacatg aggagaaagg tttattcctg 6900
atcagcatgg ggatgacaga tccagagatg gtagaagtcc atgccagctc caaagaggaa 6960
cgaaacagct ggattcagat cattcaggac acaatcaaca ccctgaacag agatgaagat 7020
gaaggaattc ctagtgagaa tgaggaagaa aagaaaatgt tggacaccag agcccgagaa 7080
ttaaaagaac aacttcacca gaaggaccaa aaaatcctac tcttgttgga agagaaggag 7140
atgatttcc gggacatggc tgagtgcagc acccctctcc cagaggattg ctccccaaca 7200

catagcccta gagttctctt ccgctccaac acagaagagg ctctcaaagg aggaccttta 7260
atgaaaagtg caataaatga ggtggagatc cttcagggtt tggtgagtgg aaatctggga 7320
ggcacacttg ggccgactgt cagcagcccc attgagcaag atgtggtcag tcccgtttcc 7380
ctgccccgga gagcagagac ctttggagga tttgacagcc atcagatgaa tgcttcaaaa 7440
ggaggcgaga aggaagaggg agatgatggc caagatctta ggagaacgga atcagatagt 7500
ggcctaaaaa agggtggaaa tgctaacctg gtatttatgc ttaaaagaaa cagtgagcag 7560
gttgtccaga gcgttgttca tctctacgag ctcctcagcg ctctgcaggg tgtggtgctg 7620
cagcaggaca gctacattga ggaccagaaa ctggtgctga gcgagagggc gctcactcgc 7680
agcttgtccc gcccgagctc cctcattgag caggagaagc agcgcagcct ggagaagcag 7740
cgccaggacc tggccaacct gcagaagcag caggcccagt acctcgagga gaagcgcagg 7800
cgcgagcgtg agtgggaagc tcgtgagagg gagctgcggg acgggaggcc ctcctggccc 7860
agcgcgagga ggaggtgcag caggggcagc aggacctgga aaaggagcgg gaggagctcc 7920
agcagaagaa gggcacatag ccagtatgac ctggagcgac tgcgtgctgc ccagaaacag 7980
cttgagaggg aacaggagca gctgcgccgg gaggcagagc ggctcagcca gcggcagaca 8040
gaacgggacc tgtgtcaggt ttcccatcca cataccaagc tgatgaggat cccatcgttc 8100
ttccccagtc ctgaggagcc cccctcgcca tctgcacctt ccatagccaa atcagggtca 8160
ttggactcag aactttcagt gtccccaaaa aggaacagca tctctcggac acacaaagat 8220
aaggggcctt ttcacatact gagttcaacc agccagacaa acaaaggacc agaagggcag 8280
agccaggccc ctgcgtccac ctctgcctct acccgcctgt ttgggttaac aaagccaaag 8340
gaaaagaagg agaaaaaaaa gaagaacaaa accagccgct ctcagcccgg tgatggtccc 8400
gcgtcagaag tatcagcaga gggtgaagag atcttctgct ga 8442

<210> 26
<211> 3777
<212> DNA
<213> Human

<400> 26

```
gaagcgcctg tgctctgccg agactgccgt gcccattgct cgcctcggtc gccgccgctt      60
tagccgcctc cgggggagcg gccgcctatt gtctttctcc gcggcgaagg tgaagagttg     120
tcccagctcg gcccgcgggg gagccccggg agccgcacgt gtcctgggtc atgaaactta     180
atccacagca agctccctta tatggtgatt gtgttgttac agtgctgctt gctgaagagg     240
acaaagctga agatgatgta gtgttttact tggtattttt gggttccacc ctccgtcact     300
gtacaagtac tcggaaggtc agttctgata cattggagac cattgctcct ggtcatgatt     360
```

```
gttgtgaaac agtgaaggtg cagctctgtg cttccaaaga gggccttccc gtgtttgtgg    420
tggctgaaga agactttcat ttcgtccagg atgaagcgta tgatgcagct caattcctag    480
caaccagtgc tggaaatcag caggctttga actttacccg ttttcttgac cagtcaggac    540
ccccatctgg ggatgtgaat tcccttgata agaagttggt gctggcattc aggcacctga    600
agctgcccac ggagtggaat gtattgggga cagatcagag tttgcatggt gagaatttat    660
atgatctaca aacacacttt aagtttgtga tatttctact tttttttaa tggggctact     720
tttctatggt catcatattc aggtcttcca cagaggctgc gtaaatctta gaattactgt    780
aggaaagtgg cctcaatggc atctcttcta ggttcttcaa ttgagctgtt aagcatgttg    840
ttgcagcgaa atgaccctag ggattaccca aggagagtgg cacgtcacct tcctgtgggt    900
ctggatttca gatttgatct ctcaagtgta gaaagtgaca cgctcttcat gaggagagta    960
gcagagttac tttttggtgt ttaatgatca aagacatggt tttcattatt gtattctgaa   1020
gttctctaaa gatagcctgg aagtgaagga gaggaaagat tgggcagtga gtgggaggag   1080
catgagaaca gggtgtgctc tgagaaacca cacacttcct taaccaggtt agtgtcactc   1140
ttactgactt tatctgttgg ccctccctat gagagttcac tgaagaaagg actttttgtt   1200
tgttttttgt tttttttga gaacgggctt tcactcttgt agtccaggct ggagtgcagt    1260
ggcatgatct cggctcactg cagcctccac ctcctgggtt caggtgattc tgctgcctca   1320
gcctcccaaa tagctgggat tacaggcatg taccaccatg cctagcaaat tttttttttt   1380
ttttaattag agacagggtt tcaccgtgtt agtcaggctg gtctcgaact cctgacctca   1440
ggtaatctac ccacctcggc ctcccaaagt gctgggatta taggcatgag ccactgcgcc   1500
cggccagaac tccacttttt caaaaatatt tacatagtaa gtcctcttac tcaaacaagg   1560
gttttcccac ataattctag tagccaatga catctacttg atgtcataat tgtatagata   1620
tttttaatg aaacataatg tgtacctgga gttcaataag taacatccag tttcccttct    1680
gagttcaagt ccaattgaac gatttgggag tcctgagata gaaaccaaaa tgtttattac   1740
tgatgaaatc tagattggaa gcctgatgtt agagctcctg ccagaagggt ctgcttgtac   1800
tttctatgtc ttcaatgaaa gagctggtca gtcacaccat tgccctgtag agtccaccca   1860
gccttgagta ttgaattatt ttgggtaagc agaccacaag tcactctccc cacaggtgag   1920
cagttcctga ggagaaatac ccagaggggc ctgagccata gcatagttag tttctacact   1980
cagatgaatt ttgtcttctt ccatggaaga atggtaaaga ggtctttgcc agctttgatt   2040
ctcttaaatc tacactaaat ttaacagtta aacctgtggt cagccgtcag aacggtgtcc   2100
catactgtca ggtgcatact gtcagattct acctggatag tcctgcccag gtaaaacctg   2160
aaaggaagta tcaaatcaag gaagaaagga tttatccttt tcttcttctt tttttaagta   2220
```

```
tagtgtccaa gaaacttctg tcttgctaat atttagagat cagaatattt gacattctga 2280
aaaggcactc atgccttata taaacctggt gatgggcctt aatacctcta gctcctccta 2340
tttctgtatt gatacaggaa tgtttattct ggctgccttt cacaaatcag tgtcaagata 2400
gtactgcatt agttatttgt ggggcttatg gtgttttatg gttttgtttt gtcttgttta 2460
aagaggggcc caggagttgg atagttttat gttgccatga ttaaaaacaa ctattagaaa 2520
aagcaaacat ctgaaacttt ggacatatca cttcctcttt ctctctcttt tttttttttt 2580
ttttgagata gagtcttgct ctgtcgccca ggctggagtg caatggtgca atctcggctc 2640
actgcaacct gcatctcctg ggttgaagcg attctcctgc cttagcctcc caagtagctg 2700
ggaatacagg cacatgccac cacaccctgc tgcttttttg tgtgttttag tagagacggg 2760
gtttcaccat gttgcccagg ctggtcttga actcctgagc tcaggcaatc cacccgcctc 2820
agcctcccaa agtgctagga ttacaggcgt gagccactgt gcccggccac ttcctctctt 2880
tgggccttaa cttccttgtc tttaatagga gggaattggc cagctttaat tttttttgt 2940
actgtcagtg tactggtaaa ttttctgttg cacaaaattg ctcaagcagt gctaacaaag 3000
gccaaaccaa atttgaacac ttgaggaagg aaatacatat taaaaccaca atgagatatc 3060
attacacatt tatcataatg gctaagatga agaataccaa gtgctggcaa agatgaagaa 3120
catatcatat gttgcttgta ggaatgcaaa atgtataacc actctagaaa tatttggcag 3180
tttcttgtaa agttaaacat acacttagcg tatgacccag taatcccact tttgggtgtt 3240
tatcctagag aaatgaaaat ctgtagtcac caaaaagtct gtgcacagac gtgctcatag 3300
taatgttatt cataatcttc aaaaactgga aataacccag atttccctca ctgagtgaat 3360
acagaagcac actataatat atccatacag tggaatacta ctcagcaata aaaagaagca 3420
aactgttgac atgtgcaaca acttggatgt gttttaaatg cattatgcgg gaaaatgaag 3480
tcagtttcca aaggttccat actatgtgat tctatttata tgatattctg gaagatgcaa 3540
aattatagaa aaagagaaca gatgagtagt tgctaggggc tgggagtggg ggaagtctga 3600
ctggaaagag gtatcatgag ggaattcttt gggttgttgg agttgttttg tcttgttcgt 3660
ggtggtggct gtaagaatct atgcatgtgt taaaactcat aggagtgtac atcccctaaa 3720
ggtgaatttt attgtacata tatttaaaat aataaaataa caaaaaaaaa aaaaaaa 3777
```

<210> 27
<211> 4290
<212> DNA
<213> Human

<400> 27

atgttgtata taaaccgaga aaactggtgt acaatagagc catgccctga tgcagcatct 60

```
cttctggctt ccaagcagag cccagaatgt gagaacttcc tggatgttgg actgggcaga     120
gagtgtacct caaaacaagg tgtacttaaa agagaatctg ggagtgattc tgacctcttt     180
cactcaccca gtgatgacat ggacagcatc atcttcccaa agccagagga agagcatttg     240
gcctgtgata tcaccggatc cagttcatcc accgatgaca cggcttcact ggaccgacat     300
tcttctcatg gcagtgatgt gtctctctcc cagattttaa agccaaacag gtcaagagat     360
cggcaaagcc ttgatggatt ctacagccat gggatgggag ctgagggtcg agaaagtgag     420
agtgagcctg ctgacccagg cgacgtggag gaggaggaga tggacagtat cactgaagtg     480
cctgcaaact gctctgtcct aaggagctcc atgcgctctc tttctccctt ccggaggcac     540
agctgggggc ctgggaaaaa tgcagccagc gatgcagaaa tgaaccaccg gagttcaatg     600
cgagttcttg gggatgttgt caggagacct cccattcata ggagaagttt cagtctagaa     660
ggcttgacag gaggagctgg tgtcggaaac aagccatcct catctctaga agtaagctct     720
gcaaatgccg aagagctcag acacccattc agtggtgagg aacgggttga ctctttggtg     780
tcactttcag aagaggatct ggagtcagac cagagagaac ataggatgtt tgatcagcag     840
atatgtcaca gatctaagca gcagggattt aattactgta catcagccat ttcctctcca     900
ttgacaaaat ccatctcatt aatgacaatc agccatcctg gattggacaa ttcacggccc     960
ttccacagta ccttccacaa taccagtgct aatctgactg agagtataac agaagagaac    1020
tataatttcc tgccacatag cccctccaag aaagattctg aatggaagag tggaacaaaa    1080
gtcagtcgta cattcagcta catcaagaat aaaatgtcta gcagcaagaa gagcaaagaa    1140
aaggaaaaag aaaaagataa gattaaggag aaggagaaag attctaaaga caaggagaaa    1200
gataagaaga ctgtcaacgg gcacactttc agttccattc ctgttgtggg tcccatcagc    1260
tgtagccagt gtatgaagcc cttcaccaac aaagatgcct atacttgtgc aaattgcagt    1320
gcttttgtcc acaaaggctg ccgagaaagt ctagcctcct gtgcaaaggt caaaatgaag    1380
cagcccaaag ggagccttca ggcacatgac acatcatcac tgcccacggt cattatgaga    1440
aacaagccct cacagcccaa ggagcgtcct cggtccgcag tcctcctggt ggatgaaacc    1500
gctaccaccc caatatttgc caatagacga tcccagcaga gtgtctcgct ctccaaaagt    1560
gtctccatac agaacattac tggagttggc aatgatgaga acatgtcaaa cacctggaaa    1620
ttcctgtctc attcaacaga ctcactaaat aaaatcagca aggtcaatga gtcaacagaa    1680
tcacttactg atgagggagt aggtacagac atgaatgaag gacaactact gggagacttt    1740
gagattgagt ccaaacagct ggaagcagag tcttggagtc ggataataga cagcaagttt    1800
ctaaaacagc aaaagaaaga tgtggtcaaa cggcaagaag taatatatga gttgatgcag    1860
```

acagagtttc atcatgtccg cactctcaag atcatgagtg gtgtgtacag ccaggggatg 1920

atggcggatc tgctttttga gcagcagatg gatgaaaagc tgttcccctg tttggatgag 1980

ctgatcagta tccatagcca attcttccag aggattctgg agcggaagaa ggagtctctg 2040

gtggataaaa gtgaaaagaa ctttctcatc aagaggatag gggatgtgct tgtaaatcag 2100

ttttcaggtg agaatgcaga acgtttaaag aagacatatg gcaagttttg tgggcaacat 2160

aaccagtctg taaactactt caaagacctt tatgccaagg ataagcgttt tcaagccttt 2220

gtaaagaaga agatgagcag ttcagttgtt agaaggcttg gaattccaga gtgcatattg 2280

cttgtaactc agcggattac caagtaccca gttttattcc aaagaatatt gcagtgtacc 2340

aaagacaatg aagtggagca ggaagatcta gcacagtcct tgagcctggt gaaggatgtg 2400

attggagctg tagacagcaa agtggcaagt tatgaaaaga aagtgcgtct caatgagatt 2460

tatacaaaga cagatagcaa gtcaatcatg aggatgaaga gtggtcagat gtttgccaag 2520

gaagatttga aacggaagaa gcttgtacgt gatgggagtg tgtttctgaa gaatgcagca 2580

ggaaggttga aagaggttca agcagttctt ctcactgaca ttttagtttt ccttcaagaa 2640

aaagaccaga agtacatctt tgcatcattg gaccagaagt caacagtgat ctctttaaag 2700

aagctgattg tgagagaagt ggcacatgag gagaaaggtt tattcctgat cagcatgggg 2760

atgacagatc cagagatggt agaagtccat gccagctcca aagaggaacg aaacagctgg 2820

attcagatca ttcaggacac aatcaacacc ctgaacagag atgaagatga aggaattcct 2880

agtgagaatg aggaagaaaa gaaaatgttg gacaccagag cccgagaatt aaaagaacaa 2940

cttcaccaga aggaccaaaa aatcctactc ttgttggaag agaaggagat gattttccgg 3000

gacatggctg agtgcagcac ccctctccca gaggattgct ccccaacaca tagccctaga 3060

gttctcttcc gctccaacac agaagaggct ctcaaaggag gacctttaat gaaaagtgca 3120

ataaatgagg tggagatcct tcagggtttg gtgagtggaa atctgggagg cacacttggg 3180

ccgactgtca gcagccccat tgagcaagat gtggtcagtc ccgtttccct gccccggaga 3240

gcagagacct ttggaggatt tgacagccat cagatgaatg cttcaaaagg aggcgagaag 3300

gaagagggag gtgatggcca agatcttagg agaacggaat cagatagtgg cctaaaaaag 3360

ggtggaaatg ctaacctggt atttatgctt aaaagaaaca gtgagcaggt tgtccagagc 3420

gttgttcatc tctacgagct cctcagcgct ctgcagggtg tggtgctgca gcaggacagc 3480

tacattgagg accagaaact ggtgctgagc gagagggcgc tcactcgcag cttgtcccgc 3540

ccgagctccc tcattgagca ggagaagcag cgcagcctgg agaagcagcg ccaggacctg 3600

gccaacctgc agaagcagca ggcccagtac ctcgaggaga agcgcaggcg cgagcgtgag 3660

tgggaagctc gtgagaggga gctgcgggac gggaggccct cctggcccag cgcgaggagg 3720

```
aggtgcagca ggggcagcag gacctggaaa aggagcggga ggagctccag cagaagaagg   3780
gcacatagcc agtatgacct ggagcgactg cgtgctgccc agaaacagct tgagagggaa   3840
caggagcacg tgcgccggga ggcagagcgg ctcagccagc ggcagacaga acgggacctg   3900
tgtcaggttt cccatccaca taccaagctg atgaggatcc catcgttctt ccccagtcct   3960
gaggagcccc cctcgccatc tgcaccttcc atagccaaat cagggtcatt ggactcagaa   4020
ctttcagtgt ccccaaaaag gaacagcatc tctcggacac acaaagataa ggggcctttt   4080
cacatactga gttcaaccag ccagacaaac aaaggaccag aagggcagag ccaggcccct   4140
gcgtccacct ctgcctctac ccgcctgttt gggttaacaa agccaaagga aaagaaggag   4200
aaaaaaaaga agaacaaaac cagccgctct cagcccggtg atggtcccgc gtcagaagta   4260
tcagcagagg gtgaagagat cttctgctga                                     4290
```

<210> 28
<211> 424
<212> PRT
<213> Human

<400> 28

```
Met Ser Asn Thr Trp Lys Phe Leu Ser His Ser Thr Asp Ser Leu Asn
1               5                   10                  15

Lys Ile Ser Lys Val Asn Glu Ser Thr Glu Ser Leu Thr Asp Glu Gly
            20                  25                  30

Thr Asp Met Asn Glu Gly Gln Leu Leu Gly Asp Phe Glu Ile Glu Ser
        35                  40                  45

Lys Gln Leu Glu Ala Glu Ser Trp Ser Arg Ile Ile Asp Ser Lys Phe
    50                  55                  60

Leu Lys Gln Gln Lys Lys Asp Val Val Lys Arg Gln Glu Val Ile Tyr
65                  70                  75                  80

Glu Leu Met Gln Thr Glu Phe His His Val Arg Thr Leu Lys Ile Met
                85                  90                  95

Ser Gly Val Tyr Ser Gln Gly Met Met Ala Asp Leu Leu Phe Glu Gln
            100                 105                 110

Gln Met Val Glu Lys Leu Phe Pro Cys Leu Asp Glu Leu Ile Ser Ile
        115                 120                 125
```

```
His Ser Gln Phe Phe Gln Arg Ile Leu Glu Arg Lys Lys Glu Ser Leu
    130                 135                 140

Val Asp Lys Ser Glu Lys Asn Phe Leu Ile Lys Arg Ile Gly Asp Val
145                 150                 155                 160

Leu Val Asn Gln Phe Ser Gly Glu Asn Ala Glu Arg Leu Lys Lys Thr
                165                 170                 175

Tyr Gly Lys Phe Cys Gly Gln His Asn Gln Ser Val Asn Tyr Phe Lys
            180                 185                 190

Asp Leu Tyr Ala Lys Asp Lys Arg Phe Gln Ala Phe Val Lys Lys Lys
        195                 200                 205

Met Ser Ser Ser Val Val Arg Arg Leu Gly Ile Pro Glu Cys Ile Leu
    210                 215                 220

Leu Val Thr Gln Arg Ile Thr Lys Tyr Pro Val Leu Phe Gln Arg Ile
225                 230                 235                 240

Leu Gln Cys Thr Lys Asp Asn Glu Val Glu Gln Glu Asp Leu Ala Gln
                245                 250                 255

Ser Leu Ser Leu Val Lys Asp Val Ile Gly Ala Val Asp Ser Lys Val
            260                 265                 270

Ala Ser Tyr Glu Lys Lys Val Arg Leu Asn Glu Ile Tyr Thr Lys Thr
        275                 280                 285

Asp Ser Lys Ser Ile Met Arg Met Lys Ser Gly Gln Met Phe Ala Lys
    290                 295                 300

Glu Asp Leu Lys Arg Lys Lys Leu Val Arg Asp Gly Ser Val Phe Leu
305                 310                 315                 320

Lys Asn Ala Ala Gly Arg Leu Lys Glu Val Gln Ala Val Leu Leu Thr
                325                 330                 335

Asp Ile Leu Val Phe Leu Gln Glu Lys Asp Gln Lys Tyr Ile Phe Ala
            340                 345                 350

Ser Leu Asp Gln Lys Ser Thr Val Ile Ser Leu Lys Lys Leu Ile Val
        355                 360                 365

Arg Glu Val Ala His Glu Glu Lys Gly Leu Phe Leu Ile Ser Met Gly
```

370 375 380

Met Thr Asp Pro Glu Met Val Glu Val His Ala Ser Ser Lys Glu Glu
385 390 395 400

Arg Asn Ser Trp Ile Gln Ile Ile Gln Asp Thr Ile Asn Thr Leu Ser
405 410 415

Gly Asn Gly Trp Arg Cys Phe Asn
420

<210> 29
<211> 2170
<212> DNA
<213> Human

<400> 29

ctgagggctc atccctctgc agagcgcggg gtcaccggga ggagacgcca tgacgcccgc 60
cctcacagcc ctgctctgcc ttgggctgag tctgggcccc aggacccgcg tgcaggcagg 120
gcccttcccc aaacccaccc tctgggctga gccaggctct gtgatcagct gggggagccc 180
cgtgaccatc tggtgtcagg ggagcctgga ggcccaggag taccgactgg ataaagaggg 240
aagcccagag cccttggaca gaaataaccc actggaaccc aagaacaagg ccagattctc 300
catcccatcc atgacagagc accatgcggg gagataccgc tgccactatt acagctctgc 360
aggctggtca gagcccagcg accccctgga gctggtgatg acaggattct acaacaaacc 420
caccctctca gccctgccca gccctgtggt ggcctcaggg gggaatatga ccctccgatg 480
tggctcacag aagggatatc accattttgt tctgatgaag gaaggagaac accagctccc 540
ccggaccctg gactcacagc agctccacag tgggggttc caggccctgt tccctgtggg 600
ccccgtgaac cccagccaca ggtggaggtt cacatgctat tactattata tgaacacccc 660
ccaggtgtgg tcccacccca gtgacccct ggagattctg ccctcaggcg tgtctaggaa 720
gccctccctc ctgaccctgc agggccctgt cctggcccct gggcagagcc tgaccctcca 780
gtgtggctct gatgtcggct acgacagatt tgttctgtat aaggaggggg aacgtgactt 840
cctccagcgc cctggccagc agccccaggc tgggctctcc caggccaact tcaccctggg 900
ccctgtgagc cctcccacg ggggccagta caggtgctat ggtgcacaca acctctcctc 960
cgagtggtcg gcccccagcg accccctgaa catcctgatg gcaggacaga tctatgacac 1020
cgtctccctg tcagcacagc cgggccccac agtggcctca ggagagaacg tgaccctgct 1080
gtgtcagtca tggtggcagt ttgacacttt ccttctgacc aaagaagggg cagcccatcc 1140
cccactgcgt ctgagatcaa tgtacggagc tcataagtac caggctgaat tccccatgag 1200

```
tcctgtgacc tcagcccacg cggggaccta caggtgctac ggctcataca gctccaaccc   1260
ccacctgctg tctttcccca gtgagcccct ggaactcatg gtctcaggac actctggagg   1320
ctccagcctc ccacccacag ggccgccctc cacacctggt ctgggaagat acctggaggt   1380
tttgattggg gtctcggtgg ccttcgtcct gctgctcttc ctcctcctct tcctcctcct   1440
ccgacgtcag cgtcacagca aacacaggac atctgaccag agaaagactg atttccagcg   1500
tcctgcaggg gctgcggaga cagagcccaa ggacaggggc ctgctgagga ggtccagccc   1560
agctgctgac gtccaggaag aaaacctcta tgctgccgtg aaggacacac agtctgagga   1620
cagggtggag ctggacagtc agagcccaca cgatgaagac ccccaggcag tgacgtatgc   1680
cccggtgaaa cactccagtc ctaggagaga aatggcctct cctccctcct cactgtctgg   1740
ggaattcctg gacacaaagg acagacaggt ggaagaggac aggcagatgg acactgaggc   1800
tgctgcatct gaagcctccc aggatgtgac ctacgcccag ctgcacagct tgacccttag   1860
acggaaggca actgagcctc ctccatccca ggaaggggaa cctccagctg agcccagcat   1920
ctacgccact ctggccatcc actagcccgg ggggtacgca gaccccacac tcagcagaag   1980
gagactcagg actgctgaag gcacgggagc tgcccccagt ggacaccagt gaaccccagt   2040
cagcctggac ccctaacaca gaccatgagg agacgctggg aacttgtggg actcacctga   2100
ctcaaagatg actaatatcg tcccattttg gaaataaagc aacagacttc tcaacaatca   2160
atgagttaat                                                          2170
```